# EUROPEAN PATENT APPLICATION

(11) **EP 4 534 104 A1**
(43) Date of publication of application: **09.04.2025**
(21) Application number: 23815167.4
(22) Date of filing: 29.05.2023
(51) Int. Cl.: A61K 47/68, A61K 39/00, C07K 16/28, C07K 5/083, C07B 37/04

(54) **PREPARATION METHOD FOR DRUG LINKER CONJUGATE AND INTERMEDIATE THEREOF**

(30) Priority: 30.05.2022 CN 202210600523; 25.08.2022 CN 202211029593
(71) Applicant: Medilink Therapeutics (Suzhou) Co., Ltd., Suzhou, Jiangsu 215000 (CN)
(72) Inventor: SONG, Shuai, Suzhou, Jiangsu 215000 (CN); SHAN, Ailin, Suzhou, Jiangsu 215000 (CN); CAI, Jiaqiang, Suzhou, Jiangsu 215000 (CN); LI, Jundong, Suzhou, Jiangsu 215000 (CN); XIAO, Liang, Suzhou, Jiangsu 215000 (CN); XUE, Tongtong, Suzhou, Jiangsu 215000 (CN)
(74) Representative: Greaves Brewster LLP
(86) International application number: PCT/CN2023/096922
(87) International publication number: WO 2023/231988

(57) **Abstract**

A preparation method for a drug linker conjugate. Provided are a method for preparing the intermediate or the salt thereof, and the use of the intermediate or the salt thereof. The raw materials involved in the preparation method are easy to obtain, the operation is simple, the product purity is high, and the method is suitable for scaled synthesis.

## Description

The present application claims priority to China Patent Application No. 2022106005234 filed on May 30, 2022 and China Patent Application No. 2022110295935 filed on August 25, 2022, both of which are incorporated herein by reference in their entireties.

### TECHNICAL FIELD

The present application belongs to the field of pharmaceutical chemistry, and particularly relates to a preparation method for a drug linker conjugate or a salt thereof and a related intermediate or a salt thereof.

### BACKGROUND

The antibody drug conjugate (ADC) is a conjugate of an antibody and a small molecule drug, which combines the tumor-targeting effect of an antibody and the activity of a bioactive molecule to become a biological missile with promising advantages of efficacy and safety. The antibody guides the ADC to bind to a target cell/tissue, and the small molecule drug is subsequently released in the cell/tissue by enzymatic hydrolysis under the action of specific enzymes to treat the disease. Among them, the drug linker conjugate (containing cytotoxic payload and linker) plays a very important role in the preparation of ADCs. Therefore, it is of great significance for the development and application of ADC drugs to develop the drug linker conjugate synthesis methods that are simple, efficient, low-cost and suitable for large-scale synthesis.

Ds8201a is a marketed ADC molecule, and a toxin linker thereof contains a special oxazaacetal structure H ( 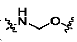 ), which has a good therapeutic effect on breast cancer with medium or high HER2 expression. However, the synthesis method for ADC molecules containing oxazaacetal structures is limited. Firstly, carboxylic acid is oxidized and rearranged by utilizing Pb(OAc)₄ to obtain an acetate structure, and then a target molecule is obtained by acid catalysis. Pb(OAc)₄ used in the method has severe limitation on substrates, and the molecule cannot contain oxidizable groups (e.g., amino groups and substituted amino groups), or otherwise, the reaction yield is extremely low. Secondly, an acetate structure is obtained by oxidizing and rearranging Pb(OAc)₄, and then reacts with a halogenating reagent (e.g., TMSCl or TMSBr) to prepare a halide, and the halide is then subjected to a nucleophilic substitution reaction with hydroxy of the substrate under an alkaline condition. Besides Pb(OAc)₄, this method uses alkali (e.g., potassium tert-butoxide) in the nucleophilic substitution reaction, which is not suitable for substrates unstable to alkali, such as camptothecin compounds. Meanwhile, as the above methods all use Pb(OAc)₄, Pb residues in the system may cause the poisoning of the corresponding metal catalyst when the amino protecting group is removed by metal catalytic hydrogenation in subsequent reactions, which will greatly affect the reaction yield. Therefore, there is a need to develop a new method for synthesizing oxazaacetal, which solves the problems of large limitation on substrates, low reaction yield, etc., thereby meeting the requirement of large-scale production. WO2022170971 discloses a class of ADC molecules containing oxazaacetal structures, which have good antitumor activity. The synthetic route for the preparation of a drug linker conjugate for ADC molecules is as follows:

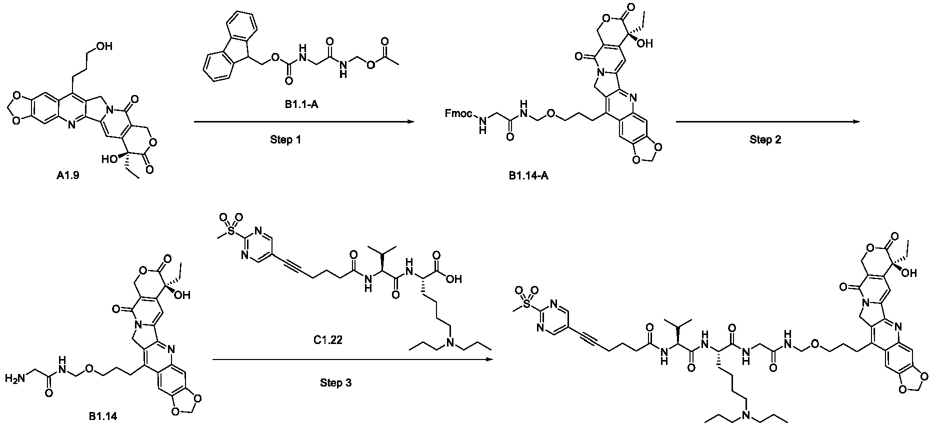

In the route, A1.9 is used as a starting material, and a target compound is obtained through a three-step reaction. The synthetic route has the following defects: 1. A1.9 is a cytotoxic compound and belongs to a high-activity molecule, and the operation is difficult when an isolator is required in the three-step reaction during process scale-up production; 2. B1.14-A is one of key intermediates in the route, column chromatography purification is needed, the using amount of a solvent is large, and large-scale production is difficult; 3. B1.14 is another key intermediate in the route, has poor chemical stability, and can be polymerized quickly, and it is difficult to control the quality during the process scale-up production; and 4. the overall yield of the route is < 5% (calculated by A1.9), and the cost is high.

In conclusion, the above route cannot be used for process scale-up production. How to reduce the reaction steps involving high-activity molecules, avoid the appearance of unstable intermediates, simplify the purification method and increase the yield is directly associated with whether the subsequent process scale-up production can be carried out smoothly, so a new synthetic route is urgently required to be developed to meet the requirement of scale-up production.

### SUMMARY

The present application provides a preparation method for a drug linker conjugate compound of formula I, a preparation method for an intermediate or a salt thereof used, and use of the intermediate or the salt thereof. The preparation method of the present application has the advantages of wide substrate adaptability, simpler operation and suitability for large-scale production.

In one aspect, the present application provides a preparation method for a compound of formula I or a salt thereof, 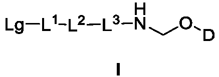 wherein,
D is a bioactive molecular fragment, preferably a camptothecin bioactive molecular fragment of formula I-A, 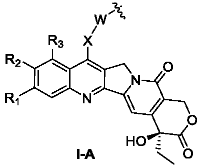
wherein,
R₁ and R₂ are each independently selected from H, halogen, -OH, optionally substituted C1-6 alkyl, and optionally substituted C1-6 alkoxy, or,
R₁ and R₂, together with the carbon atoms to which they are attached, form a 5-7 membered carbocyclic ring, or a 5-7 membered heterocyclic ring containing one or more O, S, N, carbonyl, a sulfoxide group or a sulfone group or any combination thereof;
R₃ is selected from H, halogen, -OH, -NH₂, optionally substituted C1-6 alkyl and optionally substituted C1-6 alkoxy, or,
R₃ and X, together with the carbon atoms to which they are attached, form a 5-7 membered carbocyclic ring, or a 5-7 membered heterocyclic ring containing one or more O, S, N, carbonyl, a sulfoxide group or a sulfone group or any combination thereof, or,
R₃ and R₂, together with the carbon atoms to which they are attached, form a 5-7 membered carbocyclic ring, or a 5-7 membered heterocyclic ring containing one or more O, S, N, carbonyl, a sulfoxide group or a sulfone group, or any combination thereof;
W is absent or present, and when W is present, W is selected from 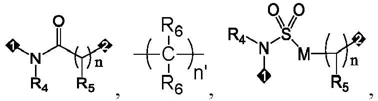 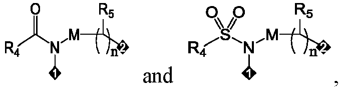 wherein position 1 is attached to X, and position 2 is attached to an oxygen atom;
X is selected from a direct bond, optionally substituted -O-(CH₂)ₙ₃-, -N(R₄)-(CH₂)ₙ₃-, -S-(CH₂)ₙ₃-, carbonyl-(CH₂)ₙ₃, -SO₂-(CH₂)ₙ₃-, 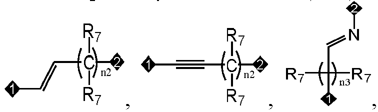 -(CH₂)ₙ₁-, C3-6 cycloalkyl, C6-10 aryl, 5-10 membered heteroaryl, and 4-10 membered heterocyclyl, wherein position 1 is attached to a parent ring, and position 2 is attached to W or an oxygen atom; the substituent is selected from one or more C1-4 alkyl, C3-6 cycloalkyl, or C3-6 cycloalkyl formed by more C1-4 alkyl together with the carbon atoms to which they are attached;
each M is independently selected from a direct bond and -CR^{5a}R^{5b}-;
R₄, R₅, R^{5a}, R^{5b}, R₆, and R₇ are each independently selected from H, optionally substituted C1-4 alkyl, optionally substituted C1-4 alkoxy, and optionally substituted C3-6 cycloalkyl;
n, n', n1, n2, and n3 are each independently selected from any integer between 0 and 6;
more preferably, formula I-A is selected from the following structures: 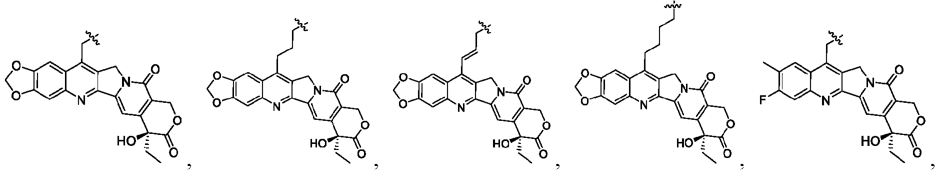 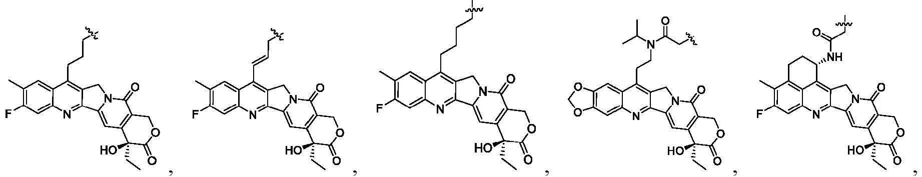 and 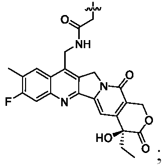
L¹ is selected from: 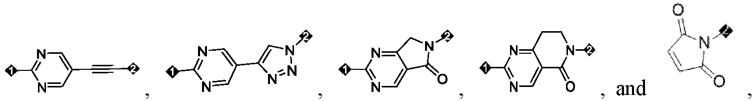 wherein position 1 is attached to Lg, and position 2 is attached to L²;
Lg is a leaving group when reacting with an antibody; Lg is selected from halogen, a sulfone group, a tertiary amine salt group (Me₃N⁺ or Et₃N⁺), a diazonium salt group, -OMs, MeSO₂-, and CF₃SO₃-; preferably, Lg is selected from F, Cl, and MeSO₂-; more preferably, Lg is MeSO₂-;
when L¹ is 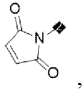 Lg is absent;
L² is selected from 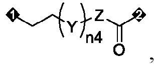 wherein position 1 is attached to L¹, and position 2 is attached to L³;
n4 is selected from any integer between 0 and 10;
Y is selected from -CH₂- and -OCH₂CH₂-;
Z is selected from CR^{m}Rⁿ and NR^{m};
R^{m} and Rⁿ are each independently selected from H, deuterium, C1-4 alkyl, C2-4 alkenyl, C2-4 alkynyl, C3-6 cycloalkyl, and 3-6 membered heterocyclyl,
or R^{m} and Rⁿ, together with the carbon atom to which they are both attached, form a 3-6 membered carbocyclic ring, or a 3-6 membered heterocyclic ring;
L³ is selected from a short peptide consisting of 2-10 amino acid residues; the amino acid residue is selected from a natural amino acid residue, a non-natural amino acid residue, or an amino acid residue represented by AA¹, or a stereoisomer thereof;
the structure of the amino acid residues represented by AA¹ is shown as follows: 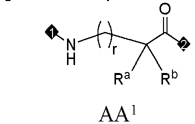
wherein,
R^{a} and R^{b} are each independently selected from H and 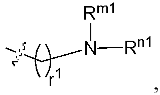 and R^{a} and R^{b} are not both H; or R^{a} and R^{b}, together with the carbon atom to which they are both attached, form a 4-10 membered heterocyclic ring, and the 4-10 membered heterocyclic ring is optionally substituted with one or more R⁰; r and r¹ are each independently selected from any integer between 0 and 20;
R^{m1} and Rⁿ¹ are each independently selected from H, C1-6 alkyl, and C3-6 cycloalkyl;
or R^{m1} and Rⁿ¹, together with the nitrogen atom to which they are both attached,form a 4-10 membered heterocyclic ring, and the 4-10 membered heterocyclic ring is optionally substituted with one or more R^{0'}; R⁰ and R^{0'} are each independently selected from C1-6 alkyl, C3-6 cycloalkyl, -NR^{m2}Rⁿ², and 4-10 membered heterocyclyl optionally substituted with C1-6 alkyl;
R^{m2} and Rⁿ² are each independently selected from H and C1-6 alkyl.

The preparation method comprises the following step:
a step of reacting a compound of formula I-1 or a salt thereof with a compound of formula X-3 or a salt thereof to obtain the compound of formula I or the salt thereof, 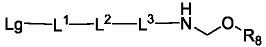 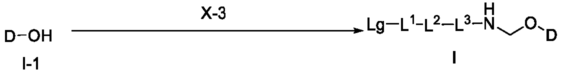
wherein R₈ is selected from hydrogen, C1-30 alkyl, C3-7 cycloalkyl, 3-20 membered heterocyclyl, 5-10 membered heteroaryl, C6-10 aryl, C1-6 alkyl-C3-6 cycloalkyl, C1-6 alkyl-4-6 membered heterocyclyl, C1-6 alkyl-5-10 membered heteroaryl, C1-6 alkyl-C6-10 aryl, and 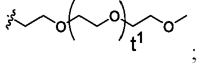 the alkyl, cycloalkyl, heterocyclyl, heteroaryl, or aryl is optionally substituted with one or more R^{x};
R^{x} is selected from hydrogen, deuterium, halogen, hydroxy, C1-4 alkyl, C1-4 alkoxy, C2-4 alkenyl, C2-4 alkynyl, -NR^{m2}Rⁿ², nitro, cyano, azido, oxo, an ester group, and carboxyl;
t¹ is selected from any integer between 0 and 10;
Lg, L¹, L², L³, D, R^{m2}, and Rⁿ² are as defined in any of the embodiments of the present application.

In another aspect, the present application provides a preparation method for a compound of formula II or a salt thereof, 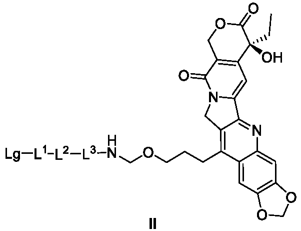 comprising step i) reacting a compound of formula III or a salt thereof with a compound of formula X-3 or a salt thereof to obtain a compound of formula II or a salt thereof, 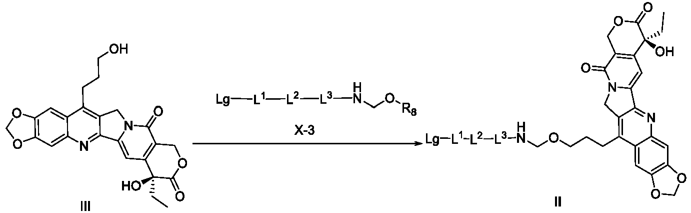 wherein Lg, L¹, L², L³, and R₈ are as defined in any of the embodiments of the present application.

In some embodiments, in step i), the compound of formula X-3 or the salt thereof is selected from the following structure: 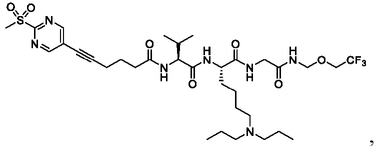 and correspondingly, the compound of formula II or the salt thereof is selected from the following structure: 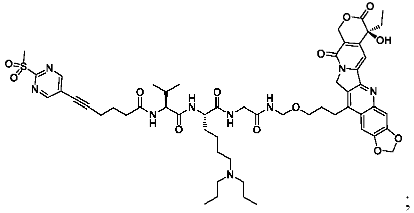 that is, step i) involves the following reaction: reacting the compound of formula III or the salt thereof with a compound of formula X-3a or a salt thereof to obtain a compound of formula IIa or a salt thereof,

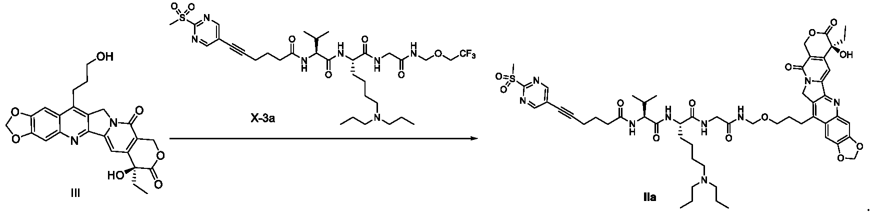

In another aspect, the present application provides a preparation method for a compound of formula X-3 or a salt thereof, which comprises the following steps:
m-1) reacting a compound of formula IV-2 or a salt thereof to obtain a compound of formula X-3-1 or a salt thereof; and 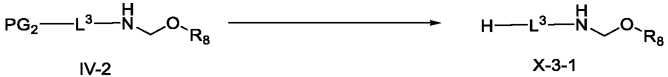
m-2) reacting the compound of formula X-3-1 or the salt thereof with a compound of formula X-1 to obtain the compound of formula X-3 or a salt thereof; 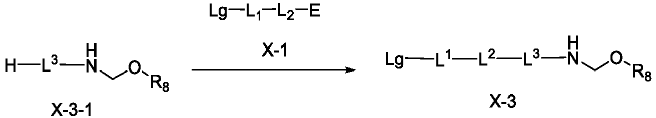
wherein E is selected from hydroxy, halogen, activated hydroxy, such as hydroxy, chlorine, bromine, 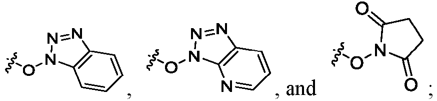
PG₂ is selected from an amino protecting group;
Lg, L¹, L², L³, and R₈ are as defined in any of the embodiments of the present application.

In some embodiments, in steps m-1 and m-2), the compound of formula IV-2 or the salt thereof is selected from the following structure: 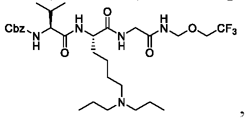 correspondingly, the compound of formula X-3-1 or the salt thereof is selected from the following structure: 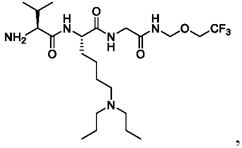 and correspondingly, the compound of formula X-3 or the salt thereof is selected from the following structure: 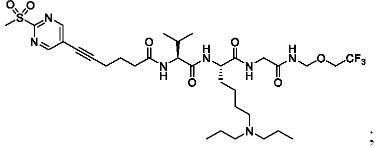 that is, step m-1) involves the following reaction: removing a protecting group Cbz from a compound of formula IV-2-A or a salt thereof in a metal reagent-hydrogen system to obtain a compound of formula X-3-1-A or a salt thereof, 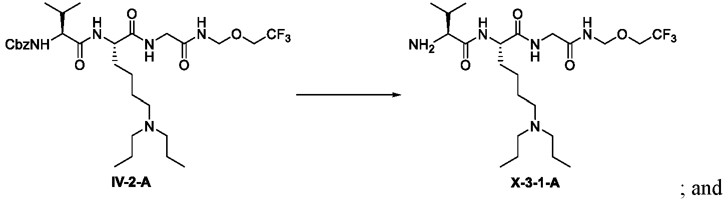 step m-2) involves the following reaction: reacting the compound of formula X-3-1-A or the salt thereof with a compound of formula X-1-A under an alkaline condition to obtain a compound of formula X-3a or a salt thereof,

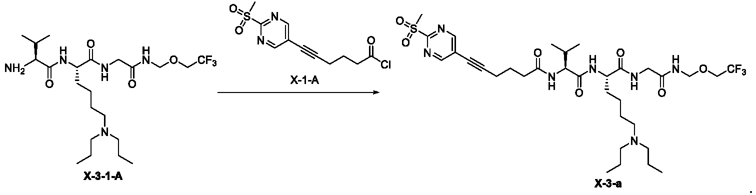

In another aspect, the present application provides a preparation method for a compound of formula IV-2 or a salt thereof,
when R₈ is not H, the method comprises the following steps:
k-1) reacting a compound of formula IV-1 or a salt thereof to obtain a compound of formula IV-2-1 or a salt thereof; and 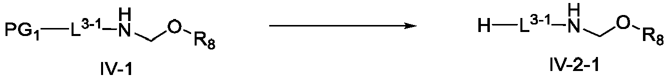
k-2) reacting the compound of formula IV-2-1 or the salt thereof with a compound of formula IV-2-2 or a salt thereof to obtain a compound of formula IV-2 or a salt thereof; 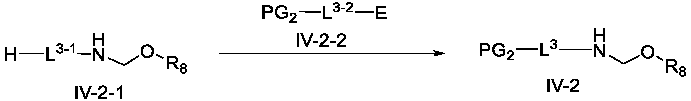
wherein PG₂-L³ is PG₂-L³⁻²-L³⁻¹;
when R₈ is H, IV-2 is IV-2a, and the method comprises the following method I or method II:
   method I, comprising the following step:
   k-3) reacting a compound of formula IV-2-3 or a salt thereof to obtain a compound of formula IV-2a or a salt thereof; 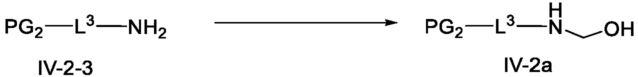
   method II, comprising the following steps:
      k-4) reacting a compound of formula IV-2-4 or a salt thereof to obtain a compound of formula IV-2-5 or a salt thereof; 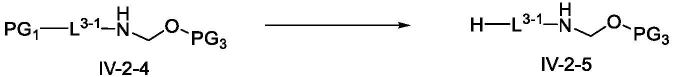
      k-5) reacting the compound of formula IV-2-5 or the salt thereof with a compound of formula IV-2-2 or a salt thereof to obtain a compound of formula IV-2-6 or a salt thereof; and 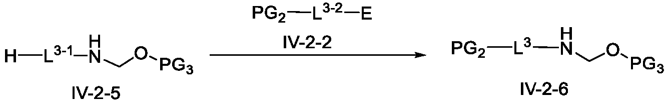
      k-6) reacting the compound of formula IV-2-6 or the salt thereof to obtain a compound of formula IV-2a or a salt thereof; 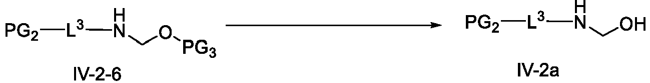
      in the method II, PG₂-L³ is PG₂-L³⁻²-L³⁻¹;
      wherein,
      L³⁻¹ is selected from an amino acid residue or a short peptide consisting of 2-9 amino acid residues; the amino acid residue is selected from a natural amino acid residue, a non-natural amino acid residue, or an amino acid residue represented by AA¹, or a stereoisomer thereof;
      PG₁ is selected from an amino protecting group;
      L³⁻² is selected from an amino acid residue or a short peptide consisting of 2-9 amino acid residues; the amino acid residue is selected from a natural amino acid residue, a non-natural amino acid residue, or an amino acid residue represented by AA¹, or a stereoisomer thereof;
      L³, PG₂, R₈, and E are as defined in any of the embodiments of the present application;
      PG₃ is a hydroxy protecting group, such as benzyl, substituted benzyl, or allyl.

In some embodiments, in steps k-1) and k-2), the compound of formula IV-1 or the salt thereof is selected from the following structure: 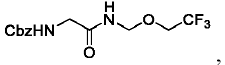 the compound of formula IV-2-2 or the salt thereof is selected from the following structure: 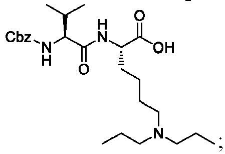 correspondingly, the compound of formula IV-2-1 or the salt thereof is selected from the following structure: 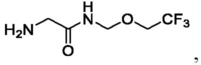 and correspondingly, the compound of formula IV-2 or the salt thereof is selected from the following structure: 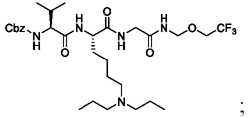 that is, step k-1) involves the following reaction: removing a protecting group Cbz from a compound of formula IV-1-A or a salt thereof in a metal reagent-hydrogen system to obtain a compound of formula IV-2-1-A or a salt thereof, 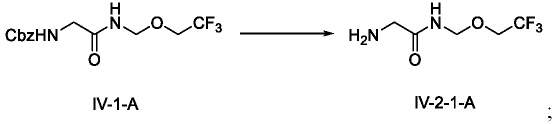 and
step k-2) involves the following reaction: reacting the compound of formula IV-2-1-A or the salt thereof with a compound of formula IV-2-2-B or a salt thereof under the action of a condensing agent and/or an anti-racemization reagent and under an alkaline or neutral condition to obtain a compound of formula IV-2-A or a salt thereof,

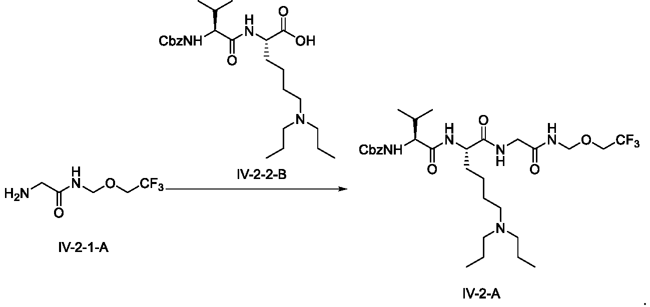

In another aspect, the present application provides two preparation methods for a compound of formula IV-1 or a salt thereof, which comprises the following method I or method II:
the method I comprises step j) reacting a compound of formula IV-1-1 or a salt thereof with a compound of formula R₈OH or a salt thereof to obtain a compound of formula IV-1 or a salt thereof; 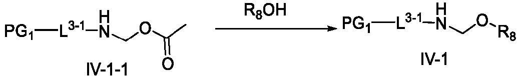
method II, comprising the following steps:
   j-1) reacting a compound of formula IV-1-2 or a salt thereof to obtain a compound of formula IV-1-3 or a salt thereof; and 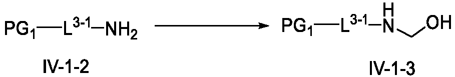
   j-2) reacting the compound of formula IV-1-3 or the salt thereof to obtain a compound of formula IV-1 or a salt thereof; 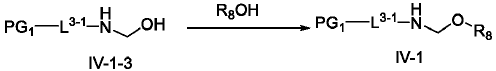
   wherein L³⁻¹, PG₁, and R₈ are as defined in any of the embodiments of the present application.

In some embodiments, in steps j-1) and j-2), the compound of formula IV-1-2 or the salt thereof is selected from the following structure: 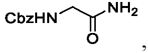 correspondingly, the compound of formula IV-1-3 or the salt thereof is selected from the following structure: 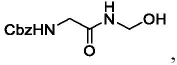 and correspondingly, the compound of formula IV-1 or the salt thereof is selected from the following structure: 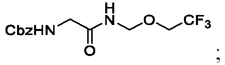 that is, step j-1) involves the following reaction: reacting a compound of formula IV-1-A-2 or a salt thereof with formaldehyde in water and under an alkaline condition to obtain a compound of formula IV-1-A-3 or a salt thereof; and 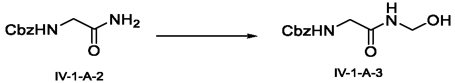 step j-2) involves the following reaction: reacting the compound of formula IV-1-A-3 or the salt thereof with CF₃CH₂OH under an acidic condition to obtain a compound of formula IV-1-A or a salt thereof;

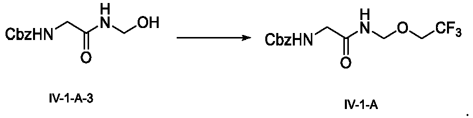

In some embodiments, L¹ is selected from 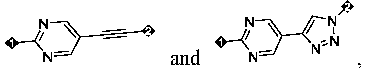 wherein position 1 is attached to Lg, and position 2 is attached to L₂.

In some embodiments, L¹ is 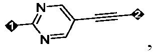 wherein position 1 is attached to Lg, and position 2 is attached to L².

In some embodiments, L¹ is selected from 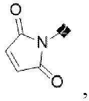 and position 2 is attached to L².

In some embodiments, L² is selected from 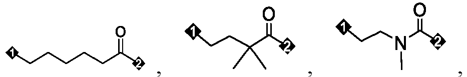 and 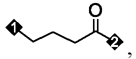 wherein position 1 is attached to L¹, and position 2 is attached to L³.

In some embodiments, L² is selected from 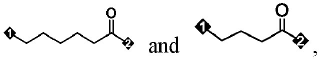 wherein position 1 is attached to L¹, and position 2 is attached to L³.

In some embodiments, L² is 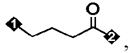 wherein position 1 is attached to L¹, and position 2 is attached to L³.

In some embodiments, Y is -CH₂-.

In some embodiments, n4 is selected from 0, 1, 2, and 3.

In some embodiments, Z is selected from -CH₂-, -C(CH₃)₂-, -N(CH₃)-, and -NH-.

In some embodiments, R^{m} and Rⁿ are each independently selected from H, C1-4 alkyl, C2-4 alkenyl, C2-4 alkynyl, C3-6 cycloalkyl, and 3-6 membered heterocyclyl;
or R^{m} and Rⁿ, together with the carbon atom to which they are both attached, form a 3-6 membered carbocyclic ring or a 3-6 membered heterocyclic ring.

In some embodiments, R^{m} and Rⁿ are each independently selected from H and Me.

In some embodiments, L¹-L² is selected from 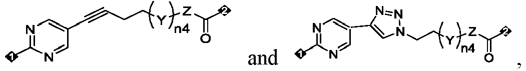 wherein position 1 is attached to Lg, and position 2 is attached to L³.

In some embodiments, L¹-L² is selected from 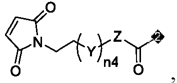 wherein position 2 is attached to L³.

In some embodiments, L¹-L² is selected from 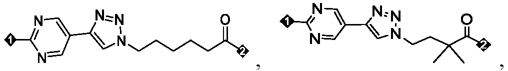 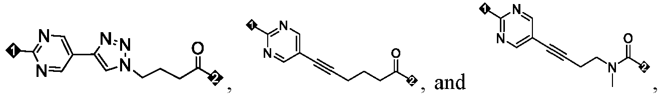 wherein position 1 is attached to Lg, and position 2 is attached to L³; and may also be selected from 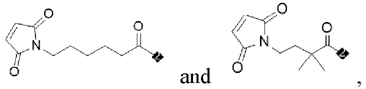 wherein position 2 is attached to L³.

In some embodiments, L¹-L² is selected from 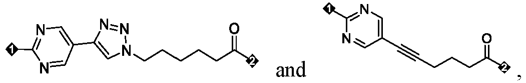 wherein position 1 is attached to Lg, and position 2 is attached to L³; and may also be selected from 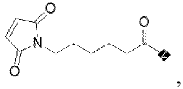 wherein position 2 is attached to L³.

In some embodiments, L¹-L² is 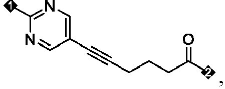 wherein position 1 is attached to Lg, and position 2 is attached to L³.

In some embodiments, L³ is selected from a short peptide consisting of 2-4 amino acid residues, such as dipeptide, tripeptide, or tetrapeptide; the amino acid residue is selected from a natural amino acid residue, a non-natural amino acid residue, or an amino acid residue represented by AA¹, or a stereoisomer thereof.

In some embodiments, L³ is selected from Val-Ala, AA¹-Val-Ala, Val-AA¹-Gly, Ala-AA'-Gly, Gly-AA¹-Gly, Val-AA¹-Ala, Val-AA¹-Val, Ala-AA'-Ala, Ala-AA'-Val, Gly-AA¹-Ala, Gly-AA¹-Val, Ala-Ala-Ala, Ala-Ala-Asn, Gly-Gly-Phe-Gly, and Gly-Gly-Val-Ala.

In some embodiments, L³ is selected from Val-AA¹-Gly, Ala-AA¹-Gly, Gly-AA¹-Gly, Val-AA¹-Ala, Val-AA¹-Val, Ala-AA¹-Ala, Ala-AA¹-Val, Gly-AA¹-Ala, Gly-AA¹-Val, Ala-Ala-Ala, Ala-Ala-Asn, and Gly-Gly-Phe-Gly.

In some embodiments, L³ is selected from Ala-Ala-Ala, Ala-Ala-Asn, Val-AA¹-Gly, and Gly-Gly-Phe-Gly. In some embodiments, L³ is Val-AA¹-Gly.

In some embodiments, L³ is selected from 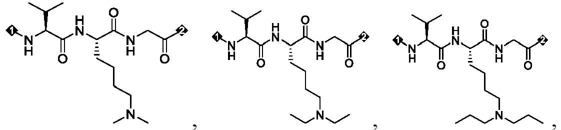 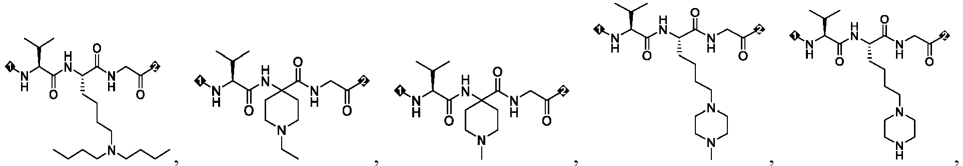 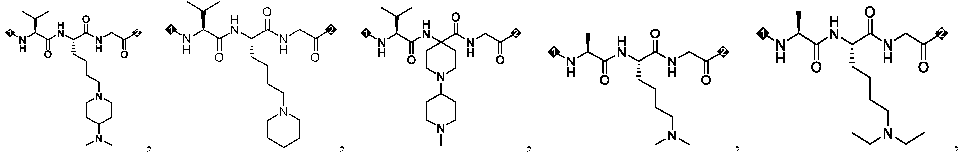 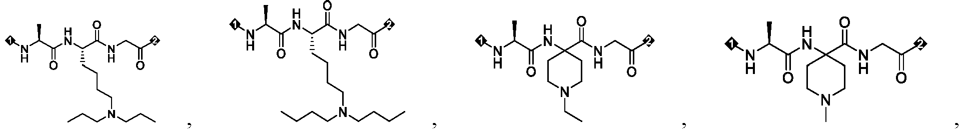 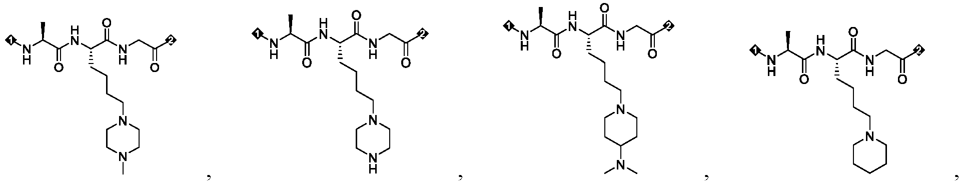 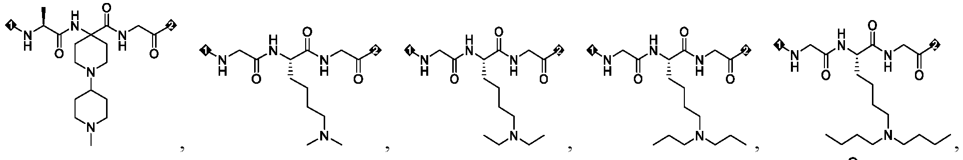 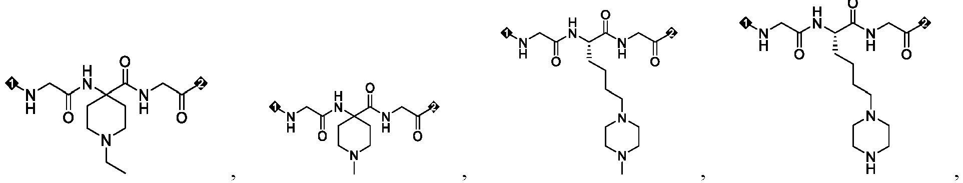 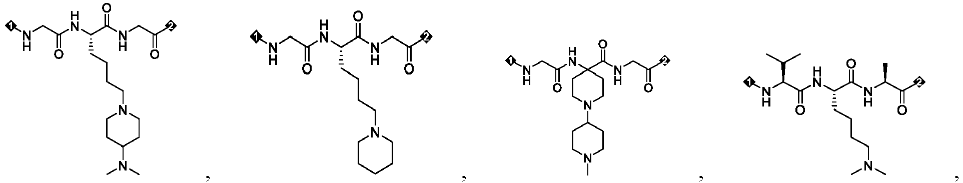 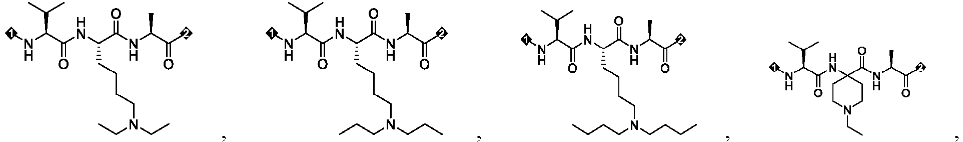 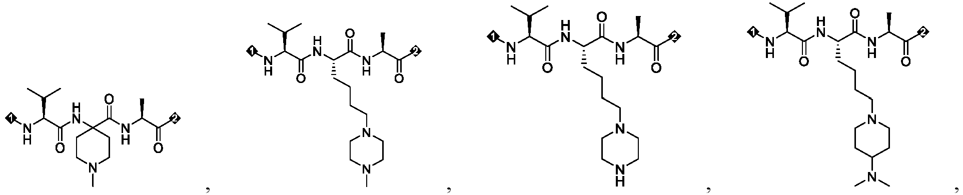 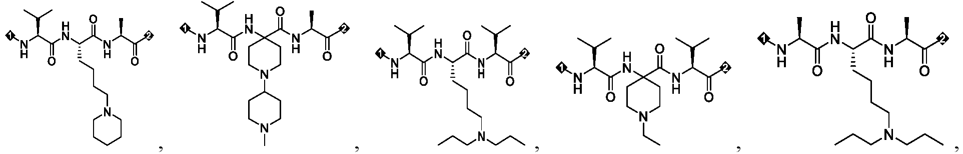 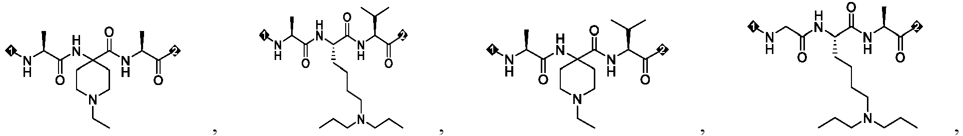 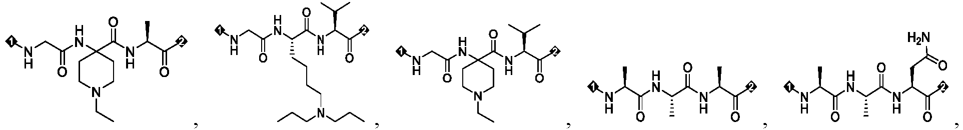 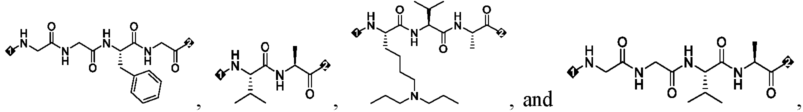 wherein position 1 is attached to L², and position 2 is attached to NH.

In some embodiments, L³ is selected from 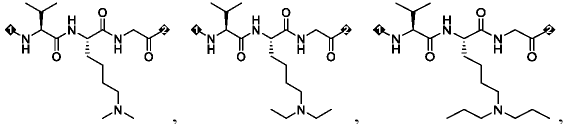 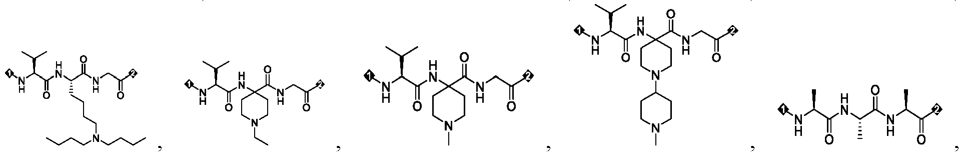 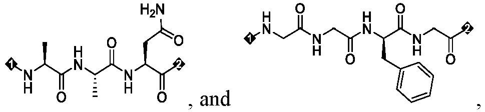 wherein position 1 is attached to L², and position 2 is attached to NH.

In some embodiments, L³ is 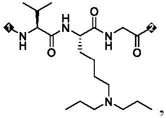 wherein position 1 is attached to L², and position 2 is attached to NH.

In some embodiments, one of R^{a} and R^{b} is H, and the other is

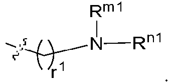

In some embodiments, R^{a} and R^{b}, together with the carbon atom to which they are both attached, form a 5-6 membered heterocyclic ring substituted with R⁰.

In some embodiments, R^{a} and R^{b}, together with the carbon atom to which they are both attached, form a piperidine ring or a piperazine ring substituted with R⁰.

In some embodiments, R^{a} and R^{b}, together with the carbon atom to which they are both attached, form a piperidine ring substituted with R⁰.

In some embodiments R^{a} and R^{b}, together with the carbon atom to which they are both attached, form 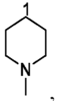 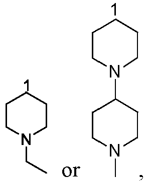 wherein the carbon atom at position 1 is the carbon atom to which R^{a} and R^{b} are both attached.

In some embodiments, r and r¹ are each independently selected from 0, 1, 2, 3, 4 and 5.

In some embodiments, r and r¹ are each independently selected from 0 and 4.

In some embodiments, r is 0, and r¹ is 4.

In some embodiments, R^{m1} and Rⁿ¹ are each independently selected from H and C1-6 alkyl.

In some embodiments, R^{m1} and Rⁿ¹ are each independently selected from H, methyl, ethyl, n-propyl, and *n-*butyl.

In some embodiments, R^{m1} and Rⁿ¹, together with the nitrogen atom to which they are both attached, form a 5-6 membered heterocyclic ring optionally substituted with R^{0'}.

In some embodiments, R^{m1} and Rⁿ¹, together with the nitrogen atom to which they are both attached, form a piperidine ring or a piperazine ring optionally substituted with R^{0'}.

In some embodiments, R^{m1} and Rⁿ¹, together with the nitrogen atom to which they are both attached, form 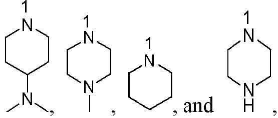 wherein the nitrogen atom at position 1 is the nitrogen atom to which R^{m1} and Rⁿ¹ are both attached.

In some embodiments, R⁰ and R^{0'} are each independently selected from C1-6 alkyl, -NR^{m2}Rⁿ², and 5-6 membered heterocyclyl optionally substituted with C1-6 alkyl.

In some embodiments, R⁰ is selected from C1-6 alkyl and 5-6 membered heterocyclyl substituted with C1-6 alkyl, and the 5-6 membered heterocyclyl is selected from piperidinyl and piperazinyl.

In some embodiments, R⁰ is selected from methyl, ethyl, and 5-6 membered heterocyclyl substituted with methyl, and the 5-6 membered heterocyclyl is piperidinyl.

In some embodiments, R⁰ is selected from methyl, ethyl and

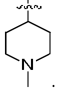

In some embodiments, R^{0'} is selected from C1-6 alkyl and -NR^{m2}Rⁿ².

In some embodiments, R^{0'} is selected from methyl and -NR^{m2}Rⁿ².

In some embodiments, R^{m2} and Rⁿ² are methyl.

In some embodiments, the structure of the amino acid residues represented by AA¹ is shown as follows: 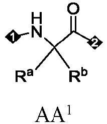 wherein,
one of R^{a} and R^{b} is H, the other is 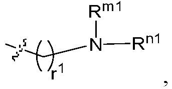 and r¹ is 4;
or R^{a} and R^{b}, together with the carbon atom to which they are both attached, form a 5-6 membered heterocyclic ring substituted with R⁰;
R^{m1} and Rⁿ¹ are each independently selected from H, C1-6 alkyl, and C3-6 cycloalkyl;
R⁰ is selected from C1-6 alkyl, C3-6 cycloalkyl, -NR^{m2}Rⁿ², and 5-6 membered heterocyclyl optionally substituted with C1-6 alkyl;
R^{m2} and Rⁿ² are each independently selected from H and C1-6 alkyl.

In some embodiments, the amino acid residue represented by AA¹ is selected from

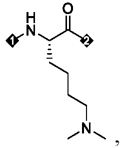

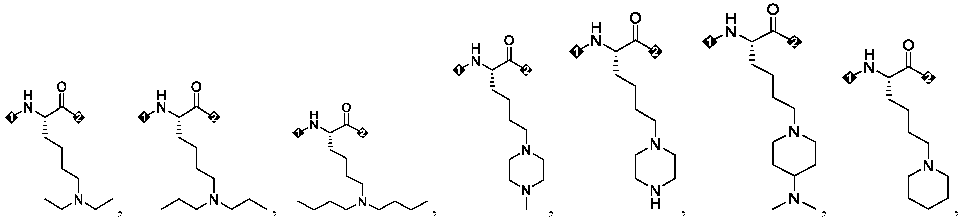

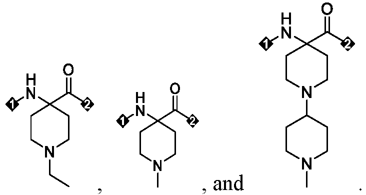

In some embodiments, the amino acid residue represented by AA¹ is selected from

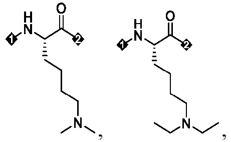

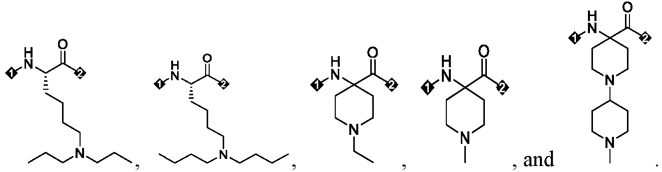

In some embodiments, the amino acid residue represented by AA¹ is

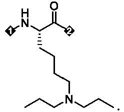

In some embodiments, L³⁻¹ is selected from an amino acid residue, and the amino acid residue is selected from 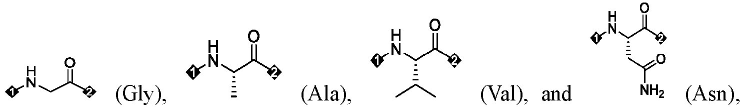 wherein position 1 is attached to L³⁻², and position 2 is attached to NH.

In some embodiments, L³⁻¹ is selected from an amino acid residue, and the amino acid residue is selected from 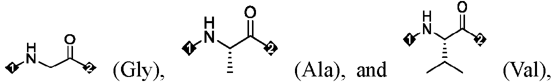 wherein position 1 is attached to L³⁻², and position 2 is attached to NH.

In some embodiments, L³⁻¹ is Gly.

In some embodiments, L³⁻² is selected from an amino acid residue or a short peptide consisting of 2-3 amino acid residues; the amino acid residue is selected from a natural amino acid residue, a non-natural amino acid residue, or an amino acid residue represented by AA¹, or a stereoisomer thereof.

In some embodiments, L³⁻² is selected from Val, Val-AA¹, Ala-AA¹, Gly-AA¹, Ala-Ala, AA¹-Val, Gly-Gly-Val, and Gly-Gly-Phe.

In some embodiments, L³⁻² is selected from Val-AA¹, Ala-AA¹, Gly-AA¹, Ala-Ala, and Gly-Gly-Phe.

In some embodiments, L³⁻² is selected from Val-AA¹, Ala-Ala, and Gly-Gly-Phe.

In some embodiments, L³⁻² is Val-AA¹.

In some embodiments, L³⁻² is selected from 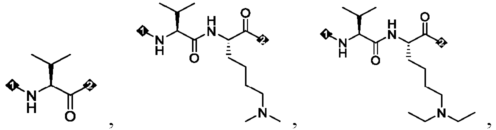 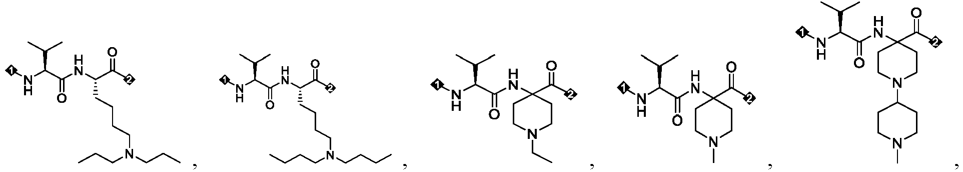 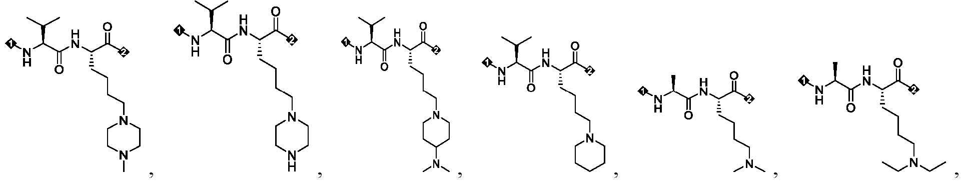 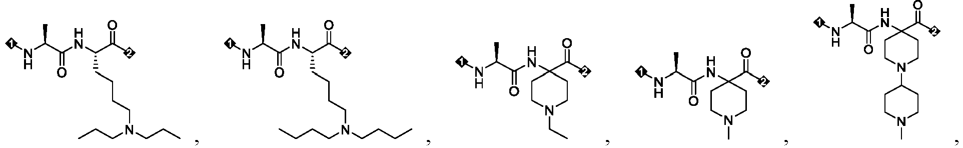 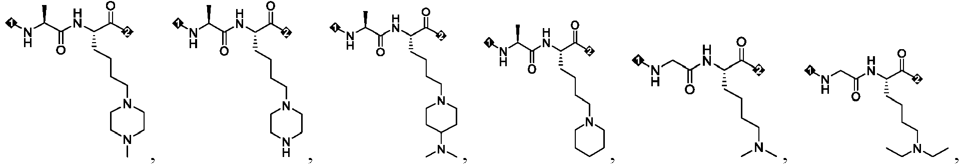 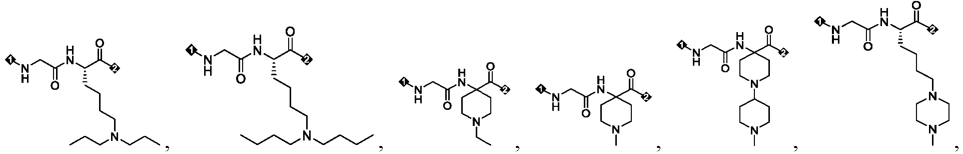 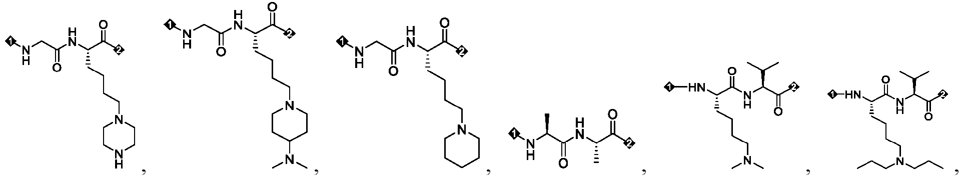 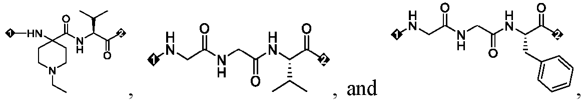 wherein position 1 is attached to L², and position 2 is attached to L³⁻¹.

In some embodiments, L³⁻² is selected from 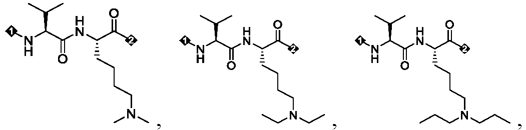 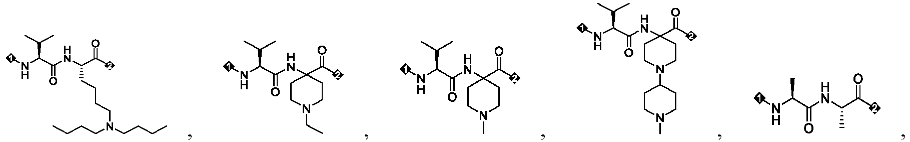 and 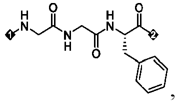 wherein position 1 is attached to L², and position 2 is attached to L³⁻¹.

In some embodiments, L³⁻² is 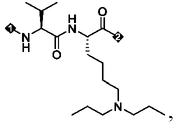 wherein position 1 is attached to L², and position 2 is attached to L³⁻¹.

In some embodiments, PG₁ and PG₂ are each independently selected from benzyloxycarbonyl (Cbz), *tert-*butoxycarbonyl (Boc), 9-fluorenylmethoxycarbonyl (Fmoc), allyloxycarbonyl (Alloc), trimethylsilylethoxycarbonyl (Teoc), methoxycarbonyl, or ethoxycarbonyl.

In some embodiments, PG₁ and PG₂ are each independently selected from benzyloxycarbonyl (Cbz), 9-fluorenylmethoxycarbonyl (Fmoc), and allyloxycarbonyl (Alloc).

In some embodiments, PG₁ and PG₂ are 9-fluorenylmethoxycarbonyl (Fmoc).

In some embodiments, PG₁ and PG₂ are benzyloxycarbonyl (Cbz).

In some embodiments, R₈ is selected from hydrogen, C1-30 alkyl, C3-7 cycloalkyl, 3-20 membered heterocyclyl, C1-6 alkyl-C3-6 cycloalkyl, C1-6 alkyl-4-6 membered heterocyclyl, C1-6 alkyl-C5-10 heteroaryl, and C1-6 alkyl-C6-10 aryl, and the alkyl, cycloalkyl, heterocyclyl, or aryl is optionally substituted with one or more R^{x}.

In some embodiments, R₈ is selected from hydrogen, C1-30 alkyl (e.g., C1-6 alkyl), and C1-6 alkyl-C6-10 aryl, and the alkyl or aryl is optionally substituted with one or more R^{x}.

In some embodiments, R₈ is selected from hydrogen, methyl, ethyl, isopropyl, *tert-butyl,* 2,2,2-trifluoroethyl, 2,2,3,3,3-pentafluoro-1-propyl, and benzyl.

In some embodiments, R₈ is selected from hydrogen, isopropyl, *tert-butyl,* 2,2,2-trifluoroethyl, 2,2,3,3,3-pentafluoro-1-propyl, and benzyl.

In some embodiments, R^{x} is selected from hydrogen, deuterium, fluoro, chloro, bromo, methyl, methoxy, amino, dimethylamino, nitro, cyano, and azido.

In some embodiments, R^{x} is selected from hydrogen, fluoro, methyl, and methoxy.

In some embodiments, PG₃ is selected from benzyl substituted with one or more R^{Y}.

In some embodiments, PG₃ is selected from benzyl and*p*-methoxybenzyl.

In some embodiments, R^{Y} is selected from hydrogen, fluoro, chloro, bromo, methyl, methoxy, dimethylamino, and nitro.

In some embodiments, in step i), the compound of formula III or the salt thereof is reacted with the compound of formula X-3 or the salt thereof after azeotropic dehydration to obtain the compound of formula II or the salt thereof.

In some embodiments, in step i), the azeotropic solvent is selected from toluene, xylene, chloroform, acetonitrile, ethyl acetate, and dichloroethane, preferably toluene and xylene.

In some embodiments, in step i), the compound of formula III or the salt thereof is reacted with the compound of formula X-3 or the salt thereof without the addition of an acid to obtain the compound of formula II or the salt thereof, wherein the reaction temperature is selected from 110 °C to 150 °C, preferably 120 °C to 130 °C. In some embodiments, in step i), the compound of formula III or the salt thereof is reacted with the compound of formula X-3 or the salt thereof under an acidic condition to obtain the compound of formula II or the salt thereof.

In some embodiments, in step i), the compound of formula III or the salt thereof is reacted with the compound of formula X-3 or the salt thereof under an acidic condition to obtain the compound of formula II or the salt thereof, wherein the reaction temperature is selected from 15 °C to 40 °C, preferably 20 °C to 35 °C.

In some embodiments, in step i), the acid is a protic or aprotic acid, for example, hydrogen chloride, hydrobromic acid, sulfuric acid, boron trifluoride etherate, p-toluenesulfonic acid, pyridinium p-toluenesulfonate, zinc acetate, aluminum trichloride (AlCl₃), ferric trichloride (FeCl₃), ytterbium(III) trifluoromethanesulfonate (Yb(OTf)₃), triethylamine hydrochloride, boron trifluoride acetonitrile, boron trifluoride tetrahydrofuran, pyridine hydrochloride, and pyridine hydrobromide; preferably, hydrogen chloride, hydrobromic acid, sulfuric acid, trifluoroacetic acid, p-toluenesulfonic acid, pyridinium p-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); for another example, hydrogen chloride or boron trifluoride etherate; more preferably, hydrogen chloride or sulfuric acid; most preferably, boron trifluoride etherate.

In some embodiments, in step i), a molar ratio of the compound of formula III or the salt thereof to the acid selected for the reaction is selected from 1:0.2-1:6, preferably 1:2-1:5, for example, 1:2.5, 1:3, or 1:5.

In some embodiments, in step i), a molar ratio of the compound of formula III or the salt thereof to the compound of formula X-3 or the salt thereof is selected from 1:1-1:5, preferably 1:2-1:3.

In some embodiments, in step i), a reaction solvent is selected from one or any combination of ethers, nitriles, amides, sulfones or water; preferably, amides or sulfones; more preferably, N,N-dimethylformamide (DMF), N,N-dimethylacetamide (DMAc), N-methylpyrrolidone (NMP), or dimethylsulfoxide (DMSO); further preferably, N,N-dimethylformamide.

In some embodiments, in step i), a mass-to-volume ratio (g/mL) of the compound of formula III or the salt thereof to the selected solvent is selected from 1:2-1:10, preferably 1:2.5-1:7.

In some embodiments, in step i), the reaction temperature is selected from 0 °C to 80 °C; preferably 15 °C to 40 °C; more preferably 25 °C to 35 °C.

In some embodiments, in step j), the compound of formula IV-1-1 or the salt thereof is reacted with the compound of formula R₈OH or the salt thereof under an acidic condition or an alkaline condition to obtain the compound of formula IV-1 or the salt thereof.

In some embodiments, in step j), the acid is a protic or aprotic acid, preferably, hydrogen chloride, hydrobromic acid, sulfuric acid, trifluoroacetic acid, p-toluenesulfonic acid, pyridinium p-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); more preferably, hydrogen chloride, sulfuric acid, or zinc acetate, for example, hydrogen chloride.

In some embodiments, in step j), the base is an organic or inorganic base, preferably sodium hydroxide, potassium tert-butoxide, potassium carbonate, triethylamine (Et₃N), *N,N-diisopropylethylamine* (DIPEA), and pyridine; more preferably, potassium tert-butoxide.

In some embodiments, in step j), a molar ratio of the compound of formula IV-1-1 or the salt thereof to the acid selected for the reaction is selected from 1:0.01-1:0.2, for example, 1:0.05.

In some embodiments, in step j), a molar ratio of the compound of formula IV-1-1 or the salt thereof to the base selected for the reaction is selected from 1:0.9-1:5.

In some embodiments, in step j), a molar ratio of the compound of formula IV-1-1 or the salt thereof to the compound of formula R₈OH or the salt thereof is selected from 1:1-1:20, preferably 1:2-1:15.

In some embodiments, in step j), the reaction solvent is selected from alcohols, ethers, haloalkanes, aromatic hydrocarbons, nitriles, amides, or sulfones; more preferably, isopropanol, benzyl alcohol, tert-butanol, 2,2,2-trifluoroethanol, 2,2,3,3,3-pentafluoro-1-propanol, ethylene glycol diethyl ether, tetrahydrofuran, 1,4-dioxane, dichloromethane, 1,2-dichloroethane, toluene, acetonitrile, DMF, DMAc, NMP, or DMSO; further preferably, isopropanol, benzyl alcohol, tert-butanol, 2,2,2-trifluoroethanol, 2,2,3,3,3-pentafluoro-1-propanol, toluene, tetrahydrofuran, acetonitrile, DMF, DMAc, or NMP, for example, 2,2,2-trifluoroethanol or DMF.

In some embodiments, in step j), a mass-to-volume ratio (g/mL) of the compound of formula IV-1-1 or the salt thereof to the selected solvent is selected from 1:2-1:10, preferably 1:2.5-1:7.

In some embodiments, in step j), the reaction temperature is selected from 0 °C to 120 °C, for example, 10 °C to 50 °C.

In some embodiments, in step j-1), the compound of formula IV-1-2 or the salt thereof is reacted with formaldehyde in water and under an alkaline condition to obtain the compound of formula IV-1-3 or the salt thereof.

In some embodiments, in step j-1), the base is selected from potassium carbonate, potassium bicarbonate, potassium phosphate, sodium carbonate, sodium bicarbonate, triethylamine, diisopropylethylamine, etc., preferably potassium carbonate.

In some embodiments, in step j-1), a molar ratio of the compound of formula IV-1-2 or the salt thereof to the base is 1:0.05-1:1, preferably 1:0.1-1:0.5.

In some embodiments, in step j-1), a molar ratio of the compound of formula IV-1-2 or the salt thereof to formaldehyde is 1:0.5-1:2.5, preferably 1:1-1:2, for example, 1:1.8.

In some embodiments, in step j-1), a mass-to-volume ratio (g/mL) of the compound of formula IV-1-2 or the salt thereof to water is 1:3-1:50, preferably 1:5-1:20, for example, 1:15.

In some embodiments, in step j-1), the reaction temperature is 0 °C to 80 °C, preferably 10 °C to 50 °C.

In some embodiments, in step j-2), the compound of formula IV-1-3 or the salt thereof is reacted with the compound of formula R₈OH or the salt thereof under an acidic condition to obtain the compound of formula IV-1 or the salt thereof.

In some embodiments, in step j-2), the acid is a protic or aprotic acid, preferably hydrogen chloride, hydrobromic acid, sulfuric acid, trifluoroacetic acid, p-toluenesulfonic acid, pyridinium p-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); more preferably hydrogen chloride, sulfuric acid, zinc acetate, for example, hydrogen chloride.

In some embodiments, in step j-2), a molar ratio of the compound of formula IV-1-3 or the salt thereof to the acid selected for the reaction is selected from 1:0.01-1:0.2, for example, 1:0.05.

In some embodiments, in step j-2), a molar ratio of the compound of formula IV-1-3 or the salt thereof to the compound of formula R₈OH or the salt thereof is selected from 1:1-1:50, preferably 1:2-1:30.

In some embodiments, in step j-2), the reaction solvent is selected from alcohols, ethers, haloalkanes, aromatic hydrocarbons, nitriles, amides, or sulfones; more preferably, isopropanol, benzyl alcohol, tert-butanol, 2,2,2-trifluoroethanol, 2,2,3,3,3-pentafluoro-1-propanol, ethylene glycol diethyl ether, tetrahydrofuran, 1,4-dioxane, dichloromethane, 1,2-dichloroethane, toluene, acetonitrile, DMF, DMAc, NMP, or DMSO; further preferably, isopropanol, benzyl alcohol, tert-butanol, 2,2,2-trifluoroethanol, 2,2,3,3,3-pentafluoro-1-propanol, toluene, tetrahydrofuran, acetonitrile, DMF, DMAc, or NMP, for example, 2,2,2-trifluoroethanol or DMF.

In some embodiments, in step j-2), a mass-to-volume ratio (g/mL) of the compound of formula IV-1-3 or the salt thereof to the selected solvent is selected from 1:2-1: 10, preferably 1:2.5-1:7.

In some embodiments, in step j-2), the reaction temperature is selected from 0 °C to 120 °C, for example, 0 °C to 50 °C.

In some embodiments, in step k-1), the protecting group PG₁ on the amino group is removed from the compound of formula IV-1 or the salt thereof to obtain the compound of formula IV-2-1 or the salt thereof.

In some embodiments, in step k-1), the reaction of removing the protecting group PG₁ on the amino group from the compound of formula IV-1 or the salt thereof may be performed using conventional reaction conditions well known to those skilled in the art, for example: the removal is carried out under an acidic condition, wherein the acid comprises a protic acid and an aprotic acid; the removal is carried out under an alkaline condition, wherein the base comprises an organic base and an inorganic base; the removal is carried out under a condition of a metal reagent, wherein the metal reagent is preferably selected from palladiums or platinums, further preferably palladium on carbon, platinum on activated carbon, platinum dioxide, and palladium hydroxide.

In some embodiments, in step k-1), when the PG₁ is 9-fluorenylmethoxycarbonyl (Fmoc), the protecting group PG₁ is removed from the compound of formula IV-1 or the salt thereof under an alkaline condition. The base is selected from organic or inorganic bases, preferably piperidine, diethylamine, morpholine, diisopropylamine, DBU, triethylamine, and *N,N*-diisopropylethylamine; more preferably, piperidine, diethylamine or morpholine, for example, diethylamine or DBU.

In some embodiments, in step k-1), when the PG₁ is 9-fluorenylmethoxycarbonyl (Fmoc), a molar ratio of the compound of formula IV-1 or the salt thereof to the selected base is selected from 1:0.2-1:40; for example, 1:0.2-1:2; preferably 1:1-1:10, for example, 1:0.4.

In some embodiments, in step k-1), when the PG₁ is 9-fluorenylmethoxycarbonyl (Fmoc), the reaction solvent is selected from one or any combination of DMF, DMAc, NMP, dichloromethane, tetrahydrofuran, 1,4-dioxane, and acetonitrile, for example, DMF or tetrahydrofuran.

In some embodiments, in step k-1), when the PG₁ is 9-fluorenylmethoxycarbonyl (Fmoc), a mass-to-volume ratio (g/mL) of the compound of formula IV-1 or the salt thereof to the selected reaction solvent is selected from 1:5-1:50, preferably 1:6-1:20.

In some embodiments, in step k-1), when the PG₁ is 9-fluorenylmethoxycarbonyl (Fmoc), the reaction temperature is selected from 0 °C to 50 °C, preferably 15 °C to 30 °C.

In some embodiments, in step k-1), when the PG₁ is benzyloxycarbonyl (Cbz), the protecting group PG₁ is removed from the compound of formula IV-1 or the salt thereof in the metal reagent-hydrogen system. The metal reagent is selected from palladiums or platinums, preferably palladium on carbon, platinum on activated carbon, platinum dioxide, and palladium hydroxide, for example, palladium on carbon or palladium hydroxide. In some embodiments, in step k-1), when the PG₁ is benzyloxycarbonyl (Cbz), the mass ratio of the compound of formula IV-1 to the metal reagent is 1:0.01-1, preferably 1:0.03-0.5, for example, 1:0.05 or 1:0.1.

In some embodiments, in step k-1), when the PG₁ is benzyloxycarbonyl (Cbz), the hydrogen used is at 1 atm to 50 atm.

In some embodiments, in step k-1), when the PG₁ is benzyloxycarbonyl (Cbz), the reaction temperature is 0 °C to 100 °C, preferably 20 °C to 70 °C.

In some embodiments, in step k-1), when the PG₁ is benzyloxycarbonyl (Cbz), the reaction solvent is selected from any one of alcohols, ethers, esters, amides, or water or a mixture thereof at any ratio, preferably methanol, ethanol, tetrahydrofuran, ethyl acetate, DMF, DMAc, NMP, or water, for example, methanol or tetrahydrofuran.

In some embodiments, in step k-1), when the PG₁ is benzyloxycarbonyl (Cbz), a mass-to-volume ratio (g/mL) of the compound of formula IV-1 or the salt thereof to the selected reaction solvent is selected from 1:3-1:20, preferably 1:5-1:10.

In some embodiments, in step k-1), when the PG₁ is allyloxycarbonyl (Alloc), the protecting group PG₁ is removed from the compound of formula IV-1 or the salt thereof under the condition of a metal reagent. The metal reagent is selected from palladiums, preferably tetrakis(triphenylphosphine)palladium(0) and trans-bis(triphenylphosphine)palladium dichloride.

In some embodiments, in step k-2), the compound of formula IV-2-1 or the salt thereof and the compound of formula IV-2-2 or the salt thereof are subjected to a condensation reaction to obtain the compound of formula IV-2.

In some embodiments, in step k-2), when E is hydroxy, the reaction is carried out under the action of a condensing agent and/or an anti-racemization reagent and under an alkaline or neutral condition; the condensing agent is well known to those skilled in the art, preferably DMTMM, HATU, HBTU, EDCI, COMU, *N*-ethynyl-*N*-methylmethanesulfonamide, EEDQ, and T₃P; more preferably DMTMM or HBTU.

In some embodiments, in step k-2), when E is hydroxy, the anti-racemization reagent is selected from HOAt and HOBt.

In some embodiments, in step k-2), when E is hydroxy, the base is selected from DBU, triethylamine, *N,N-*diisopropylethylamine, and N-methylmorpholine.

In some embodiments, in step k-2), when E is hydroxy, a molar ratio of the compound of formula IV-2-2 or the salt thereof to the condensing agent is selected from 1:1-1:5, preferably 1:1-1:1.5.

In some embodiments, in step k-2), when E is hydroxy, a molar ratio of the compound of formula IV-2-1 or the salt thereof to the compound of formula IV-2-2 or the salt thereof is selected from 1:0.8-1:3, preferably 1:0.8-1:1.2.

In some embodiments, in step k-2), when E is hydroxy, the reaction solvent is selected from one or any combination of DMF, acetonitrile, tetrahydrofuran, methanol, dichloromethane, and water, preferably DMF or tetrahydrofuran.

In some embodiments, in step k-2), when E is hydroxy, a mass-to-volume ratio (g/mL) of the compound of formula IV-2-1 or the salt thereof to the selected reaction solvent is selected from 1:5-1:20.

In some embodiments, in step k-2), when E is hydroxy, the reaction temperature is selected from -20 °C to 100 °C; preferably -15 °C to 50 °C; most preferably below -15 °C.

In some embodiments, in step k-2), when E is activated hydroxy, the compound of formula IV-2-1 or the salt thereof is reacted with the compound of formula IV-2-2 or the salt thereof under an alkaline or neutral condition; the base is selected from organic or inorganic bases, preferably triethylamine, *N,N-*diisopropylethylamine, DBU, and N-methylmorpholine.

In some embodiments, in step k-2), when E is activated hydroxy, the reaction solvent is selected from one or any combination of DMF, tetrahydrofuran, and dichloromethane.

In some embodiments, in step k-2), when E is activated hydroxy, a molar ratio of the compound of formula IV-2-1 or the salt thereof to the compound of formula IV-2-2 or the salt thereof is selected from 1:0.8-1:1.2. In some embodiments, in step k-3), the compound of formula IV-2-3 or the salt thereof is reacted with an aldehyde reagent under an alkaline condition to obtain the compound of formula IV-2a or the salt thereof.

In some embodiments, in step k-3), the aldehyde reagent is selected from an aqueous formaldehyde solution, trioxymethylene, and paraformaldehyde.

In some embodiments, in step k-3), the base is an inorganic base, preferably potassium carbonate, sodium carbonate, sodium bicarbonate, potassium bicarbonate, potassium tert-butoxide, potassium phosphate, dipotassium phosphate, and potassium dihydrogen phosphate.

In some embodiments, in step k-3), a molar ratio of the compound of formula IV-2-3 or the salt thereof to the aldehyde reagent is selected from 1:20-1:200, preferably 1:40-1:90.

In some embodiments, in step k-3), the reaction solvent is selected from one or any combination of alcohols, ethers, nitriles, amides, or water; preferably one or any combination of methanol, ethanol, tetrahydrofuran, 1,4-dioxane, acetonitrile, and DMF.

In some embodiments, in step k-3), the reaction temperature is selected from 25 °C to 100 °C, preferably 25 °C to 80 °C.

In some embodiments, in step k-4), the protecting group PG₁ on the amino group is removed from the compound of formula IV-2-4 or the salt thereof to obtain the compound of formula IV-2-5 or the salt thereof. In some embodiments, in step k-4), the reaction of removing the protecting group PG₁ on the amino group from the compound of formula IV-2-4 or the salt thereof may be performed using conventional reaction conditions well known to those skilled in the art, for example: the removal is carried out under an acidic condition; the removal is carried out under an alkaline condition; and the removal is carried out under a condition of a metal reagent.

In some embodiments, in step k-4), when the PG₁ is 9-fluorenylmethoxycarbonyl (Fmoc), the protecting group PG₁ is removed from the compound of formula IV-2-4 or the salt thereof under an alkaline condition. The base is selected from organic or inorganic bases, preferably piperidine, diethylamine, morpholine, diisopropylamine, and DBU; more preferably diethylamine.

In some embodiments, in step k-4), when the PG₁ is 9-fluorenylmethoxycarbonyl (Fmoc), a molar ratio of the compound of formula IV-2-4 or the salt thereof to the selected base is selected from 1:0.2-1:40, preferably 1:1-1:10.

In some embodiments, in step k-4), when the PG₁ is 9-fluorenylmethoxycarbonyl (Fmoc), the reaction solvent is selected from DMF, DMAc, NMP, dichloromethane, tetrahydrofuran, 1,4-dioxane, and acetonitrile, preferably DMF.

In some embodiments, in step k-4), when the PG₁ is 9-fluorenylmethoxycarbonyl (Fmoc), a mass-to-volume ratio (g/mL) of the compound of formula IV-2-4 or the salt thereof to the selected reaction solvent is selected from 1:5-1:50, preferably 1:6-1:20.

In some embodiments, in step k-4), when the PG₁ is 9-fluorenylmethoxycarbonyl (Fmoc), the reaction temperature is selected from 0 °C to 50 °C, preferably 15 °C to 30 °C.

In some embodiments, in step k-4), when the PG₁ is benzyloxycarbonyl (Cbz), the protecting group PG₁ is removed from the compound of formula IV-2-4 or the salt thereof in the metal reagent-hydrogen system. The metal reagent is selected from palladiums or platinums, preferably palladium on carbon, platinum on activated carbon, platinum dioxide, and palladium hydroxide.

In some embodiments, in step k-4), when the PG₁ is allyloxycarbonyl (Alloc), the protecting group PG₁ is removed from the compound of formula IV-2-4 or the salt thereof under the condition of a palladium reagent. The metal reagent is selected from palladiums, preferably tetrakis(triphenylphosphine)palladium(0) and trans-bis(triphenylphosphine)palladium dichloride.

In some embodiments, in step k-5), the compound of formula IV-2-5 or the salt thereof and the compound of formula IV-2-2 or the salt thereof are subjected to a condensation reaction to obtain the compound of formula IV-2-6.

In some embodiments, in step k-5), when E is hydroxy, the reaction is carried out under the action of a condensing agent and/or an anti-racemization reagent and under an alkaline or neutral condition; the condensing agent is well known to those skilled in the art, preferably DMTMM, HATU, HBTU, EDCI, COMU, N-ethynyl-N-methylmethanesulfonamide, EEDQ, and T₃P; more preferably DMTMM or HBTU.

In some embodiments, in step k-5), when E is hydroxy, the anti-racemization reagent is selected from HOAt and HOBt.

In some embodiments, in step k-5), when E is hydroxy, the base is selected from triethylamine, *N,N-*diisopropylethylamine, and *N*-methylmorpholine.

In some embodiments, in step k-5), when E is hydroxy, a molar ratio of the compound of formula IV-2-2 or the salt thereof to the condensing agent is selected from 1:1-1:5, preferably 1:1-1:1.5.

In some embodiments, in step k-5), when E is hydroxy, a molar ratio of the compound of formula IV-2-5 or the salt thereof to the compound of formula IV-2-2 or the salt thereof is selected from 1:0.8-1:1.2.

In some embodiments, in step k-5), when E is hydroxy, the reaction solvent is selected from one or any combination of DMF, acetonitrile, tetrahydrofuran, methanol, dichloromethane, and water, preferably DMF. In some embodiments, in step k-5), when E is hydroxy, a mass-to-volume ratio (g/mL) of the compound of formula IV-2-5 or the salt thereof to the selected reaction solvent is selected from 1:5-1:20.

In some embodiments, in step k-5), when E is hydroxy, the reaction temperature is selected from -20 °C to 100 °C, preferably -15 °C to 50 °C.

In some embodiments, in step k-5), when E is activated hydroxy, the compound of formula IV-2-5 or the salt thereof is reacted with the compound of formula IV-2-2 or the salt thereof under an alkaline or neutral condition; the base is selected from organic or inorganic bases, preferably triethylamine, *N,N-*diisopropylethylamine, and N-methylmorpholine.

In some embodiments, in step k-5), when E is activated hydroxy, the reaction solvent is selected from one or any combination of DMF, acetonitrile, tetrahydrofuran, dichloromethane, and water.

In some embodiments, in step k-5), when E is activated hydroxy, a molar ratio of the compound of formula IV-2-5 or the salt thereof to the compound of formula IV-2-2 or the salt thereof is selected from 1:0.8-1:1.2. In some embodiments, in step k-6), the protecting group on hydroxy is removed from the compound of formula IV-2-6 or the salt thereof under an acidic condition in a metal catalyst-hydrogen system to obtain the compound of formula IV-2a.

In some embodiments, in step k-6), the acid is a protic acid, preferably formic acid, acetic acid, trifluoroacetic acid, or hydrochloric acid.

In some embodiments, in step k-6), a molar ratio of the compound of formula IV-2-6 or the salt thereof to the acid is 1:0.2-1:2.

In some embodiments, in step k-6), the metal catalyst is selected from palladium on carbon, platinum on activated carbon, platinum dioxide, and palladium hydroxide.

In some embodiments, in step k-6), the ratio of the compound of formula IV-2-6 or the salt thereof to the metal catalyst is 0.5%-20%.

In some embodiments, in step k-6), the hydrogen source is selected from hydrogen and formic acid.

In some embodiments, in step k-6), the hydrogen source pressure is selected from 1 atm to 5 atm.

In some embodiments, in step k-6), the reaction solvent is selected from one or any combination of DMF, acetonitrile, tetrahydrofuran, methanol, and ethyl acetate.

In some embodiments, in step m-1), the protecting group PG₂ on the amino group is removed from the compound of formula IV-2 or the salt thereof to obtain the compound of formula X-3-1 or the salt thereof.

In some embodiments, in step m-1), the reaction in which the protecting group PG₂ on the amino group is removed from the compound of formula IV-2 or the salt thereof can be carried out under conventional reaction conditions well known to those skilled in the art, for example: the removal is carried out under an acidic condition, wherein the acid comprises a protic acid and an aprotic acid; the removal is carried out under an alkaline condition, wherein the base comprises an organic base and an inorganic base; the removal is carried out under a condition of a metal reagent, wherein the metal reagent is preferably selected from palladiums or platinums, further preferably palladium on carbon, platinum on activated carbon, platinum dioxide, and palladium hydroxide.

In some embodiments, in step m-1), when the PG₂ is 9-fluorenylmethoxycarbonyl (Fmoc), the protecting group PG₂ is removed from the compound of formula IV-2 or the salt thereof under an alkaline condition. The base is selected from piperidine, diethylamine, morpholine, and DBU, preferably diethylamine.

In some embodiments, in step m-1), when the PG₂ is 9-fluorenylmethoxycarbonyl (Fmoc), a molar ratio of the compound of formula IV-2 or the salt thereof to the selected base is selected from 1:0.2-1:40, preferably 1:1-1:10.

In some embodiments, in step m-1), when the PG₂ is 9-fluorenylmethoxycarbonyl (Fmoc), the reaction solvent is selected from DMF, dichloromethane, tetrahydrofuran, and 1,4-dioxane, preferably DMF.

In some embodiments, in step m-1), when the PG₂ is 9-fluorenylmethoxycarbonyl (Fmoc), a mass-to-volume ratio (g/mL) of the compound of formula IV-2 or the salt thereof to the selected reaction solvent is selected from 1:5-1:50, preferably 1:6-1:20.

In some embodiments, in step m-1), when the PG₂ is 9-fluorenylmethoxycarbonyl (Fmoc), the reaction temperature is selected from 0 °C to 50 °C, preferably 15 °C to 30 °C.

In some embodiments, in step m-1), when the PG₂ is benzyloxycarbonyl (Cbz), the protecting group PG₂ is removed from the compound of formula IV-2 or the salt thereof in the metal reagent-hydrogen system. The metal reagent is palladiums or platinums, preferably palladium on carbon, platinum on activated carbon, platinum dioxide, and palladium hydroxide, for example, palladium on carbon or palladium hydroxide.

In some embodiments, in step m-1), when the PG₂ is benzyloxycarbonyl (Cbz), the mass ratio of the compound of formula IV-2 to the metal reagent is 1:0.01-1, preferably 1:0.03-0.5, for example, 1:0.3.

In some embodiments, in step m-1), when the PG₂ is benzyloxycarbonyl (Cbz), the hydrogen used is at 1 atm to 50 atm, preferably 1 atm to 4 atm.

In some embodiments, in step m-1), when the PG₂ is benzyloxycarbonyl (Cbz), the reaction temperature is 0 °C to 100 °C, preferably 20 °C to 70 °C.

In some embodiments, in step m-1), when the PG₂ is benzyloxycarbonyl (Cbz), the reaction solvent is selected from any one of alcohols, ethers, esters, amides, or water or a mixture thereof at any ratio, preferably methanol, ethanol, tetrahydrofuran, ethyl acetate, DMF, DMAc, NMP, or water, for example, methanol or tetrahydrofuran.

In some embodiments, in step m-1), when the PG₂ is benzyloxycarbonyl (Cbz), the mass-to-volume ratio (g/mL) of the compound of formula IV-2 or the salt thereof to the selected reaction solvent is selected from 1:3-1:50, preferably 1:10-1:30.

In some embodiments, in step m-1), when the PG₂ is allyloxycarbonyl (Alloc), the protecting group PG₂ is removed from the compound of formula IV-2 or the salt thereof under the condition of a metal reagent. The metal reagent is palladiums, preferably tetrakis(triphenylphosphine)palladium(0) and trans-bis(triphenylphosphine)palladium dichloride.

In some embodiments, in step m-2), the compound of formula X-3-1 or the salt thereof and the compound of formula X-1 or the salt thereof are subjected to a condensation reaction to obtain the compound of formula X-3 or the salt thereof.

In some embodiments, in step m-2), when E is hydroxy, the reaction is carried out under the action of a condensing agent and under an alkaline or neutral condition; the condensing agent is well known to those skilled in the art, preferably DMTMM, HATU, HBTU, COMU, or N-ethynyl-N-methylmethanesulfonamide, more preferably DMTMM or HBTU.

In some embodiments, in step m-2), when E is hydroxy, the base is selected from organic or inorganic bases, preferably triethylamine, *N,N-*diisopropylethylamine*,* and N-methylmorpholine, more preferably *N,N-*diisopropylethylamine.

In some embodiments, in step m-2), when E is hydroxy, a molar ratio of the compound of formula X-1 or the salt thereof to the condensing agent is selected from 1:1-1:5, preferably 1:1-1:1.5.

In some embodiments, in step m-2), when E is hydroxy, a molar ratio of the compound of formula X-3-1 or the salt thereof to the compound of formula X-1 or the salt thereof is selected from 1:0.9-1:1.2.

In some embodiments, in step m-2), when E is hydroxy, the reaction solvent is selected from one or any combination of DMF, acetonitrile, tetrahydrofuran, methanol, dichloromethane, and water, preferably DMF. In some embodiments, in step m-2), when E is hydroxy, the reaction temperature is selected from -20 °C to - 100 °C; for example -20 °C to 30 °C; preferably 0 °C to 30 °C.

In some embodiments, in step m-2), when E is selected from halogen (e.g., chlorine, fluorine, or bromine), the compound of formula X-3-1 or the salt thereof is reacted with the compound of formula X-1 or the salt thereof under an alkaline condition. The base is selected from triethylamine, *N,N-diisopropylethylamine,* DBU, and N-methylmorpholine, preferably *N,N-*diisopropylethylamine.

In some embodiments, in step m-2), when E is selected from halogen (e.g., chlorine, fluorine, or bromine), a molar ratio of the compound of formula X-1 or the salt thereof to the base of the reaction is selected from 1:0.1-1:5; for example, 1:1-1:5; preferably 1:0.2-1:1.

In some embodiments, in step m-2), when E is halogen (e.g., chlorine, fluorine, or bromine), a molar ratio of the compound of formula X-1 or the salt thereof to the compound of formula X-3-1 or the salt thereof is selected from 1:0.8-1:2.0, preferably 1:0.9-1:1.2.

In some embodiments, in step m-2), when E is halogen (e.g., chlorine, fluorine, or bromine), the reaction solvent is selected from one or any combination of DMF, tetrahydrofuran, dichloromethane, and acetonitrile, preferably acetonitrile.

In some embodiments, in step m-2), when E is halogen (e.g., chlorine, fluorine, or bromine), a mass-to-volume ratio (g/mL) of the compound of formula X-1 or the salt thereof to the selected reaction solvent is selected from 1:2-1:50, preferably 1:3-1:10.

In some embodiments, in step m-2), when E is halogen (e.g., chlorine, fluorine, or bromine), the reaction temperature is selected from -20 °C to 100 °C, preferably 0 °C to 30 °C.

In some embodiments, in step m-2), when E is activated hydroxy, a molar ratio of the compound of formula X-3-1 or the salt thereof to the compound of formula X-1 or the salt thereof is selected from 1:0.9-1:1.2.

In some embodiments, in step m-2), when E is activated hydroxy, the reaction solvent is selected from one or any combination of DMF, acetonitrile, tetrahydrofuran, and dichloromethane.

In some embodiments, in step m-2), when E is activated hydroxy, the compound of formula X-3-1 or the salt thereof is reacted with the compound of formula X-1 or the salt thereof under an alkaline condition. The base is selected from organic or inorganic bases, preferably pyridine, triethylamine, *N,N-diisopropylethylamine,* and N-methylmorpholine.

In another aspect, the present application provides a preparation method for a compound of formula IV-2-2 or a salt thereof,
q-1) reacting a compound of formula IV-2-2-1 or a salt thereof with a compound of formula IV-2-2-2 or a salt thereof to obtain a compound of formula IV-2-2-3 or a salt thereof; 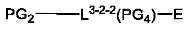 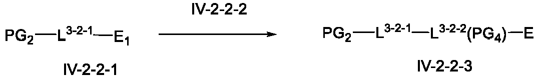
q-2) removing a protecting group from the compound of formula IV-2-2-3 or the salt thereof to obtain a compound of formula IV-2-2-4 or a salt thereof; and 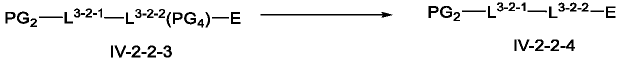
q-3) reacting the compound of formula IV-2-2-4 or the salt thereof to obtain a compound of formula IV-2-2 or a salt thereof; 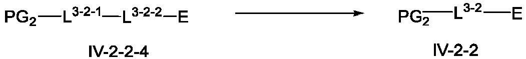
wherein,
L³⁻²⁻¹ and L³⁻²⁻² are each independently selected from an amino acid residue or a short peptide consisting of 2-3 amino acid residues; the amino acid residue is selected from a natural amino acid residue, a non-natural amino acid residue, or an amino acid residue represented by AA¹, or a stereoisomer thereof; E₁ is selected from hydroxy, halogen, activated hydroxy, such as hydroxy, chlorine, bromine, 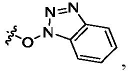 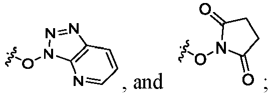
PG₄ is selected from an amino protecting group selected from benzyloxycarbonyl (Cbz), tert-butoxycarbonyl (Boc), 9-fluorenylmethoxycarbonyl (Fmoc), allyloxycarbonyl (Alloc), trimethylsilylethoxycarbonyl (Teoc), methoxycarbonyl, or ethoxycarbonyl; preferably, PG₄ is selected from tert-butoxycarbonyl (Boc), 9-fluorenylmethoxycarbonyl (Fmoc), or allyloxycarbonyl (Alloc); more preferably, PG₄ is tert-butoxycarbonyl (Boc); and PG₄ is different from PG₂;
PG₂ and E are as defined in any of the embodiments of the present application.

In some embodiments, in step q-1), the compound of formula IV-2-2-1 or the salt thereof and the compound of formula IV-2-2-2 or the salt thereof are subjected to a condensation reaction to obtain the compound of formula IV-2-2-3 or the salt thereof.

In some embodiments, in step q-1), when E is activated hydroxy, a molar ratio of the compound of formula IV-2-2-1 or the salt thereof to the compound of formula IV-2-2-2 or the salt thereof is selected from 1:0.9-1:1.2.

In some embodiments, in step q-1), when E is activated hydroxy, the compound of formula IV-2-2-1 or the salt thereof is reacted with the compound of formula IV-2-2-2 or the salt thereof under an alkaline condition. The base is selected from organic or inorganic bases, preferably sodium bicarbonate, potassium bicarbonate, sodium carbonate, potassium carbonate, sodium hydroxide, or potassium hydroxide; further preferably sodium bicarbonate.

In some embodiments, in step q-1), when E is activated hydroxy, a molar ratio of the compound of formula IV-2-2-1 or the salt thereof to the base is selected from 1:1-1:8, preferably 1:1-1:4.

In some embodiments, in step q-1), when E is activated hydroxy, the reaction solvent may be selected from one or any combination of ketones, chloroalkanes, ethers, esters, nitriles, amides, and water; preferably one or any combination of acetone, tetrahydrofuran, ethyl acetate, dichloromethane, methyl *tert-*butyl ether, and water; further preferably a mixed solvent of acetone, tetrahydrofuran, methylene chloride and water.

In some embodiments, in step q-1), when E is activated hydroxy, the reaction temperature is selected from 0 °C to 50 °C, preferably 15 °C to 30 °C.

In some embodiments, in step q-1), when E is hydroxy, the reaction is carried out under the action of a condensing agent and under an alkaline or neutral condition; the condensing agent is well known to those skilled in the art, preferably DMTMM, HATU, HBTU, COMU, or *N*-ethynyl-*N*-methylmethanesulfonamide.

In some embodiments, in step q-1), when E is hydroxy, a molar ratio of the compound of formula IV-2-2-1 or the salt thereof to the condensing agent is selected from 1:1-1:5, preferably 1:1-1:1.5.

In some embodiments, in step q-1), when E is hydroxy, the base is selected from organic or inorganic bases, preferably triethylamine, *N,N*-diisopropylethylamine, and *N*-methylmorpholine.

In some embodiments, in step q-1), when E is hydroxy, a molar ratio of the compound of formula IV-2-2-1 or the salt thereof to the compound of formula IV-2-2-2 or the salt thereof is selected from 1:0.9-1:1.2.

In some embodiments, in step q-1), when E is hydroxy, the reaction solvent is selected from one or any combination of DMF, acetonitrile, tetrahydrofuran, methanol, dichloromethane, and water, preferably DMF. In some embodiments, in step q-1), when E is hydroxy, the reaction temperature is selected from -20 °C to 100 °C, preferably 0 °C to 30 °C.

In some embodiments, in step q-2), the compound of formula IV-2-2-3 or the salt thereof is subjected to a deprotection reaction to obtain the compound of formula IV-2-2-4 or the salt thereof.

In some embodiments, in step q-2), when PG₄ is Boc, the protecting group is removed from the compound of formula IV-2-2-3 or the salt thereof under an acidic condition; the acid is a protic acid selected from hydrochloric acid, trifluoroacetic acid, hydrobromic acid, sulfuric acid, hydrogen chloride/dioxane, and hydrogen chloride/ethyl acetate, preferably hydrochloric acid, hydrogen chloride/dioxane, and hydrogen chloride/ethyl acetate, further preferably hydrochloric acid.

In some embodiments, in step q-2), when PG₄ is Boc, a molar ratio of the compound of formula IV-2-2-3 or the salt thereof to the acid is selected from 1:1-1:50; preferably 1:1-1:30; for example, 1:1-1:20; further preferably 1:1-1:5.

In some embodiments, in step q-2), when PG₄ is Boc, the reaction solvent is one or any combination of ethers, esters, haloalkanes and water, and is preferably selected from one or any combination of tetrahydrofuran, 1,4-dioxane and water; for example 1,4-dioxane or tetrahydrofuran; preferably a mixed solvent of tetrahydrofuran and water.

In some embodiments, in step q-2), when PG₄ is Boc, the reaction temperature is 0 °C to 120 °C, preferably 25 °C to 80 °C.

In some embodiments, in step q-3), the compound of formula IV-2-2-4 or the salt thereof is subjected to a reductive amination reaction with an aldehyde under the action of a reducing agent and under an acidic or neutral condition to obtain the compound of formula IV-2-2 or the salt thereof.

In some embodiments, in step q-3), the reducing agent is selected from sodium borohydride, potassium borohydride, sodium cyanoborohydride, and sodium triacetoxyborohydride; preferably sodium cyanoborohydride and/or sodium triacetoxyborohydride.

In some embodiments, in step q-3), the acid is selected from acetic acid, formic acid, propionic acid, trifluoroacetic acid, and hydrochloric acid, preferably acetic acid or hydrochloric acid.

In some embodiments, in step q-3), a molar ratio of the compound of formula IV-2-2-4 or the salt thereof to the selected reducing agent is selected from 1:2-1:10, preferably 1:2-1:4.

In some embodiments, in step q-3), a molar ratio of the compound of formula IV-2-2-4 or the salt thereof to the aldehyde is selected from 1:1-1:10, preferably 1:3-1:5.

In some embodiments, in step q-3), a molar ratio of the compound of formula IV-2-2-4 or the salt thereof to the acid is selected from 1:1-1:10, preferably 1:1-1:5.

In some embodiments, in step q-3), the reaction solvent is selected from one or any combination of alcohols, ethers and water, preferably one or any combination of methanol, ethanol, tetrahydrofuran and water; more preferably a mixed solvent of tetrahydrofuran and water.

In some embodiments, in step q-3), the reaction temperature is -10 °C to 50 °C, preferably 0 °C to 30 °C.

### Intermediate

In one aspect, the present application provides a compound of formula IV-1 or a salt thereof, 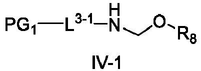 wherein L³⁻¹, PG₁, and R₈ are as defined in any of the embodiments of the present application. In another aspect, the present application provides a compound of IV-2-1 or a salt thereof, 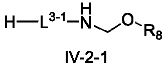 wherein L³⁻¹ and R₈ are as defined in any of the embodiments of the present application.

In another aspect, the present application provides a compound of formula IV-2 or a salt thereof, 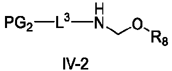 wherein L³, PG₂, and R₈ are as defined in any of the embodiments of the present application.

In another aspect, the present application provides a compound of formula X-3-1 or a salt thereof, 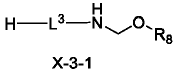 wherein L³ and R₈ are as defined in any of the embodiments of the present application.

In another aspect, the present application provides a compound of formula X-3 or a salt thereof, 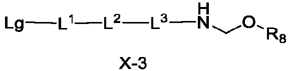 wherein Lg, L¹, L², L³, and R₈ are as defined in any of the embodiments of the present application.

In another aspect, the present application provides use of the compound of formula IV-1 or the salt thereof in the preparation of a compound of formula IV-2 or a salt thereof, a compound of formula IV-2-1 or a salt thereof, a compound of formula X-3-1 or a salt thereof, a compound of formula X-3 or a salt thereof, a compound of formula II or a salt thereof, or a compound of formula I or a salt thereof, 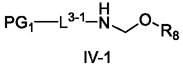 wherein L³⁻¹, PG₁, and R₈ are as defined in any of the embodiments of the present application.

In another aspect, the present application provides use of the compound of formula IV-2-1 or the salt thereof in the preparation of a compound of formula IV-2 or a salt thereof, a compound of formula X-3-1 or a salt thereof, a compound of formula X-3 or a salt thereof, a compound of formula II or a salt thereof, or a compound of formula I or a salt thereof, 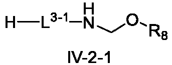 wherein L³⁻¹ and R₈ are as defined in any of the embodiments of the present application.

In another aspect, the present application provides use of the compound of formula IV-2 or the salt thereof in the preparation of a compound of formula X-3 or a salt thereof, a compound of formula X-3-1 or a salt thereof, a compound of formula II or a salt thereof, or a compound of formula I or a salt thereof, 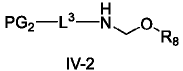 wherein L³, PG₂, and R₈ are as defined in any of the embodiments of the present application.

In another aspect, the present application provides use of the compound of formula X-3-1 or the salt thereof in the preparation of a compound of formula X-3 or a salt thereof, a compound of formula II or a salt thereof, or a compound of formula I or a salt thereof, 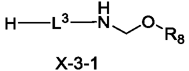 wherein L³ and R₈ are as defined in any of the embodiments of the present application.

In another aspect, the present application provides use of the compound of X-3 or the salt thereof in the preparation of a compound of formula II or a salt thereof, or a compound of formula I or a salt thereof, 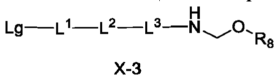 wherein Lg, L¹, L², L³, and R₈ are as defined in any of the embodiments of the present application.

In another aspect, the present application provides use of the compound of formula I or the salt thereof in the preparation of an ADC drug, 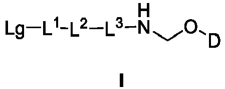 wherein Lg, L¹, L², L³, and D are as defined in any of the embodiments of the present application.

In another aspect, the present application provides use of the compound of formula II or the salt thereof in the preparation of an ADC drug, 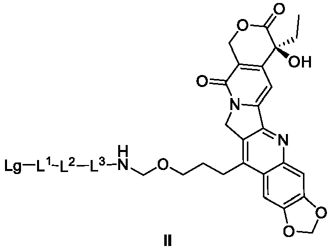 wherein Lg, L¹, L², and L³ are as defined in any of the embodiments of the present application.

In some embodiments of the present application, the compound of formula IV-1 or the salt thereof is selected from the following structures:

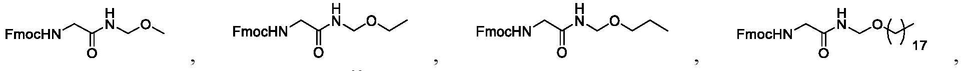

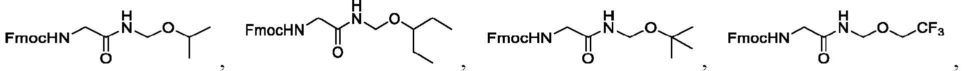

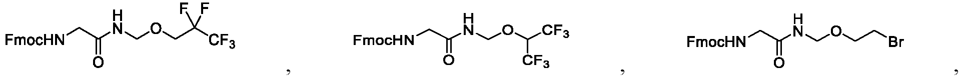

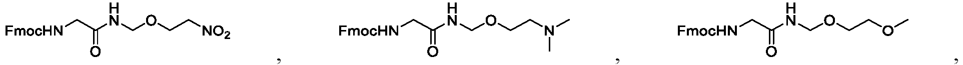

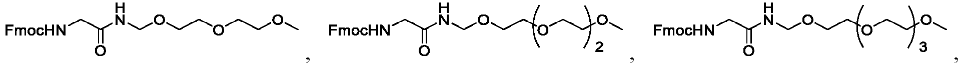

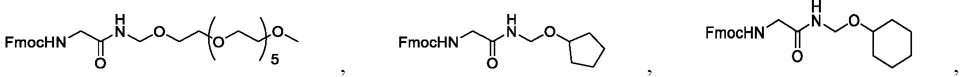

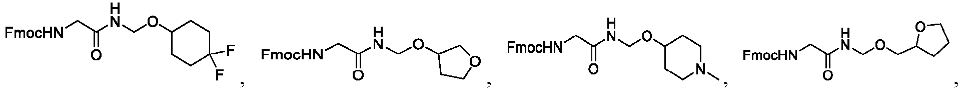

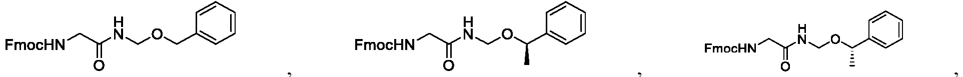

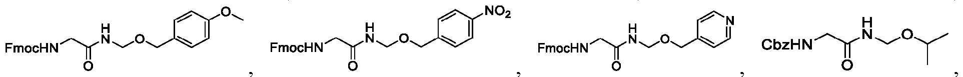

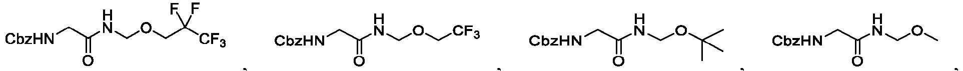

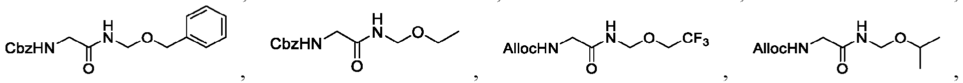

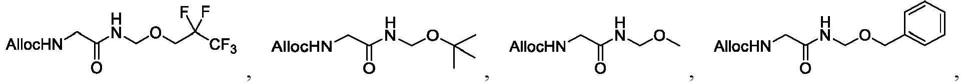

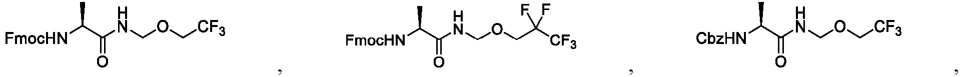

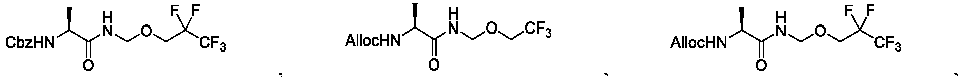

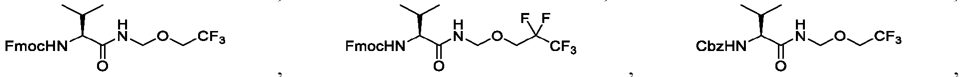

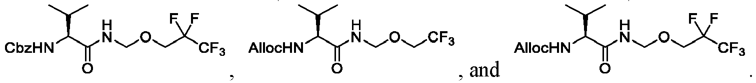

In some embodiments of the present application, the compound of formula IV-2-1 or the salt thereof is selected from the following structures:

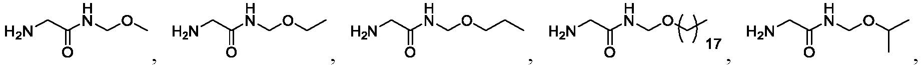

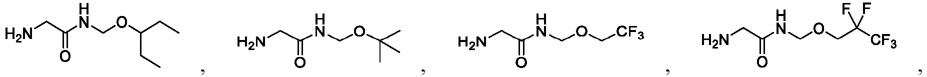

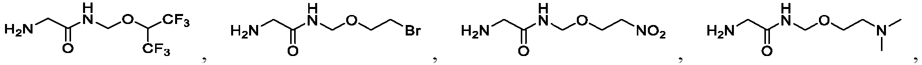

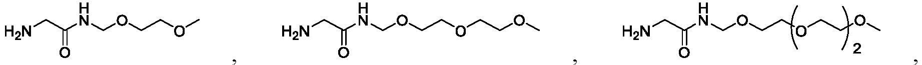

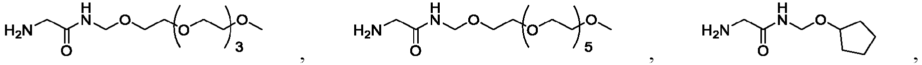

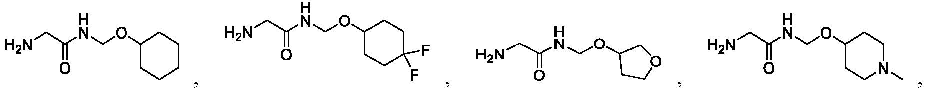

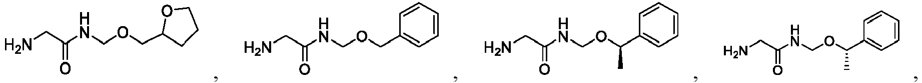

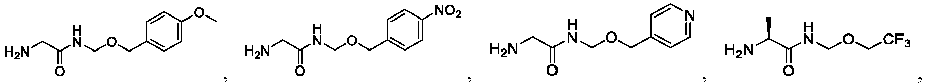

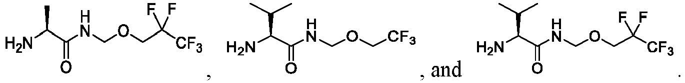

In some embodiments of the present application, the compound of formula IV-2 or the salt thereof is selected from the following structures:

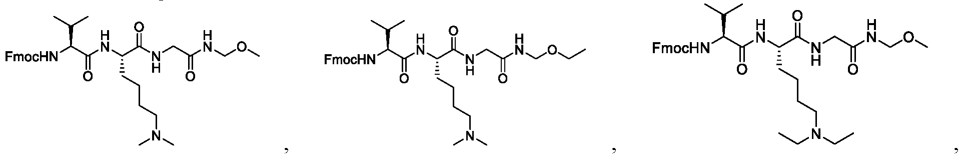

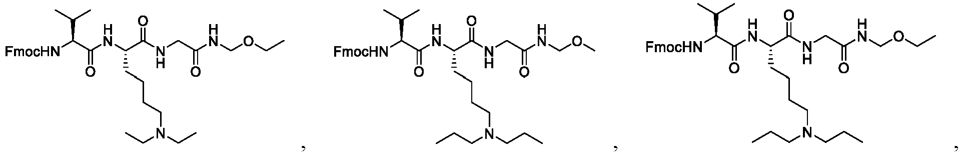

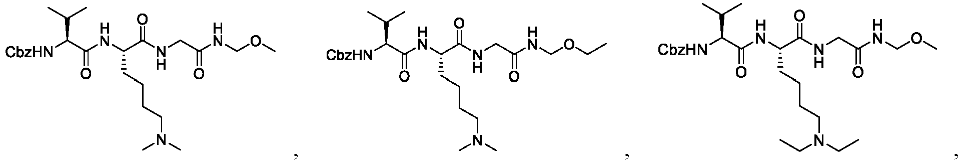

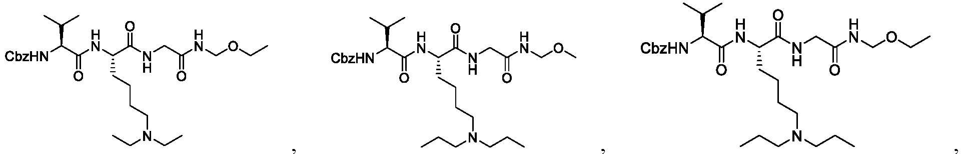

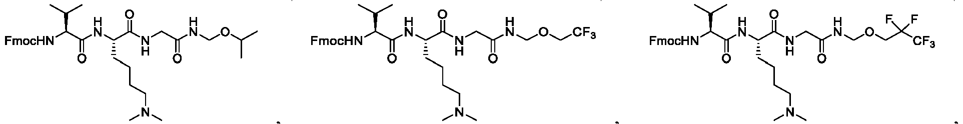

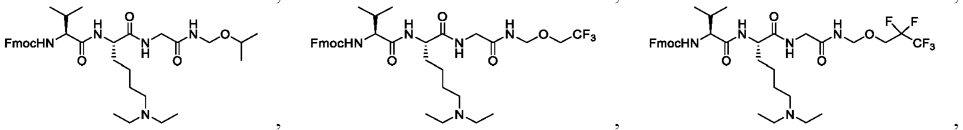

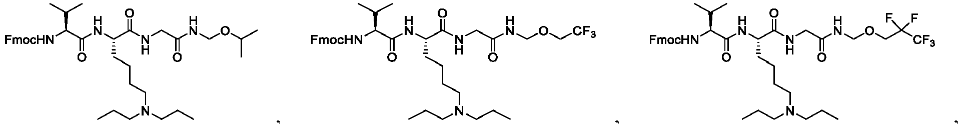

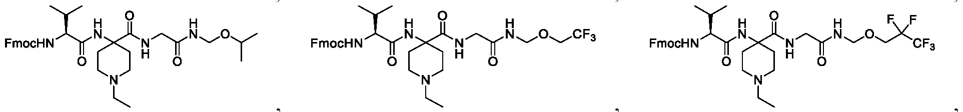

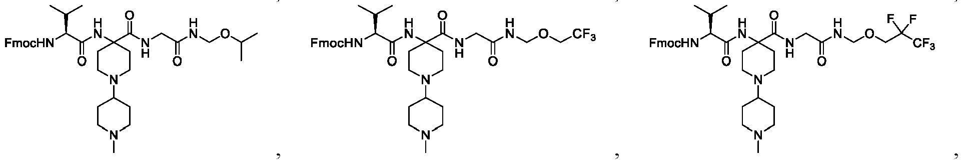

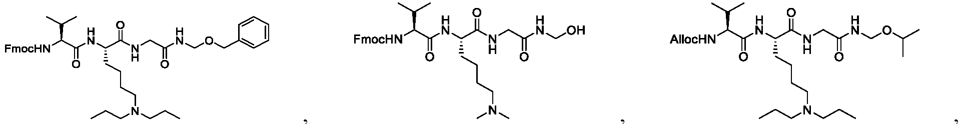

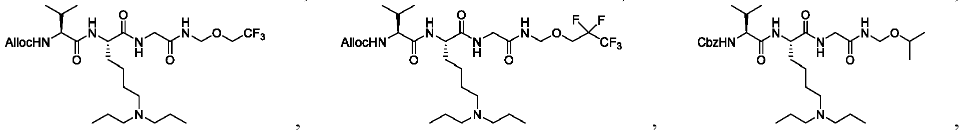

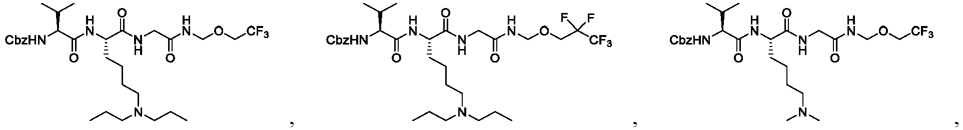

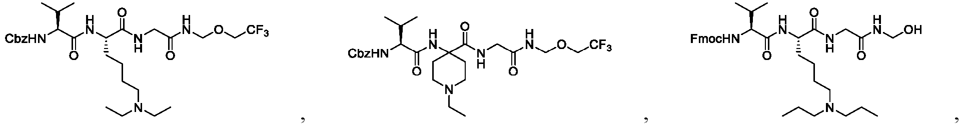

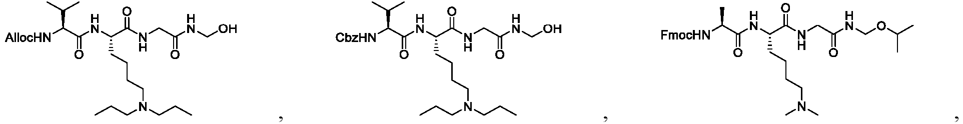

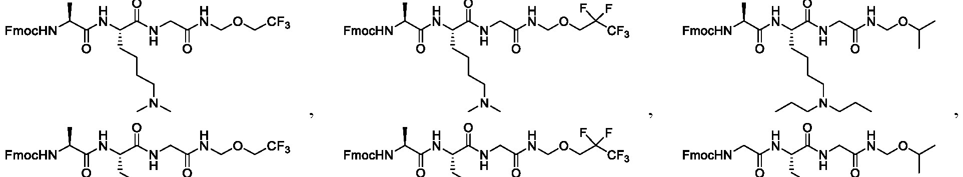

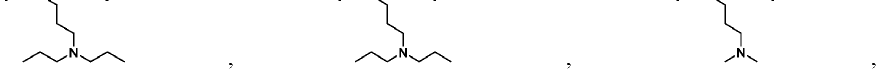

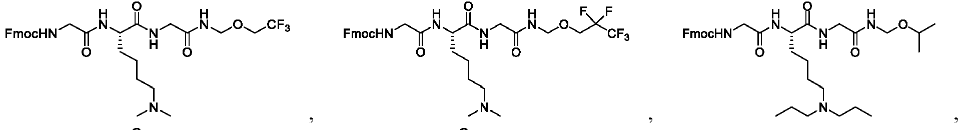

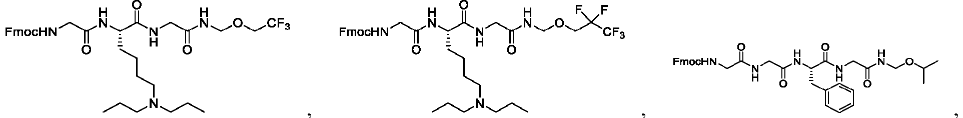

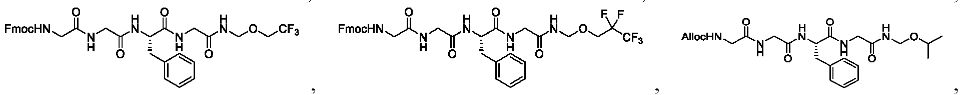

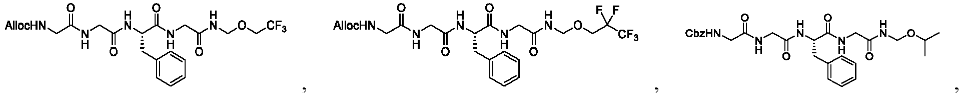

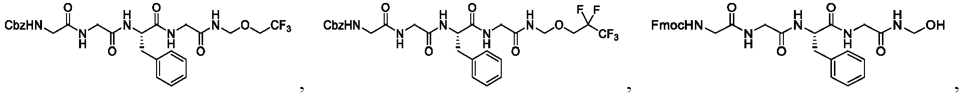

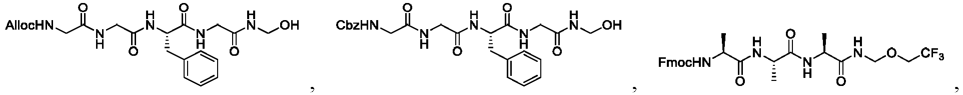

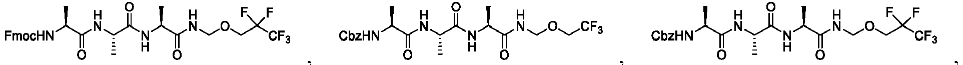

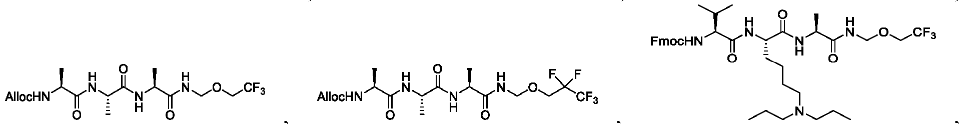

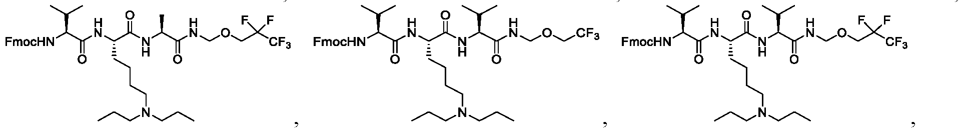

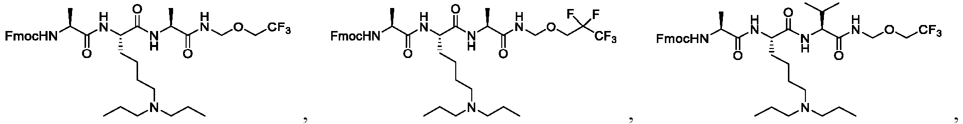

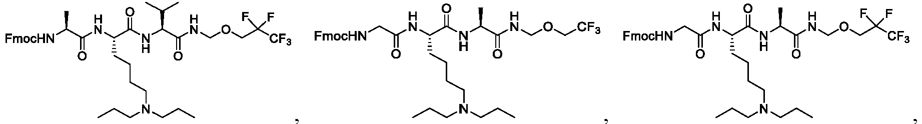

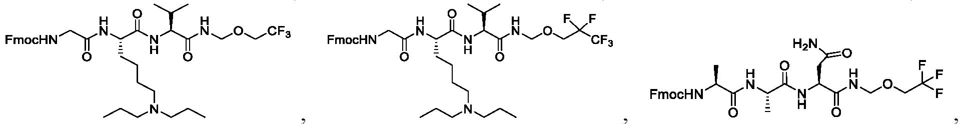

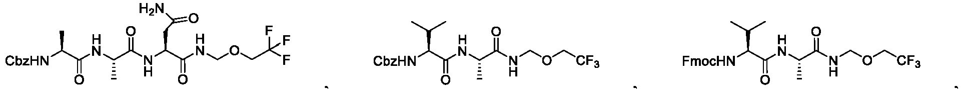

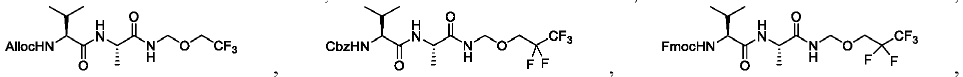

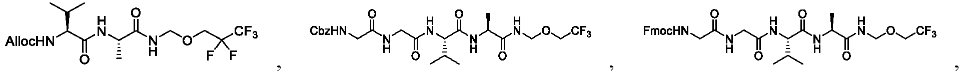

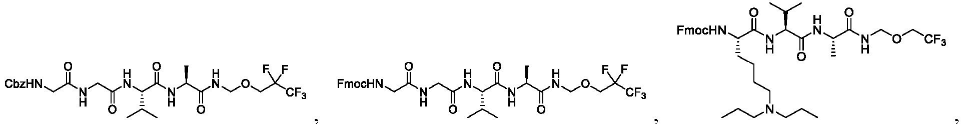

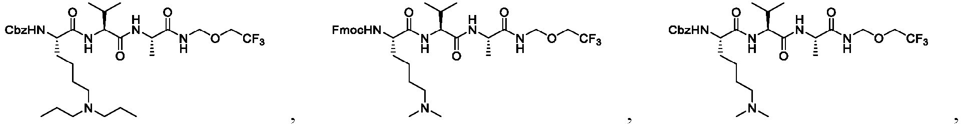

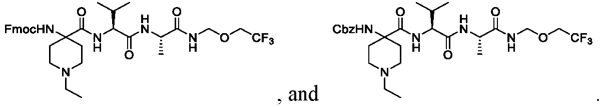

In some embodiments of the present application, the compound of formula X-3-1 or the salt thereof is selected from the following structures:

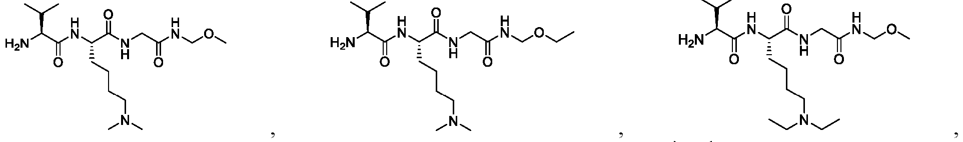

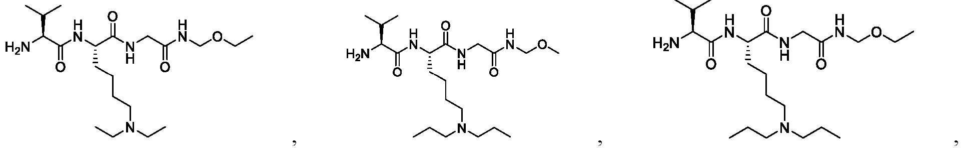

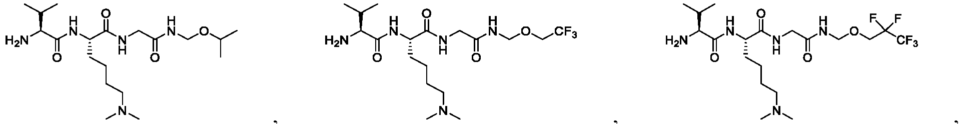

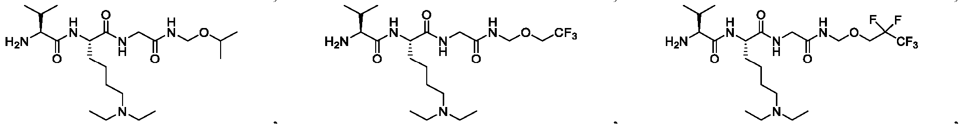

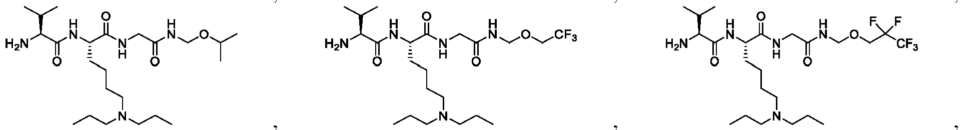

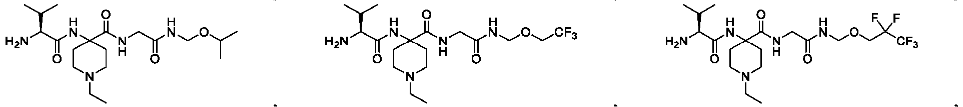

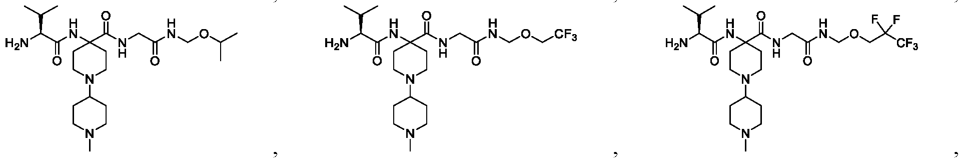

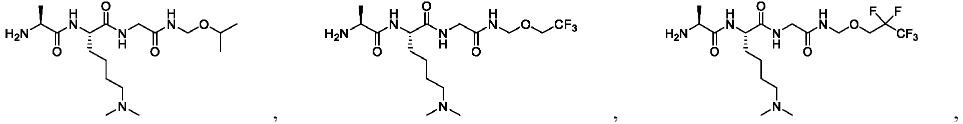

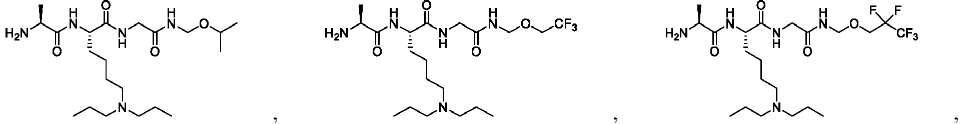

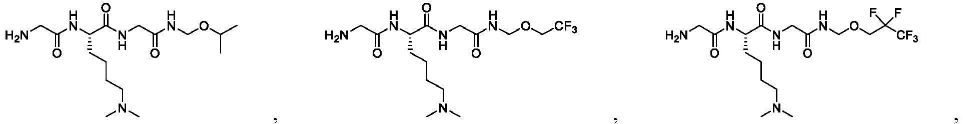

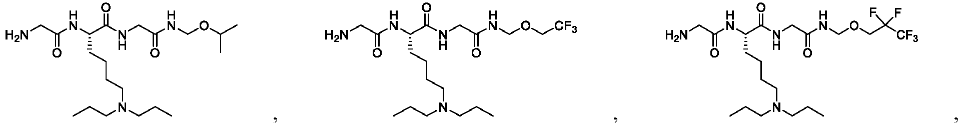

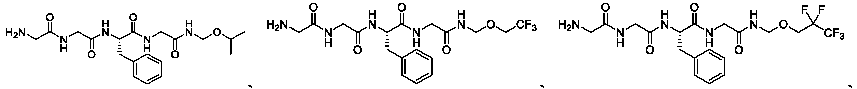

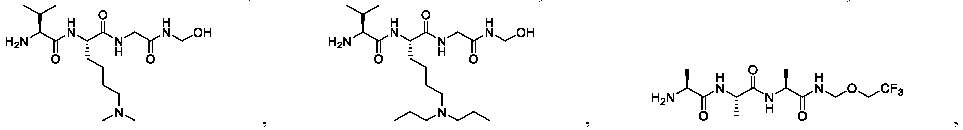

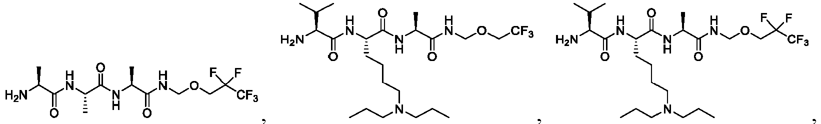

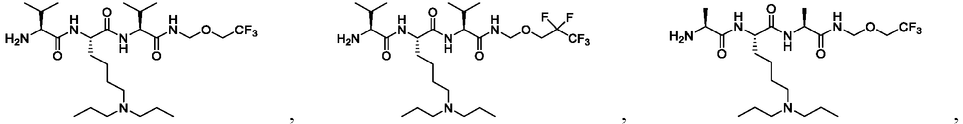

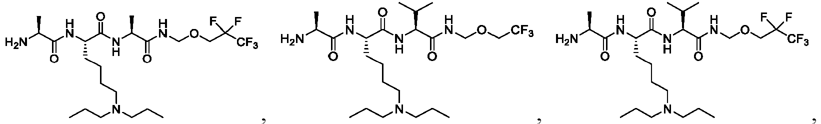

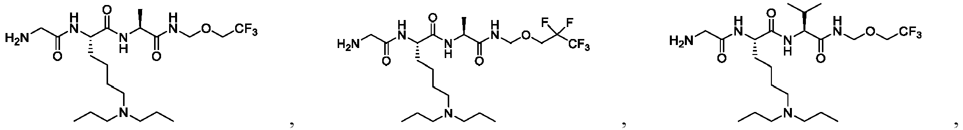

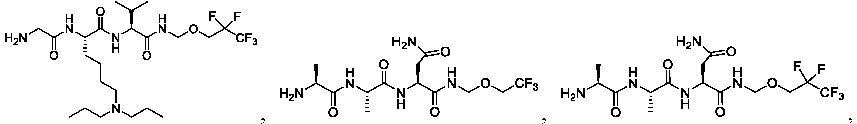

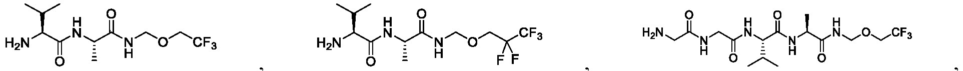

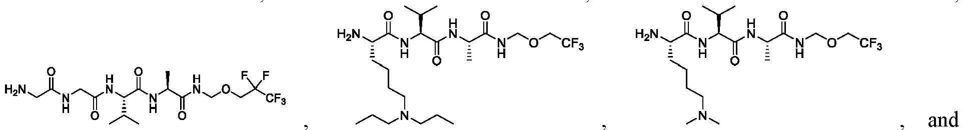

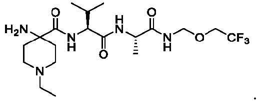

In some embodiments of the present application, the compound of formula X-3 or the salt thereof is selected from the following structures: 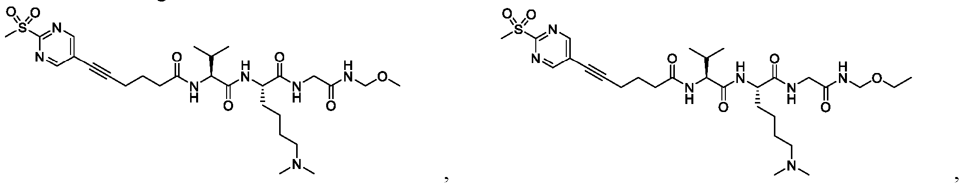 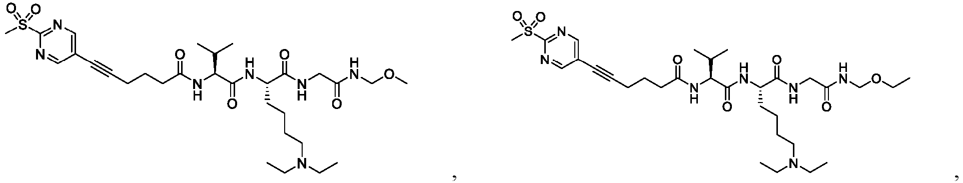 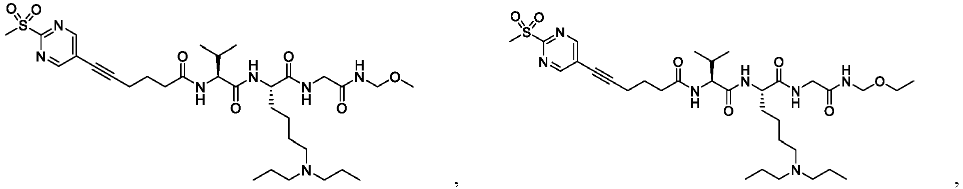 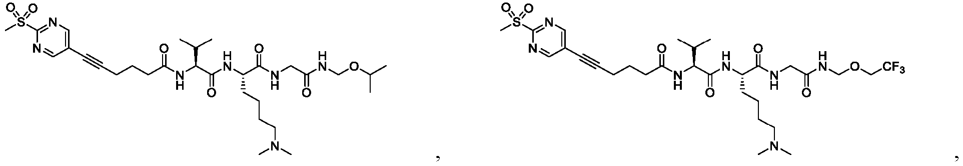 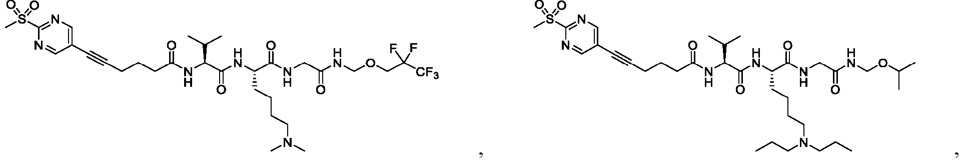 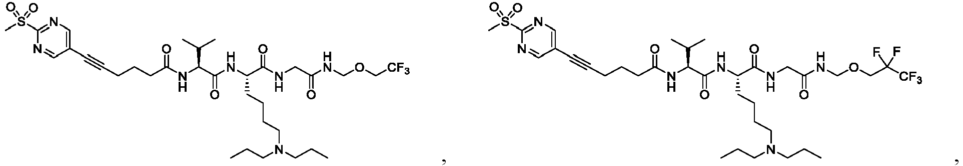 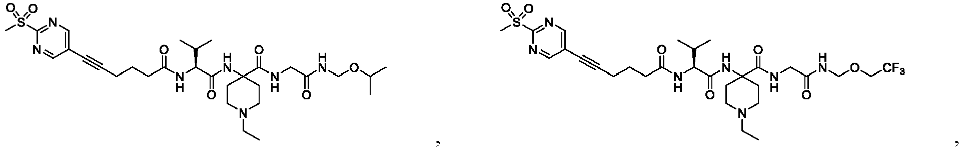 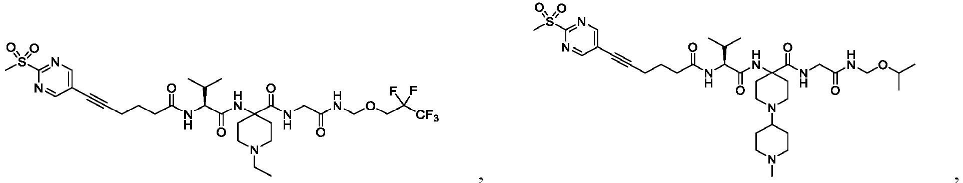 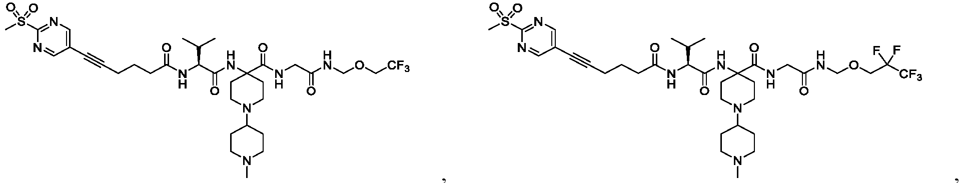 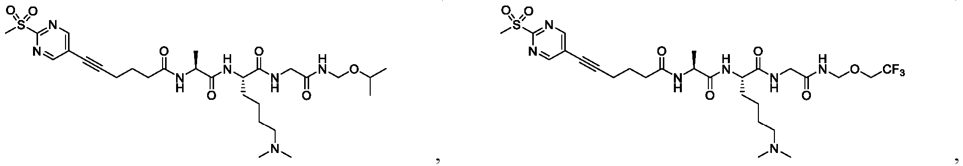 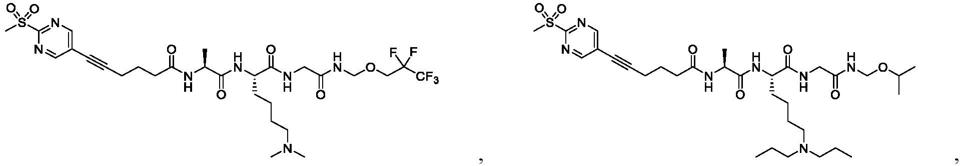 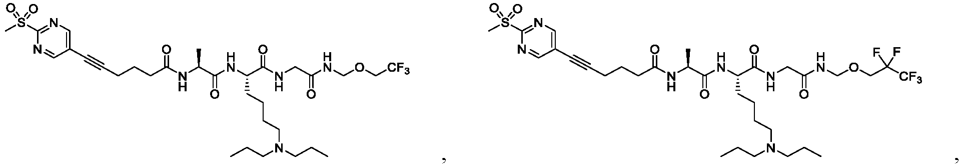 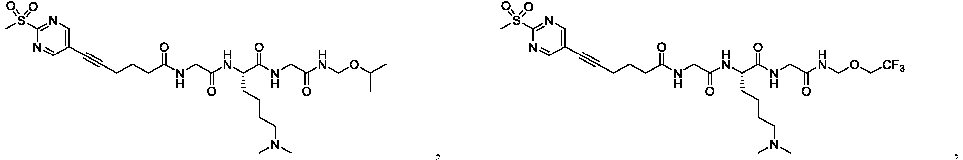 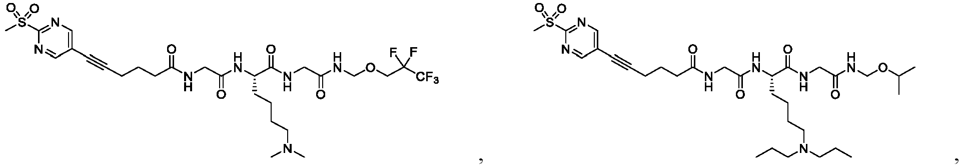 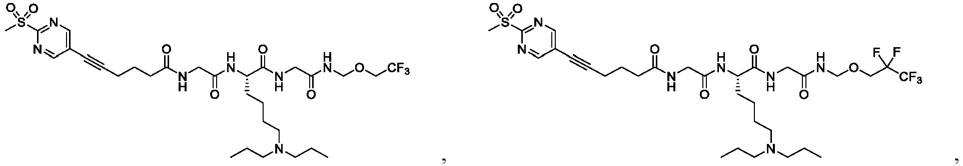 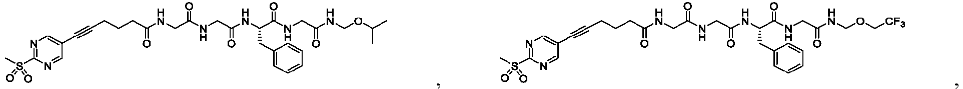 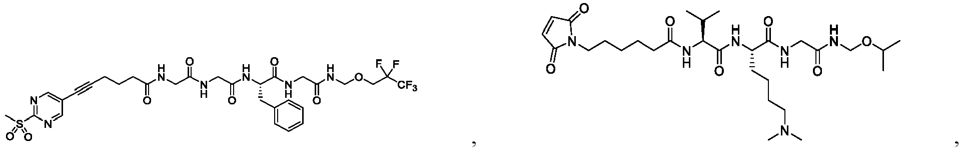 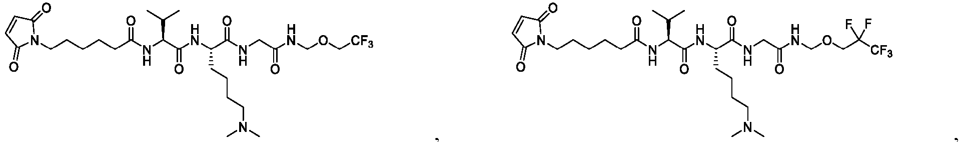 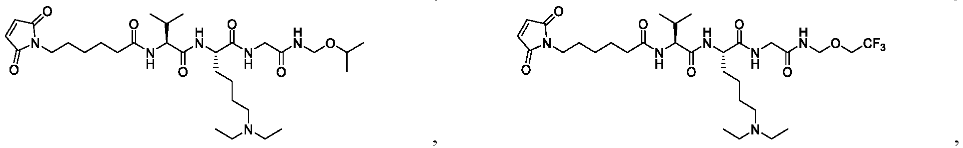 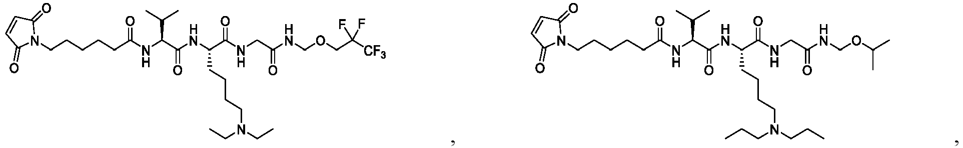 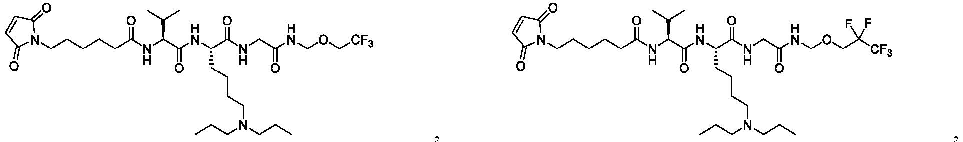 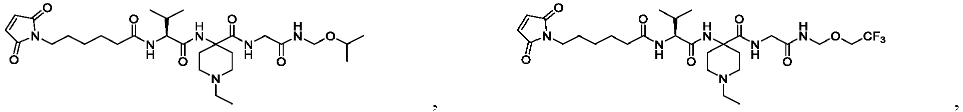 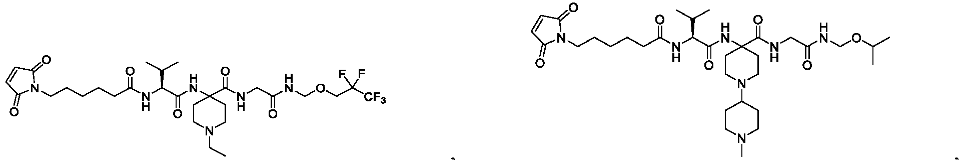 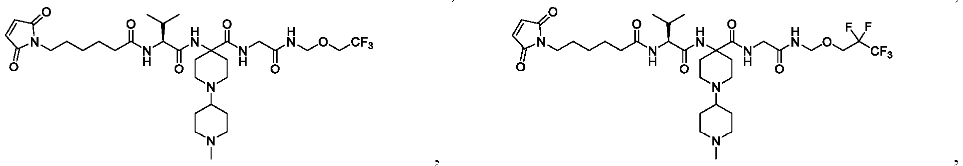 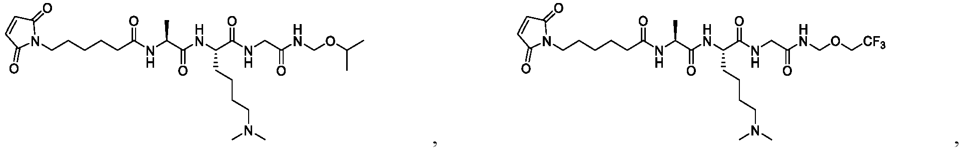 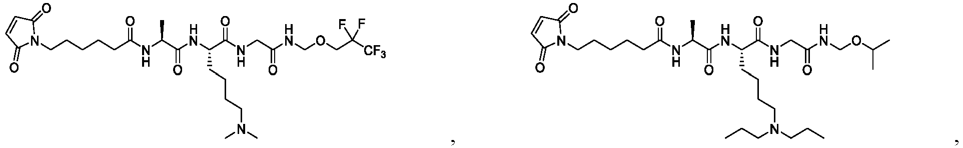 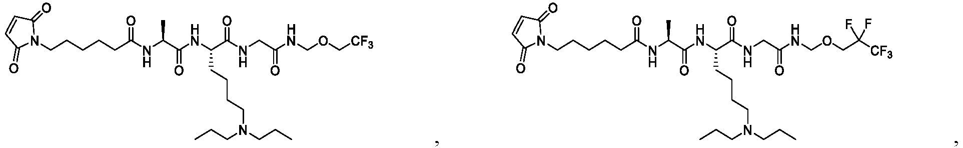 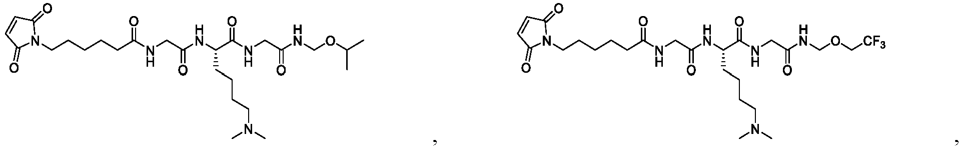 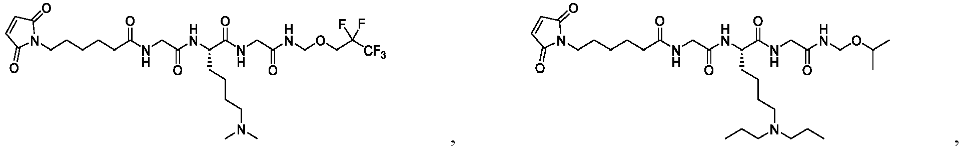 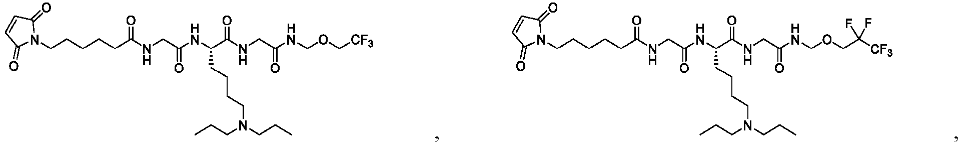 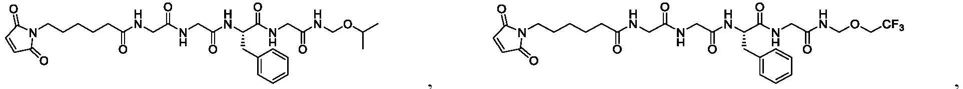 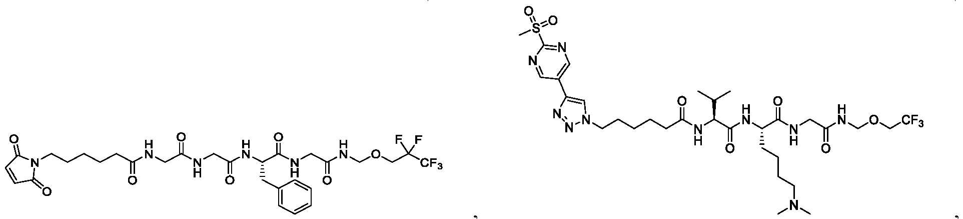 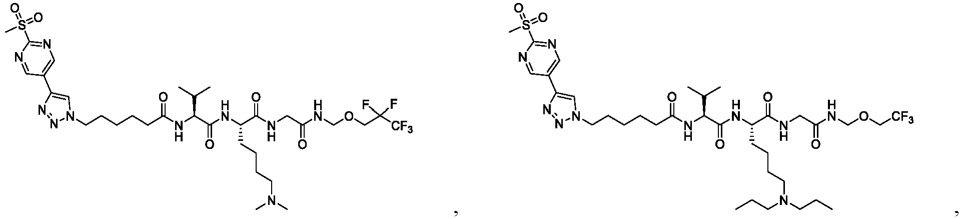 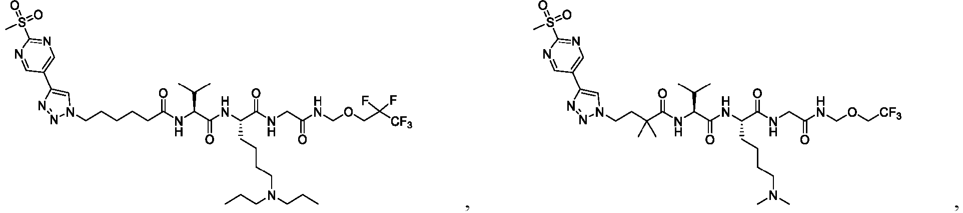 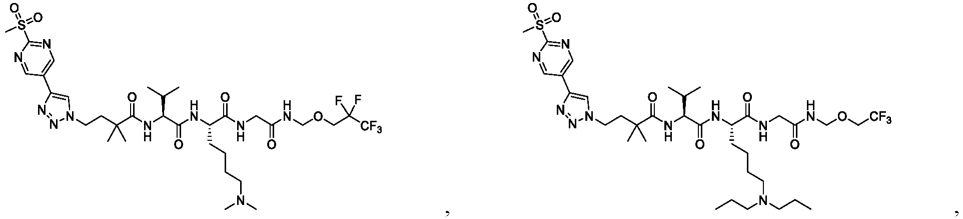 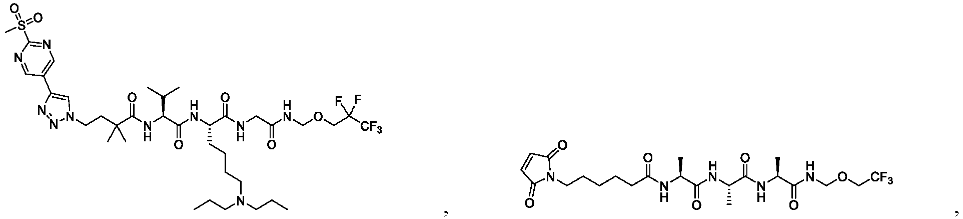 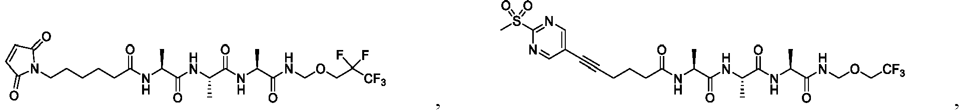 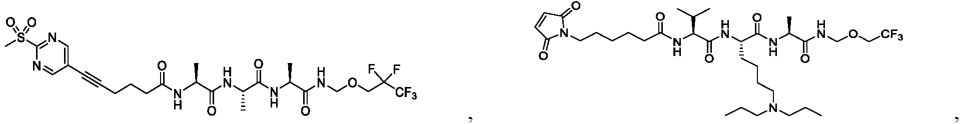 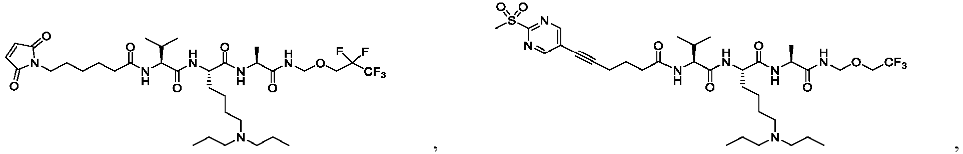 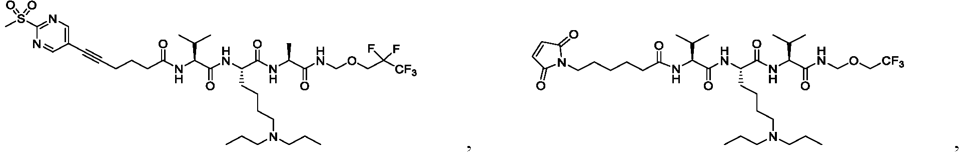 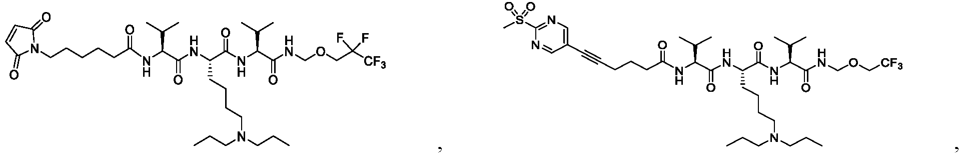 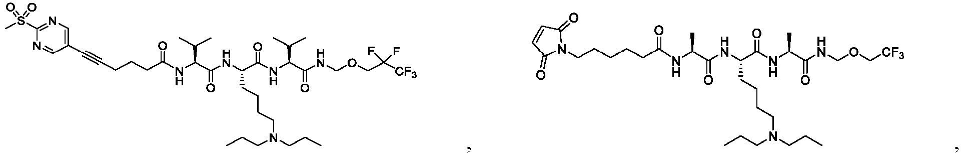 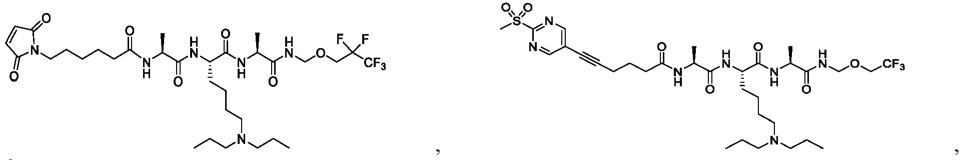 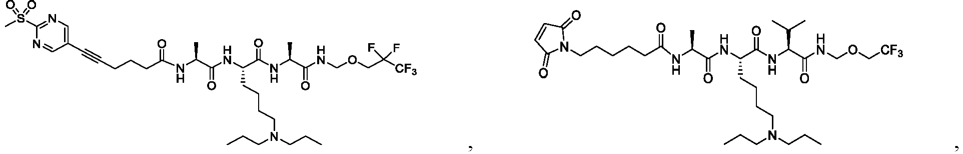 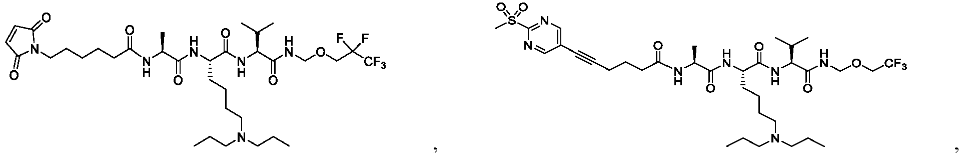 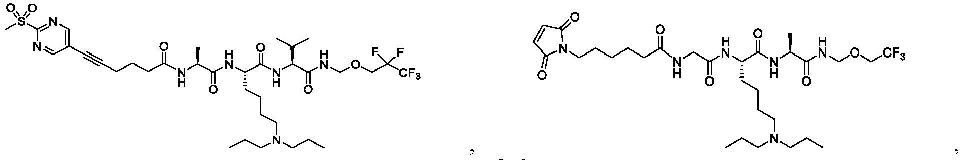 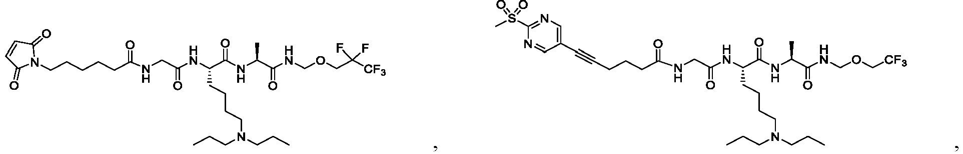 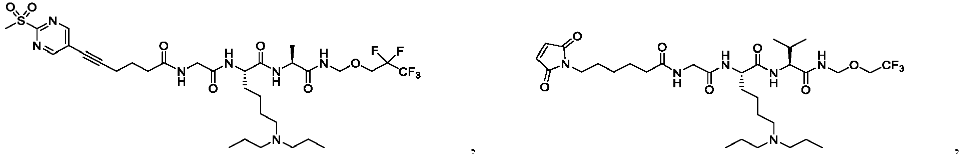 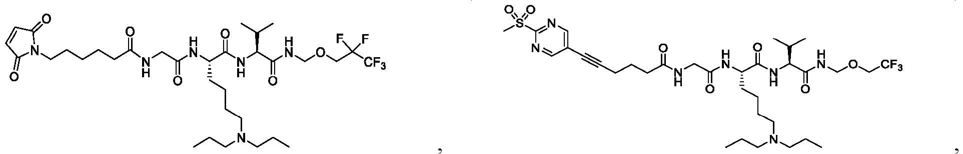 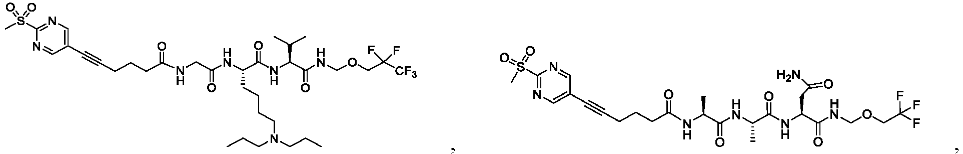 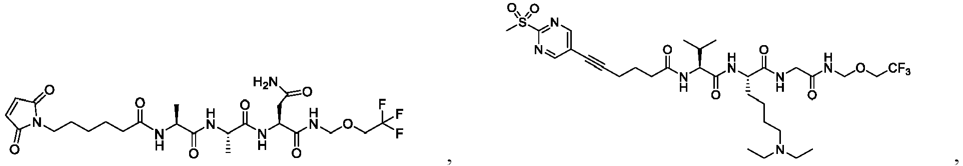 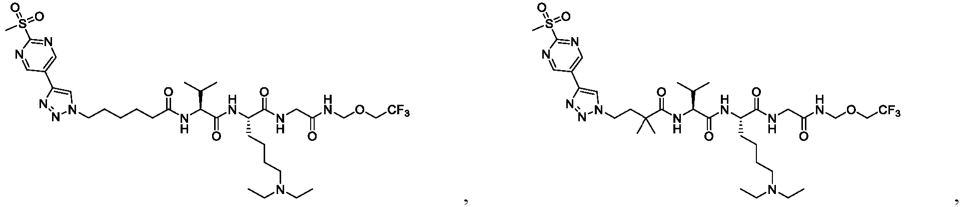 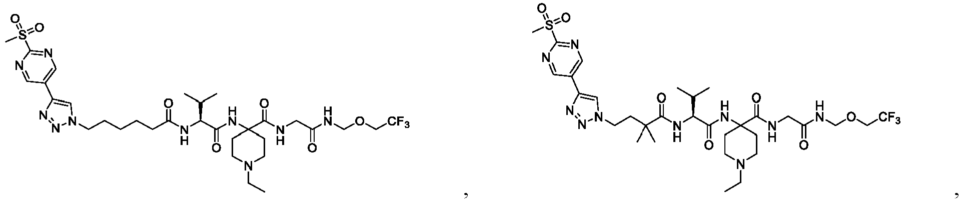 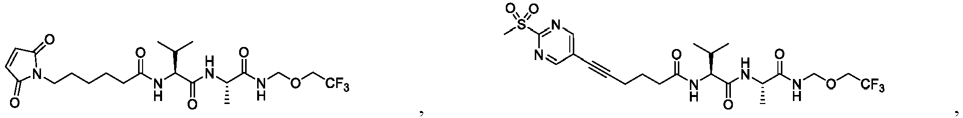 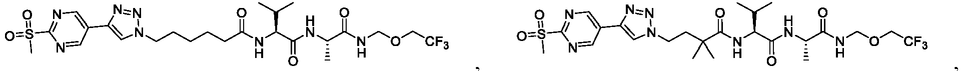 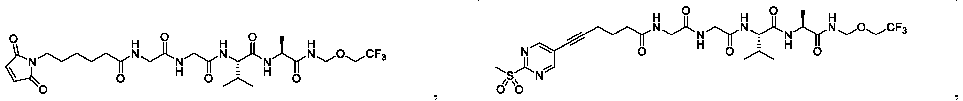 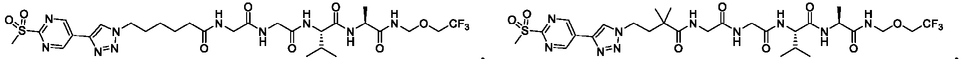 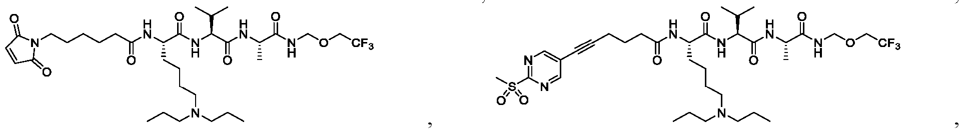 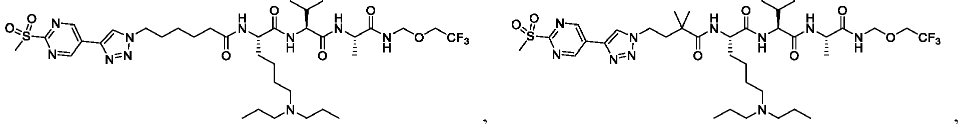 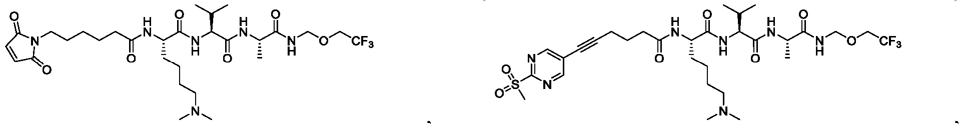 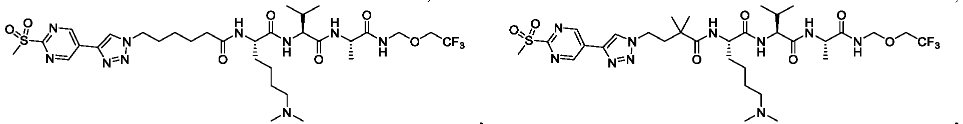 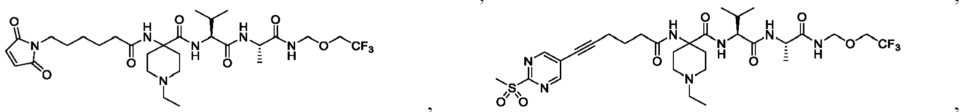 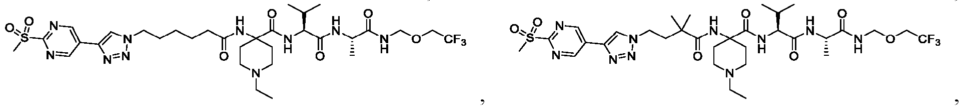 and

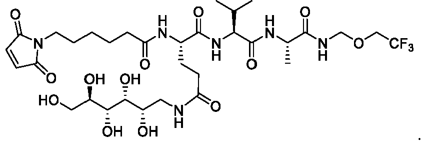

In some embodiments of the present application, the compound of formula II or the salt thereof is selected from the following structures:

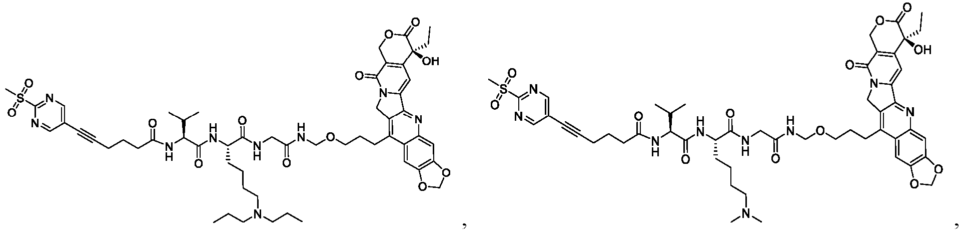

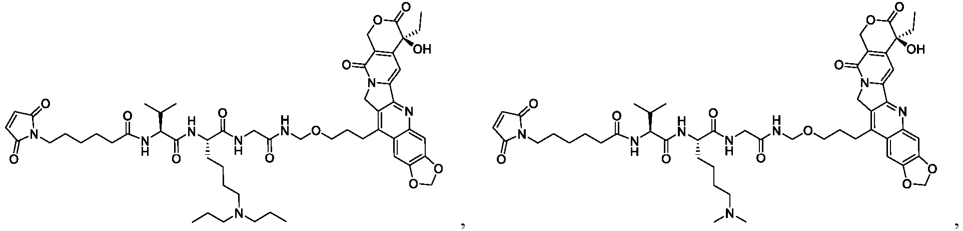

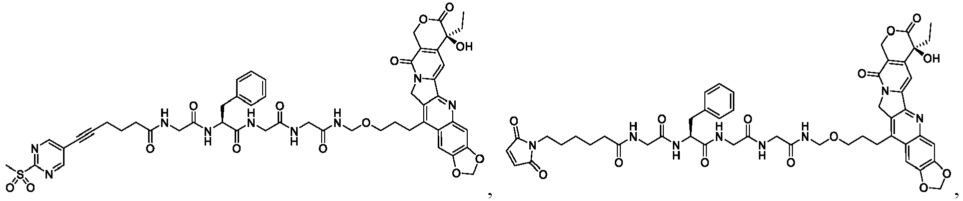

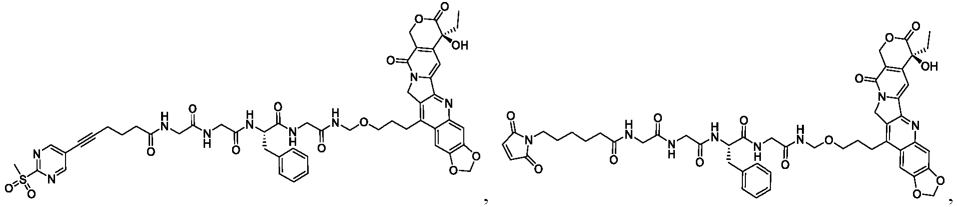

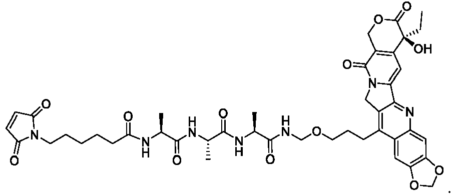

### Definitions and Terms

Unless otherwise stated, the terms and phrases used herein have the meanings set forth as follows. A specific term or phrase should not be considered uncertain or unclear in the absence of a particular definition, but construed according to its common meaning understood by those skilled in the art. When referring to a trade name, it is intended to refer to its corresponding commercial product or its active ingredient.

In the present application, a word in the singular form includes the plural form thereof, unless otherwise stated or implied in the context. Thus, the singular terms and "the" and "said" as mentioned herein generally include the plural of each term. The terms "comprise", "include" and "contain" should be construed as inclusive and not exclusive.

In the present application, the term "salt thereof" as used in the present application refers to a salt form of a compound (e.g., a compound of formula 1), unless otherwise stated or implied in the context. The compound in a salt form has one or more inner salt forms and/or comprises another molecule. The counterion of the compound in the salt form is typically an organic or inorganic moiety that stabilizes the charge of the parent compound. A salt form of a compound has one or more charged atoms in the structure thereof. Where a plurality of charged atoms are part of a salt form, there are a plurality of counterions and/or a plurality of charged counterions. Thus, a salt form of a compound typically has one or more charged atoms, which correspond to a non-salt form of the compound and one or more counterions. In some aspects, a non-salt form of a compound contains at least one amino group or other basic moieties, thus in the presence of an acid, an acid addition salt having a basic moiety is obtained. In other aspects, a non-salt form of a compound contains at least one carboxylic acid group or other acidic moieties, thus in the presence of a base, a carboxylate or other anionic moieties are obtained.

In the present application, unless otherwise stated, the scientific and technical terms used herein have the meanings generally understood by those skilled in the art. In addition, the laboratory operations of cell culture, molecular genetics, nucleic acid chemistry, and immunology used herein are the conventional procedures widely used in the corresponding fields. Meanwhile, in order to better understand the present application, the definitions and explanations of the relevant terms are provided as follows.

As used herein, the term "stereoisomer" refers to an isomer formed due to at least one asymmetric center. In compounds having one or more (e.g., one, two, three or four) asymmetric centers, racemic mixtures, single enantiomers, mixtures of diastereoisomers and individual diastereomers may be produced. Certain individual molecules may also exist as geometric isomers (cis/trans). Similarly, the compound of the present invention may exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present application encompasses all such isomers or mixtures thereof in any proportion (e.g., 60%, 65%, 70%, 75%, 80%, 85%, 90%, 95%, 96%, 97%, 98% or 99%).

A solid line (-), a solid wedge or a dashed wedge may be used in the present application to depict carbon-carbon bonds of the compound of the present invention. Using the solid line to depict a bond that is bonded to an asymmetric carbon atom is intended to indicate that all possible stereoisomers (e.g., particular enantiomers, and racemic mixtures) at the carbon atom are included. Using the solid or dashed wedge to depict a bond that is bonded to an asymmetric carbon atom is intended to indicate that the stereoisomers shown are present. When present in a racemic mixture, the solid and dashed wedges are used to define the relative stereochemistry rather than the absolute stereochemistry. Unless otherwise indicated, the compound of the present invention is intended to be present in the form of stereoisomers (including cis and trans isomers, optical isomers (e.g., *R* and S enantiomers), diastereomers, geometric isomers, rotamers, conformers, atropisomers, and mixtures thereof). The compound of the present invention may exhibit more than one type of isomerism and consist of mixtures thereof (e.g., racemic mixtures and diastereomer pairs).

In the present application, the term "bioactive molecule" refers to a substance that inhibits or prevents cell functions and/or causes cell death or cell destruction, and in some embodiments of the present application, the bioactive molecule in the conjugate is a molecule having antitumor bioactivity; for example: radioisotopes, such as At211 and radioisotopes of Lu; metal complexes, such as metal platinum complexes, metal gold complexes, and oxaliplatin; glycopeptide antibiotics, such as bleomycin and pingyangmycin; DNA topoisomerase inhibitors such as topoisomerase 1 inhibitor, camptothecin, hydroxycamptothecin, 9-aminocamptothecin, SN-38, irinotecan, topotecan, belotecan, rubitecan, topoisomerase II inhibitor, actinomycin D, adriamycin, doxorubicin, duocarmycin, daunorubicin, mitoxantrone, podophyllotoxin, and etoposide; drugs that interfere with DNA synthesis, such as methotrexate, 5-fluorouracil, cytarabine, gemcitabine, mercaptopurine, pentostatin, fludarabine, cladribine, and nelarabine; drugs that act on structural proteins, such as tubulin inhibitors, vinca alkaloid, vincristine, vinblastine, paclitaxel, docetaxel, and cabazitaxel; tumor signaling pathway inhibitors, such as serine/threonine kinase inhibitors, tyrosine kinase inhibitors, aspartate kinase inhibitors, or histidine kinase inhibitors; also comprises proteasome inhibitors, histone deacetylase inhibitors, tumor angiogenesis inhibitors, cyclin inhibitors, maytansine derivatives, calicheamicin derivatives, auristatin derivatives, PBD derivatives, melphalan, mitomycin C or other active substances that inhibit tumor cell growth and promote tumor cell apoptosis and necrosis; enzymes and fragments thereof, etc.

In the present application, the term "drug" refers to a substance that inhibits or prevents cell functions and/or causes cell death or cell destruction.

In the present application, the term "linker" refers to a fragment that attaches a bioactive compound fragment (drug molecule) to an antibody moiety.

In the present application, the term "antibody" is interpreted in its broadest sense and includes intact monoclonal antibodies, polyclonal antibodies, and multispecific antibodies (e.g., bispecific antibodies) formed from at least two intact antibodies, so long as they possess the desired bioactivity. In the present application, "antibody" and "immunoglobulin" may be used interchangeably.

In the present application, the term "monoclonal antibody" refers to an antibody from a population of substantially homogeneous antibodies, i.e., the individual antibodies constituting the population are identical except for a small number of natural mutations that may be present. Monoclonal antibodies have high specificity for one determinant (epitope) of an antigen, while polyclonal antibodies in contrast thereto comprise different antibodies against different determinants (epitopes). In addition to specificity, monoclonal antibodies have the advantage that they can be synthesized without contamination by other antibodies. The modifier "monoclonal" used herein indicates that the antibody is characterized as being from a substantially homogeneous population of antibodies, and should not be construed as requiring a particular preparation method.

In the present application, unless otherwise expressly indicated, the description "each ... be independently selected from" and "... be each independently selected from" are used interchangeably throughout the present application and should be understood in a broad sense, and it may mean that specific items expressed by the same or different symbols in different groups do not affect each other, or that specific items expressed by the same or different symbols in the same group do not affect each other.

In the present application, in the structure 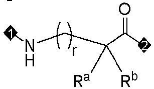 of the amino acid residue represented by AA¹, if r is 0, it will be understood by those skilled in the art that the structure of the amino acid residue represented by AA¹ will become 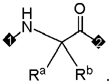 In the structure 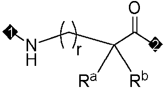 of the amino acid residue represented by AA¹, if R^{a} and R^{b}, together with the carbon atom to which they are both attached, form a 4-10 membered heterocyclic ring, the 4-10 membered heterocyclic ring is optionally substituted with one or more R⁰, wherein the term "the 4-10 membered heterocyclic ring is optionally substituted with one or more R⁰" means that the 4-10 membered heterocyclic ring may be unsubstituted or substituted with one or more R⁰, and in the more R⁰, the definition of each R⁰ may be identical or different. Other similar definitions may be understood with reference to the aforementioned content.

In each part of this specification, substituents for the compounds of the present application are disclosed according to group types or ranges. It is specifically noted that each independent sub-combination of the various members of these group types and ranges is included in the present application. For example, the term "C1-6 alkyl" refers specifically to independently disclosed methyl, ethyl, C3 alkyl, C4 alkyl, C5 alkyl, and C6 alkyl.

In the present application, the terms "include", "comprise", "have", "contain" or "involve", and other variant forms thereof herein, are inclusive or open-ended and do not exclude other unrecited elements or method steps.

In the present application, the "parent ring" is

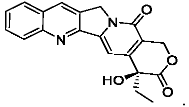

In the present application, the term "C1-30 alkyl" refers to linear or branched alkyl containing 1-30 carbon atoms, including, for example, "C1-6 alkyl"; the term "C1-6 alkyl" refers to linear or branched alkyl containing 1-6 carbon atoms, including, for example, "C1-3 alkyl" or "C1-4 alkyl", methyl, and ethyl, and specific examples include, but are not limited to: methyl, ethyl, n-propyl, isopropyl, n-butyl, isobutyl, sec-butyl, *tert-*butyl, pentyl, and hexyl.

In the present application, the term "C2-6 alkenyl" refers to linear, branched or cyclic alkenyl containing at least one double bond and 2-6 carbon atoms, including, for example, "C2-4 alkenyl". Examples of alkenyl include, but are not limited to: ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, 1,4-cyclohexadienyl, etc.

In the present application, the term "C2-6 alkynyl" refers to linear or branched alkynyl containing at least one triple bond and 2-6 carbon atoms, including, for example, "C2-4 alkynyl". Examples of alkynyl include, but are not limited to: ethynyl, propynyl, 2-butynyl, 2-pentynyl, 3-pentynyl, 4-methyl-2-pentynyl, 2-hexynyl, 3-hexynyl, 5-methyl-2-hexynyl, etc.

In the present application, the term "halogen" includes fluorine, chlorine, bromine and iodine.

In the present application, the term "C3-7 cycloalkyl" refers to saturated cyclic alkyl containing 3-7 carbon atoms, and the term "C3-6 cycloalkyl" refers to saturated cyclic alkyl containing 3-6 carbon atoms. Optionally, the carbon atom in the cyclic structure may be substituted with oxo. Examples include cyclopropylalkyl (i.e., cyclopropyl), cyclobutylalkyl (i.e., cyclobutyl), cyclopentylalkyl (i.e., cyclopentyl), and cyclohexyl.

In the present application, the term "C1-6 alkoxy" refers to alkyl as defined above which is attached to the parent molecular moiety via an oxygen atom, including, for example, "C1-3 alkoxy" or "C1-4 alkoxy". Specific examples include, but are not limited to, methoxy, ethoxy, propoxy, isopropoxy, n-propoxy, isopropoxy, n-butoxy, isobutoxy, tert-butoxy, pentoxy, hexoxy, etc.

In the present application, the term "C1-6 haloalkyl" refers to alkyl as defined above which is attached to the parent molecular moiety via halogen, including, for example, "C1-3 haloalkyl" or "C1-4 haloalkyl". Specific examples include, but are not limited to, chloromethyl, fluoroethyl, bromopropyl, etc.

In the present application, the term "3-20 membered heterocyclyl" refers to a cyclic group containing 3-20 ring atoms (at least one of which is a heteroatom, such as a nitrogen atom, an oxygen atom or a sulfur atom). The term "4-10 membered heterocyclyl" refers to a cyclic group containing 4-10 ring atoms (at least one of which is a heteroatom, such as a nitrogen atom, an oxygen atom or a sulfur atom). The term "3-6 membered heterocyclyl" refers to a cyclic group containing 3-6 ring atoms (at least one of which is a heteroatom, such as a nitrogen atom, an oxygen atom or a sulfur atom). The term "5-6 membered heterocyclyl" refers to a cyclic group containing 5-6 ring atoms (at least one of which is a heteroatom, such as a nitrogen atom, an oxygen atom or a sulfur atom). Optionally, the ring atom (e.g., the carbon atom, the nitrogen atom or the sulfur atom) in the cyclic structure may be substituted with oxo. "4-8 membered heterocyclyl" includes, for example, "4-8 membered nitrogen-containing heterocyclyl", "4-8 membered oxygen-containing heterocyclyl", "4-7 membered heterocyclyl", "4-7 membered oxygen-containing heterocyclyl", "4-7 membered heterocyclyl", "4-6 membered heterocyclyl", "5-7 membered heterocyclyl", "5-6 membered heterocyclyl", and "5-6 membered nitrogen-containing heterocyclyl". Specific examples include, but are not limited to, oxocyclobutanyl, pyrrolidinyl, tetrahydrofuranyl, piperidinyl, piperazinyl, tetrahydropyranyl, homopiperazinyl, etc.

In the present application, the term "4-10 membered heterocyclic ring" refers to a ring containing 4-10 ring atoms (at least one of which is a heteroatom, such as a nitrogen atom, an oxygen atom or a sulfur atom). The term "3-6 membered heterocyclic ring" refers to a ring containing 3-6 ring atoms (at least one of which is a heteroatom, such as a nitrogen atom, an oxygen atom or a sulfur atom). The term "5-6 membered heterocyclic ring" refers to a ring containing 5-6 ring atoms (at least one of which is a heteroatom, such as a nitrogen atom, an oxygen atom or a sulfur atom). Optionally, the ring atom (e.g., the carbon atom, the nitrogen atom or the sulfur atom) in the cyclic structure may be substituted with oxo. Examples include, but are not limited to, pyrrolidine, tetrahydrofuran, piperidine, piperazine, tetrahydropyran, and other rings.

In the present application, the term "aryl" refers to a monocyclic or polycyclic hydrocarbon group which is aromatic, such as 6-10 membered aryl, and 5-8 membered aryl. Specific examples include, but are not limited to, phenyl, naphthyl, anthryl, phenanthryl, etc. The "6-10 membered aryl" refers to aryl containing 6-10 ring atoms. The "C6-10 aryl" refers to aryl containing 6-10 carbon atoms.

In the present application, the term "heteroaryl" refers to a cyclic group having aromaticity, wherein at least one ring atom is a heteroatom such as a nitrogen atom, an oxygen atom or a sulfur atom. Optionally, the ring atom (e.g., the carbon atom, the nitrogen atom or the sulfur atom) in the cyclic structure may be substituted with oxo. Specific examples include, but are not limited to, 5-10 membered heteroaryl, 5-6 membered heteroaryl, 5-10 membered nitrogen-containing heteroaryl, 6-10 membered oxygen-containing heteroaryl, 6-8 membered nitrogen-containing heteroaryl, 5-8 membered oxygen-containing heteroaryl , etc., such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridinyl, 2-pyridonyl, 4-pyridonyl, pyrimidinyl, 1,4-dioxacyclohexadienyl, 2H-1,2-oxazinyl, 4H-1,2-oxazinyl, 6H-1,2-oxazinyl, 4H-1,3-oxazinyl, 6H-1,3-oxazinyl, 4H-1,4-oxazinyl, pyridazinyl, pyrazinyl, 1,2,3-triazinyl, 1,3,5-triazinyl, 1,2,4,5-tetrazinyl, azepinyl, 1,3-diazepinyl, and azocinyl.

The bond in the structural formula represented by a wave line "~~" in the present application is intended to represent that the structure represents a cis or trans isomer, or a mixture of cis and trans isomers in any proportion.

In the present application, the term "room temperature" refers to 25±5 °C.

In the present application, the protecting group and the method for linking or removing the protecting group may be achieved by using conventional methods in the art, and the method may be a one-step reaction or a multi-step reaction, for example, but not limited to, see "Greene's Protective Groups in Organic Synthesis - 4th Edition" published by Wiley (publisher) or "Protecting Group Chemistry" published by Chemical Industry Press.

In the present application, the term "amino protecting group" refers to a group protecting an amino group in a compound, and may be selected from amino protecting groups known in the art. The medium for the protection reaction according to the present application is preferably selected from aprotic solvents, preferably dichloromethane. The reaction of adding a protecting group for protecting the amino group may be carried out by using conventional reaction conditions well known to those skilled in the art. The reaction for removing the protecting group from the amino group may be carried out by using conventional reaction conditions well known to those skilled in the art. Preferably, the amino protecting group used in the present invention is selected from alkoxycarbonyl protecting groups, which may be unsubstituted or substituted, such as benzyloxycarbonyl, tert-butoxycarbonyl, fluorenylmethoxycarbonyl, allyloxycarbonyl, trimethylsilylethoxycarbonyl, methoxycarbonyl, or ethoxycarbonyl.

In the present application, the term "hydroxy protecting group" refers to a group protecting hydroxy in a compound, and may be selected from hydroxy protecting groups known in the art. The reaction for adding a protecting group for protecting hydroxy may be carried out by using conventional reaction conditions well known to those skilled in the art. The reaction for removing the protecting group from hydroxy may be carried out by using conventional reaction conditions well known to those skilled in the art. Preferably, the hydroxy protecting group used in the present invention is selected from benzyl, which may be unsubstituted or substituted, such as benzyl, *p*-nitrobenzyl, and *p*-methoxybenzyl.

In the present application, when the term "about" is used with a value or a range of values, it adjusts the value or the range of values by extending the boundary of the value upward and/or downward. For example, the term "about" is intended to modify a value by less than or equal to 20%, more preferably by less than or equal to 10%, both above and below the stated value.

In the present application, if the name and the structural formula of a compound are given to the compound at the same time, in the case where the name and the structural formula are not identical, the structure of the compound is taken as the reference unless the context indicates that the structure of the compound is incorrect and the name is correct.

In the present application, the compound may be in the form of a geometric isomer or stereoisomer. The present application contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present application. Additional asymmetric carbon atoms can be present in substituents such as an alkyl group. All these isomers and mixtures thereof are encompassed within the scope of the present invention.

The reagents and starting materials used in the present invention are commercially available.

The beneficial effects of the present invention are as follows:
Through a great deal of research, the present disclosure develops a synthesis method for preparing a drug linker conjugate containing an oxazaacetal structure ( 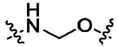 ), which is suitable for industrial production. The preparation method has the following advantages:
1) The applicability of the substrate is wide. It has very good applicability for molecules containing labile groups (e.g., amino, or substituted amino) in the presence of Pb(OAc)₄. It has very good applicability for substrates which are unstable to alkaline conditions, such as camptothecin compounds.
2) The separation and purification of the compound are simple and convenient to operate, the yield is high (the yield of key steps is up to 95%), and the method is suitable for industrial production.
3) The reaction condition is mild, the reaction is carried out under normal pressure, and the reaction temperature is easy to control.
4) The related starting materials are easy to obtain and low in price.
5) A1.9 (namely the compound III of the present application) is used as a starting material, and a target molecule can be obtained through a one-step reaction, so the reaction steps (the original route is three steps) involving high-activity molecules are greatly reduced.
6) The reaction yield is greatly increased. The overall yield of the original route is < 5% and the overall yield of the new route is > 50%, based on A1.9 (i.e., the compound III of the present application).
7) All the intermediates are simple in purification mode and easy to be scaled up.
8) All the intermediates have good stability and no polymer is generated.
9) The whole process is green and environment-friendly, which does not use class 1 and class 2A metals, and has no influence on the reaction and the final product caused by elemental impurity residues (for example, in the prior art, the use of Pb(OAc)₄ may cause the poisoning of a metal catalyst for removing an amino protecting group by subsequent metal catalytic hydrogenation, and the reaction yield is low).
10) In the whole process (especially when a Cbz protecting group is used, the method is more suitable for liquid-phase reaction), the corresponding intermediate has better stability, the reaction condition is greener, and a byproduct is toluene, which is more convenient to remove.

### DETAILED DESCRIPTION

### Sequence

Information about the sequences to which the present application relates is described in the following table.

| SEQ ID NO | Description | Sequence |
|---|---|---|
| 1 | 2E3-02 heavy chain | EVQLLESGGGLVQPGGSLRLSCAASGFTFSSYAMNWVRQAPGKGLEWVSGI SGSGGSTYYADSVQGRFTISRDNSKNTLYLQMNSLRAEDTAVYYCAKARSG YDYFFDYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNH |
| | | KPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLTV DKSRWQQGNVFSCSVMHEALHNHYTQKSLSLSPGK |
| 2 | 2E3-02 light chain | EIVLTQSPGTLSLSPGERATLSCRASQRISSSFLAWYQQKPGQAPSLLIYGASS RATGIPDRFSGSGSGTDFTLTISSLEPEDFAVFYCQQYSNSPLTFGGGTKVEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC |

All of the features disclosed in this specification, or all of the steps of any method or process disclosed, may be combined in any combination, except for features and/or steps that are mutually exclusive.

Any feature disclosed in this specification may be replaced by other alternative features serving an equivalent or similar purpose, unless otherwise stated. That is, unless otherwise specifically stated, each feature is only an example of a series of equivalent or similar features.

The implementation conditions used in the examples may be further adjusted according to specific requirements, and the unspecified implementation conditions are generally those used in routine experiments. The chemical reagents used in the following examples are all commercially available chemical reagents.

In conventional synthesis methods, as well as in the examples and intermediate synthesis examples, the meanings of the abbreviations or English expressions are shown in the following table:

| | | | |
|---|---|---|---|
| Cbz | Benzyloxycarbonyl | Alloc | Allyloxycarbonyl |
| Fmoc | 9-fluorenylmethoxycarbonyl | Boc | *Tert*-butoxycarbonyl |
| Teoc | Trimethylsilylethoxycarbonyl | Pd(PPh₃)Cl₂ | Bis(triphenylphosphine)palladiumchloride |
| HCl | Hydrogen chloride | PTSA | *p*-toluenesulfonic acid monohydrate |
| DMTMM | 4-(4,6-dimethoxytriazin-2-yl)-4-methylmorpholine hydrochloride | NaHCO₃ | Sodium bicarbonate |
| Na₂CO₃ | Sodium carbonate | K₂CO₃ | Potassium carbonate |
| Cs₂CO₃ | Cesium carbonate | Et₃N | Triethylamine |
| KHCO₃ | Potassium bicarbonate | DIPEA | *N,N*-diisopropylethylamine |
| THF | Tetrahydrofuran | Acetone | Acetone |
| CH₃CN | Acetonitrile | 1,4-dioxane | 1,4-dioxane |
| DMF | *N,N*-dimethylformamide | DMSO | Dimethyl sulfoxide |
| NMP | *N*-methylpyrrolidone | SnCl₂ | Stannous chloride |
| DCM | Dichloromethane | EA | Ethyl acetate |
| MeOH | Methanol | EtOH | Ethanol |
| MTBE | Methyl *tert*-butyl ether | h | Hours |
| min | Min | | |

In an exemplary embodiment of the present invention, the drug linker conjugate IIa is synthesized using the following routes:

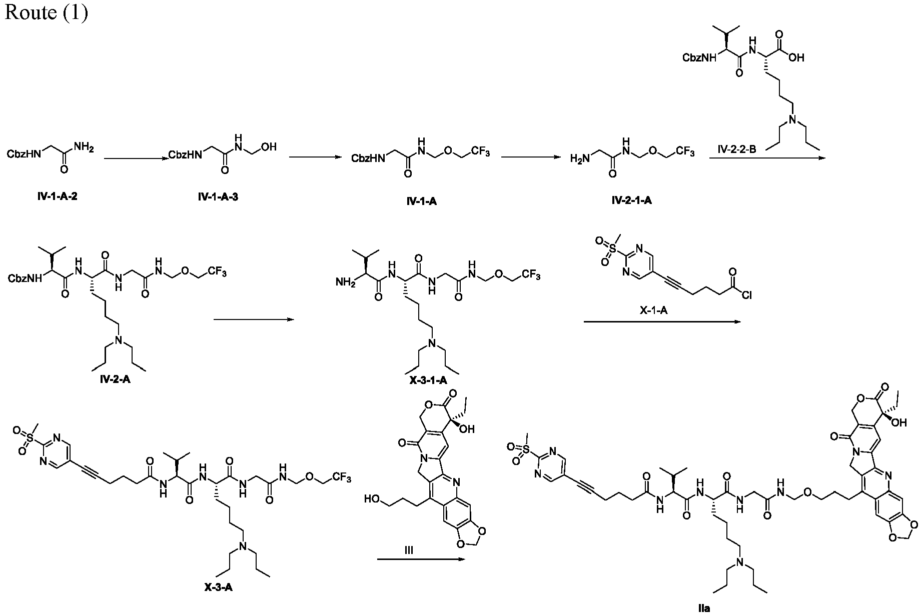

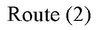

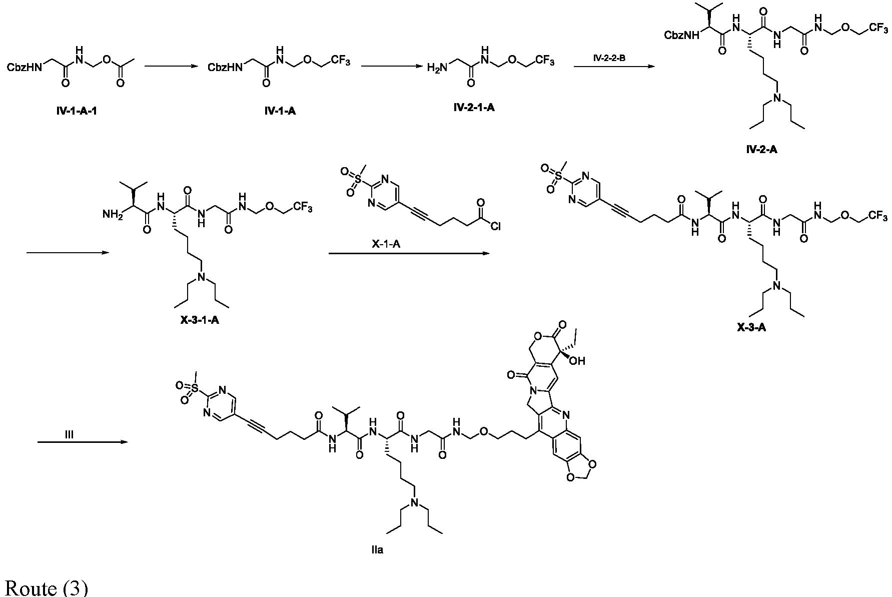

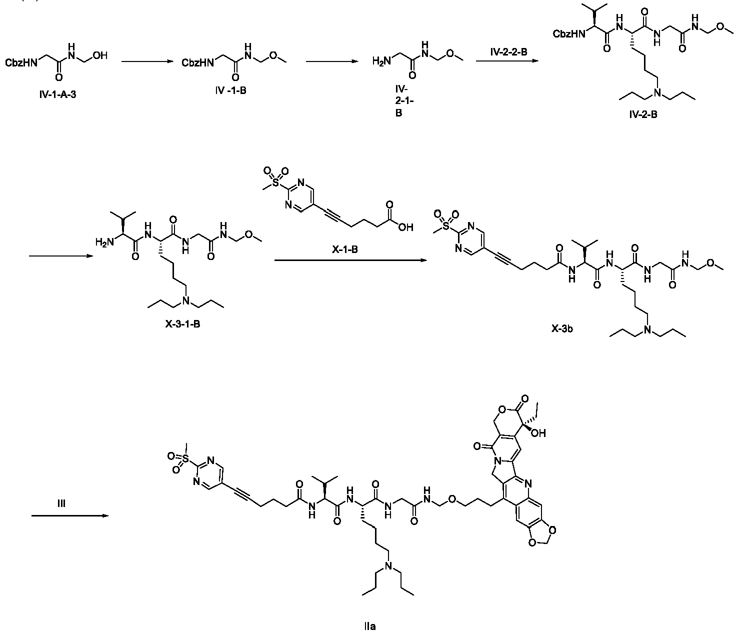

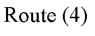

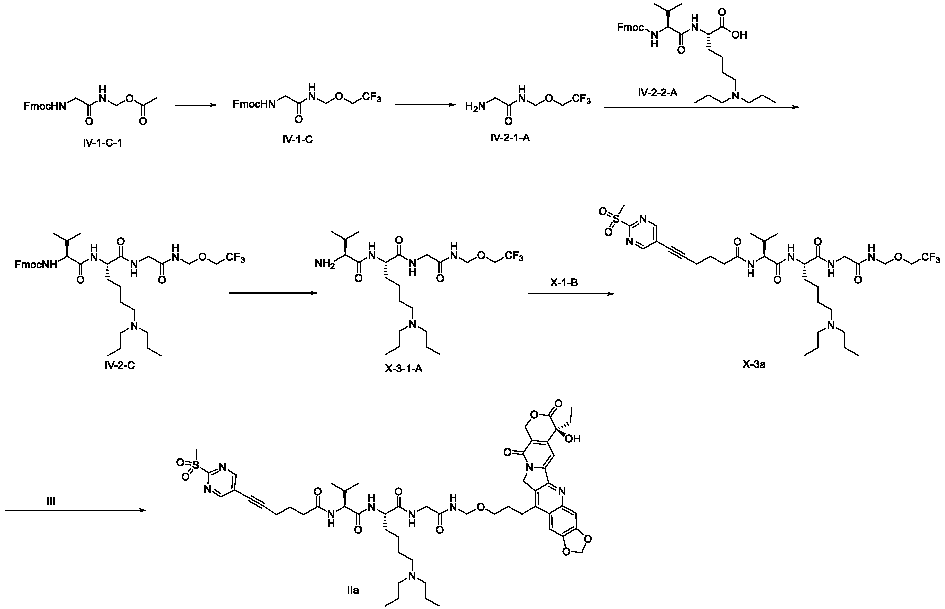

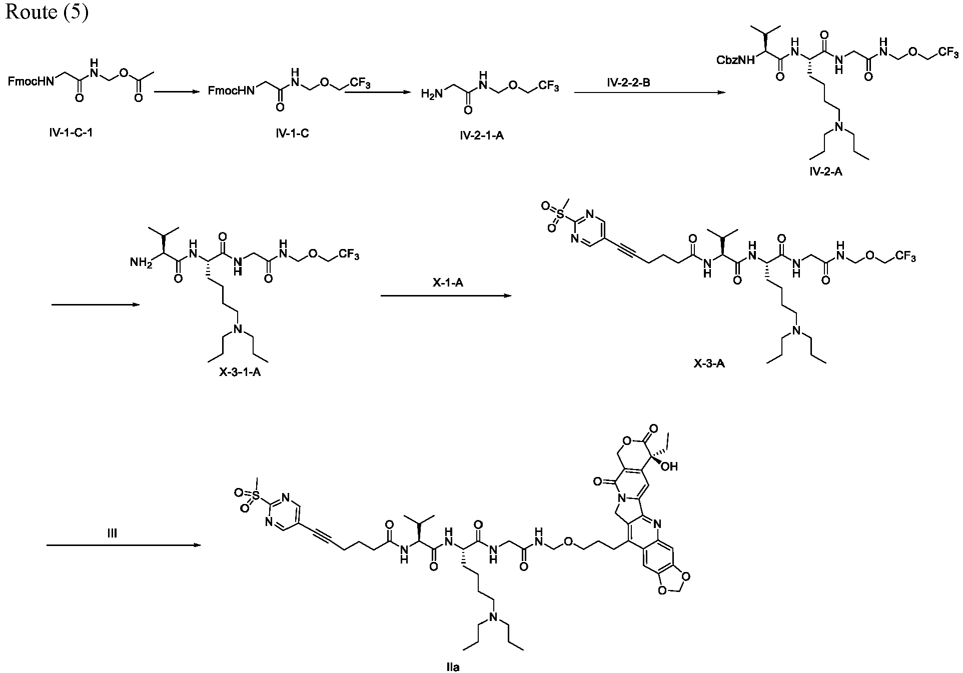

### Preparative HPLC chromatographic condition I

Chromatographic column: C18 preparative column (100 mm × 250 mm,10 µm), wavelength: 214 nm; flow rate: 250 mL/min; mobile phase A: 0.1% aqueous formic acid solution, mobile phase B: acetonitrile

### Gradient table:

| | | | | | | | | |
|---|---|---|---|---|---|---|---|---|
| Time/min | 0 | 8 | 18 | 30 | 35 | 45 | 50 | 55 |
| Mobile phase A (%) | 85 | 85 | 76 | 76 | 70 | 70 | 65 | 60 |
| Mobile phase B (%) | 15 | 15 | 24 | 24 | 30 | 30 | 35 | 40 |

### Preparative HPLC chromatographic condition II

### Step 1: purification

| | | | | | | |
|---|---|---|---|---|---|---|
| Chromatographic column | DAC100 (Filler YMC-ODS-AQ-HG, 10 µm) | | | | | |
| Mobile phase | Mobile phase A: ammonium acetate solution (5 g/L, adjusted pH to 4.4 with acetic acid) | | | | | |
| | Mobile phase B: acetonitrile | | | | | |

| Phase | Equilibrium stage | Elution stage | | | | |
|---|---|---|---|---|---|---|
| Time (min) | 10 | 0 | 2 | 3 | 43 | 60 |
| A% | 90 | 90 | 80 | 72 | 64 | 60 |
| B% | 10 | 10 | 20 | 28 | 36 | 40 |
| Flow rate | 240ml/min | | | | | |

### Step 2: desalting and concentration

| | | | | | |
|---|---|---|---|---|---|
| Chromatographic column | DAC100 (Filler YMC-ODS-AQ-HG, 10 µm) | | | | |
| Mobile phase | Mobile phase A: 0.1% aqueous formic acid solution; | | | | |
| | Mobile phase B: acetonitrile | | | | |

| Phase | Equilibrium stage | Elution stage | | | |
|---|---|---|---|---|---|
| Time (min) | 10 | 0 | 10 | 15 | 50 |
| A% | 100 | 100 | 100 | 20 | 20 |
| B% | 0 | 0 | 0 | 80 | 80 |
| Flow rate | 240ml/min | | | | |

### HPLC chromatographic conditions (method I)

| Chromatographic conditions | Chromatographic parameters | | | | | |
|---|---|---|---|---|---|---|
| Chromatographic column | Waters CORTECS T3, 3.0 × 100 mm 2.7 µm | | | | | |
| Detection wavelength | UV 254 nm | | | | | |
| Injection volume | 5 µl | | | | | |
| Column temperature | 40 °C | | | | | |
| Flow rate | 0.8 ml/min | | | | | |
| Elution time | 18 min (gradient elution) | | | | | |
| Diluent | 0.1% trifluoroacetic acid-acetonitrile (60-40) | | | | | |
| Needle wash solution | Acetonitrile | | | | | |
| Sample concentration | 0.5mg/ml | | | | | |
| Mobile phase A | 0.1% trifluoroacetic acid | | | | | |
| Mobile phase B | Acetonitrile | | | | | |

| Elution program | | | | | | |
|---|---|---|---|---|---|---|
| Time (min) | 0 | 8 | 12 | 15 | 15.1 | 18 |
| Mobile phase A | 80 | 50 | 10 | 10 | 80 | 80 |
| Mobile phase B | 20 | 50 | 90 | 90 | 20 | 20 |

### Chiral HPLC chromatographic conditions

| Name | Parameter |
|---|---|
| Chromatographic column | CHIRALPAK AGP 0.4cm I.D. ×15cm L ×5.0µm |
| Mobile phase | 50 mM ammonium acetate solution (pH 7.0)-isopropanol (96/4) |
| Flow rate | 0.5ml/min |
| Injection volume | 3µl |
| Wavelength | 254nm |
| Column temperature | 20 °C |
| Sample solution | 2 mg/mL, dissolved and mixed with 10 mM ammonium formate solution (pH 4.5). |

### Synthesis of drug linker conjugate IIa

### Example 1: Synthesis of Intermediate III

### Step 1: Preparation of 4-(6-nitrobenzo[d][1,3]dioxol-5-yl)but-3-yn-1-ol (III-2)

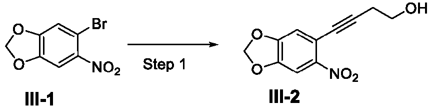

Compound III-1 (100 g, 408 mmol) and 3-butyn-1-ol (11.4 g, 163 mmol) were dissolved in NMP (300 mL), then triethylamine (102 g, 1.02 mol, 141 mL) was added, and the reaction system was reacted under nitrogen atmosphere. CuI (3.88 g, 20.4 mmol) and Pd(PPh₃)₂Cl₂ (2.9 g, 4.1 mmol) were added sequentially and reacted at 50 °C for 0.5 h. 3-butyn-1-ol (22.8 g, 326 mmol) was added dropwise, and after the dropwise addition was completed, the reaction was continued at 50 °C for 4 h. The reaction solution was cooled to room temperature and poured into water (4.5 L) containing ammonia water (3.26 mol) to precipitate a solid, followed by stirring for 0.5 h. The reaction system was filtered under vacuum, and the filter cake was added to water (4.5 L) and stirred for 0.5 h. The reaction system was filtered under vacuum, and the filter cake was added to water (4.5 L) and stirred for 0.5 h. The reaction system was filtered under vacuum, and the filter cake was dried to obtain the target product III-2 (85.0 g, yield: 88%).
LCMS (ESI) [M+H]⁺ = 236.0
¹H NMR (400 MHz, DMSO-*d₆*) δ 7.66 (s, 1H), 7.18 (s, 1H), 6.25 (s, 2H), 4.91 (*t, J =* 5.6 Hz, 1H), 3.59 (dd, *J* = 12.6, 6.7 Hz, 2H), 2.59 (t, *J =* 6.9 Hz, 2H).

### Step 2: Preparation of 1-(6-aminobenzo[d][1,3]dioxol-5-yl)-4-hydroxybutan-1-one (III-3)

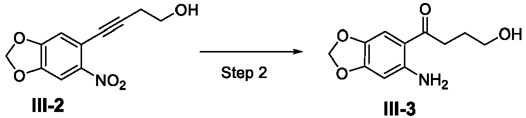

Compound III-2 (8.42 g, 35.79 mmol) was dissolved in a solution of EtOH/H₂O (v:v = 9:1, 152 mL), Sn (8.46 g, 71.58 mmol), Na₂S•9H₂O (2.58 g, 10.74 mmol), and concentrated hydrochloric acid (30 mL, 358 mmol) were added, and the reaction system was stirred at 78 °C for 1 h. The reaction solution was filtered under vacuum through celite while it was hot, and the filtrate was concentrated to obtain a crude product, which was slurried with ethyl acetate (20 mL), and then filtered under vacuum, and the filter cake was washed twice with ethyl acetate (5 mL) to obtain the crude product. Water (50 mL) was added to the crude product, a saturated sodium bicarbonate solution was added dropwise to adjust the pH value to 8.0, and the reaction system was stirred at room temperature for 1 h. The reaction system was filtered under vacuum, and the filtrate was extracted with ethyl acetate (50 mL × 2), and concentrated to obtain a solid; the solid obtained by concentration and the filter cake were combined and dried to obtain the target product III-3 (3.6 g).
LCMS (ESI) [M-18+H]⁺ =206.2
¹H NMR (400 MHz, CD₃OD) δ 7.18 (s, 1H), 6.24 (s, 1H), 5.87 (s, 2H), 3.62 (t, *J =* 6.7 Hz, 2H), 2.95-2.84 (m, 2H), 1.95-1.82 (m, 2H).

### Step 3: Preparation of (S)-7-ethyl-7-hydroxy-14-(3-hydroxypropyl)-10,13-dihydro-11H-[1,3]dioxolo[4,5-g]pyrano[3',4':6,7]indolizino[1,2-b]quinoline-8,11(7H)-dione (III)

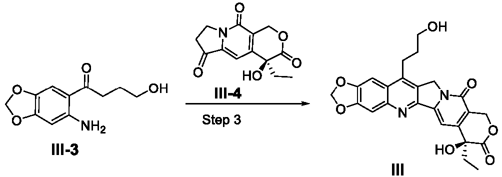

Compound III-3 (41.80 g, 0.19 mol), III-4 (110.00 g, 0.42 mol) and *p*-toluenesulfonic acid monohydrate (31.90 g, 0.17 mol) were added to a three-necked flask, 550 mL of NMP was measured and added to the flask, and the mixture was heated and stirred at 110 °C under nitrogen atmosphere . III-3 (31.35 g, 0.14 mol) was added to the reaction system for a total of 3 times at intervals of 0.5 h. After the addition was completed, the reaction system was stirred for another 1 h. Isopropanol (110 mL) was added to the reaction solution, water (1.32 L) was then added dropwise, and the reaction system was cooled to 5 °C and stirred for 1.0 h. After filtration under vacuum, the filter cake was washed with water and slurried with water (13.75 L) for 2 h. After filtration under vacuum, the filter cake was washed again with water. The filter cake was added to a reaction flask, DMF (165 mL) and methanol (1.32 L) were added sequentially, and the mixture was stirred for 12 h at 25 °C. After filtration under reduced pressure, the filter cake was washed with methanol, and dried under vacuum for 48 h at 50 °C to obtain the target product III (184.0 g).
LCMS (ESI) [M+H]⁺ = 451.4
¹H NMR (400 MHz, DMSO-*d₆*) δ 7.59 (s, 1H), 7.49 (s, 1H), 7.25 (s, 1H), 6.49 (s, 1H), 6.30 (s, 2H), 5.54 - 5.36 (m, 2H), 5.19 (s, 2H), 4.68 (t, *J =* 5.0 Hz, 1H), 3.51 (q, *J =* 5.6 Hz, 2H), 3.14 (t, *J =* 7.6 Hz, 2H), 1.99-1.73 (m, 4H), 0.92 (t, *J =* 7.3 Hz, 3H).

### Example 2: Synthesis of Intermediate IV-2-2

### I. Synthesis of intermediate IV-2-2-A

### Step 1: Preparation of N²-((((9H-fluoren-9-yl)methoxy)carbonyl)-L-valyl)-N⁶-(tert-butoxycarbonyl)-L-lysine (IV-2-2-A-3)

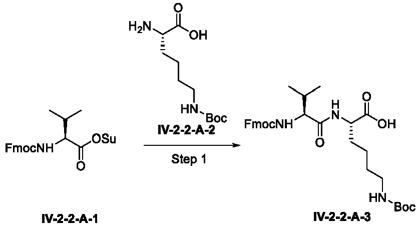

Compound IV-2-2-A-1 (100 g, 0.229 mol) and compound IV-2-2-A-2 (56.4 g, 0.229 mol) were placed in a reaction flask, acetone/water (1 L, v:v = 1:1) was added with stirring, and NaHCO₃ (76.95 g, 0.916 mol) was then added. The mixture was stirred at room temperature overnight, and filtered to remove the insoluble substances. The filtrate was concentrated, hydrochloric acid (4 N) was added to the concentrated solution to adjust the pH value to 5, and the reaction system was extracted with ethyl acetate (0.75 L × 2). The organic phases were combined and washed with saturated brine (300 mL × 2), and dried over anhydrous sodium sulfate. The filtrate was concentrated under reduced pressure to obtain the target compound IV-2-2-A-3 (117 g, yield: 90%).
LCMS (ESI) [M+H]⁺ = 568.3
¹H NMR (400 MHz, DMSO-*d₆*) δ 7.91 (d, *J =* 7.5 Hz, 2H), 7.82-7.73 (m, 2H), 7.64 (d, *J =* 6.9 Hz, 1H), 7.58 (d, *J =* 9.1 Hz, 1H), 7.44 (t, *J =* 7.4 Hz, 2H), 7.40-7.31 (m, 2H), 6.68 (brs, 1H), 4.38-4.21 (m, 3H), 3.99-3.91 (m, 1H), 3.87 (dd, *J =* 8.8, 6.8 Hz, 1H), 2.92-2.80 (m, 2H), 2.17-2.01 (m, 1H), 1.70-1.67 (m, 1H), 1.64-1.52 (m, 1H), 1.42-1.16 (m, 13H), 0.93-0.83 (m, 6H).

### Step 2: Preparation of (((9H-fluoren-9-yl)methoxy)carbonyl)-L-valyl-L-lysine hydrochloride (IV-2-2-A-4)

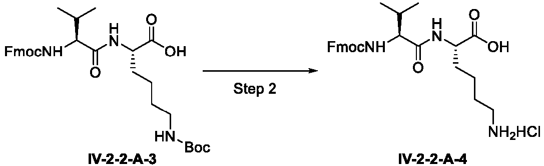

Compound IV-2-2-A-3 (117 g, 206.2 mmol) was added to dioxane (1.5 L) and stirred for 10 min, then HCl/dioxane (0.75 L, 4 mol/L) was added, and the mixture was stirred at room temperature for 12 h. Methyl *tert-butyl* ether (2 L) was added to the reaction solution, the reaction solution was stirred for 30 min and filtered, and the filter cake was dried to obtain the target compound IV-2-2-A-4 (94 g, yield: 91%).
LCMS (ESI) [M+H]⁺ = 468.3
¹H NMR (400 MHz, DMSO-*d₆*) δ 8.24 (d, *J =* 7.3 Hz, 1H), 8.07 (brs, 3H), 7.90 (d, *J =* 7.5 Hz, 2H), 7.77 (dd, *J =* 7.1, 3.4 Hz, 2H), 7.47-7.37 (m, 3H), 7.34 (td, *J =* 7.4, 1.7 Hz, 2H), 4.34-4.17 (m, 4H), 3.96 (dd, *J =* 8.6, 7.4 Hz, 1H), 2.83-2.65 (m, 2H), 2.04 (dd, *J =* 13.5, 6.7 Hz, 1H), 1.82-1.52 (m, 4H), 1.51-1.30 (m, 2H), 0.96-0.84 (m, 6H).

### Step 3: Preparation of N²-((((9H-fluoren-9-yl)methoxy)carbonyl)-L-valyl)-N⁶, N⁶-dipropyl-L-lysine (IV-2-2-A)

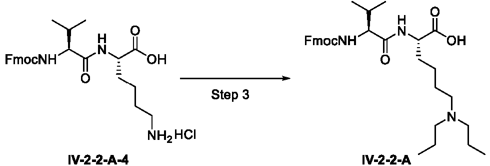

Compound IV-2-2-A-4 (55.0 g, 109.1 mmol) and acetic acid (10 mL) were dissolved in a solution of methanol (500 mL), *n*-propionaldehyde (25.35 g, 436.4 mmol) was added to the reaction solution in an ice-water bath, followed by stirring for reaction at room temperature for 30 min, and then sodium cyanoborohydride (27.43 g, 436.4 mmol) was added to the reaction solution in an ice-water bath, followed by stirring for reaction at room temperature for 1 h. After the starting materials were not completely consumed as shown by LCMS, *n-*propionaldehyde (12.67 g, 218.2 mmol) and sodium cyanoborohydride (13.72 g, 218.2 mmol) were added, and the reaction was continued for 0.5 h. The reaction solution was filtered, and the filtrate was concentrated. Purified water (300 mL) was added to the concentrated solution, and the resulting mixture was extracted with DCM (300 mL × 2). The organic phase was washed with saturated brine, dried over anhydrous Na₂SO₄ and concentrated to obtain a crude product. The crude product was purified by column chromatography (eluents: MeOH and DCM) to obtain compound IV-2-2-A (39 g, yield: 65%).
LCMS (ESI) [M+H]⁺ = 552.3
¹H NMR (400 MHz, DMSO-*d₆*) δ 7.95-7.85 (m, 3H), 7.76 (t, *J =* 7.3 Hz, 2H), 7.51-7.40 (m, 3H), 7.35 (t, *J* = 7.4 Hz, 2H), 4.34-4.22 (m, 3H), 4.13 (dd, *J =* 12.6, 7.2 Hz, 1H), 3.91 (dd, *J = 8.8,* 7.1 Hz, 1H), 2.52 - 2.41 (m, 6H), 2.03 (dt, *J =* 13.5, 6.7 Hz, 1H), 1.79-1.67 (m, 1H), 1.66-1.56 (m, 1H), 1.42-1.36 (m, 6H), 1.36-1.21 (m, 2H), 0.90 (t, *J* = 6.9 Hz, 6H), 0.82 (t, *J =* 6.9 Hz, 6H).

### II. Synthesis of intermediate IV-2-2-B

### Step 1: Synthesis of N-((benzyloxy)carbonyl)-L-valyl)-N-(tert-butoxycarbonyl)-L-lysine (IV-2-2-B-3)

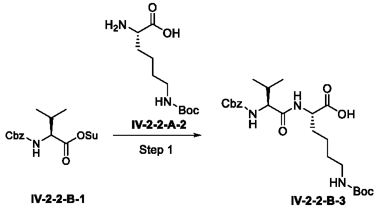

### Method I:

Compound IV-2-2-B-1 (50.0 g, 143.53 mmol) and compound IV-2-2-A-2 (35.35 g, 143.53 mmol) were weighed and dissolved in acetone (250 mL) and water (250 mL), then NaHCO₃ (48.23 g, 574.12 mmol) was added, and the mixture was stirred overnight at room temperature, and then filtered under vacuum. The filtrate was concentrated to remove acetone, hydrochloric acid (4 mol/L) was added to adjust the pH value to 5, and a large amount of solid precipitated. After filtration under vacuum, and the filter cake was rinsed with water (100 mL), then slurried with DCM (700 mL) for 30 min, filtered under vacuum, and dried at 50 °C to obtain the target product IV-2-2-B-3 (65.0 g, yield: 94.5%).

### Method II:

Compound IV-2-2-A-2 (35.35 g, 143.53 mmol) was weighed and added to a solution of NaHCO₃ (24.12 g, 287.06 mmol) in water (250 mL), and a solution of IV-2-2-B-1 (50.0 g, 143.53 mmol) in DCM (250 mL) was added dropwise in an ice-water bath. After the dropwise addition was completed, the reaction system was stirred at room temperature for 10 h. Hydrochloric acid (4 mol/L) was added to the reaction system to adjust the pH value to 5, the reaction system was stirred for 1 h and then filtered under vacuum, and the filter cake was dried to obtain the target product IV-2-2-B-3 (65.0 g, yield: 94.5%).

### Method III:

Compound IV-2-2-A-2 (35.35 g, 143.53 mmol) was weighed and added to a solution of NaHCO₃ (24.12 g, 287.06 mmol) in water (250 mL), and a solution of IV-2-2-B-1 (50.0 g, 143.53 mmol) in THF (250 mL) was added dropwise in an ice-water bath. After the dropwise addition was completed, the reaction system was stirred for 4 h at room temperature, the reaction solution was concentrated to remove the organic solvent, and hydrochloric acid (4 mol/L) was added to adjust the pH value to 5. The reaction system was stirred for 1 h and then filtered under vacuum, and the filter cake was slurried with DCM (700 mL) for 30 min and then filtered under vacuum, and dried to obtain the target product IV-2-2-B-3 (65.0 g, yield: 94.5%).
LCMS (ESI) [M+H-Boc]⁺ = 380.31
¹H NMR (400 MHz, DMSO-*d₆*) δ 8.07 (d, *J =* 8.0 Hz, 1H), 7.36 - 7.30 (m, 5H), 7.23 (d, *J =* 8.0 Hz, 1H), 6.74 (t, *J =* 8 Hz, 1H), 5.04 (s, 2H), 4.18-4.12 (m, 1H), 3.94-3.90 (m, 1H), 2.91-2.87 (m, 2H), 1.97-1.85 (m, 1H), 1.74 - 1.64 (m, 1H), 1.62 - 1.52 (m, 1H), 1.42 - 1.23 (m, 13H), 0.90 - 0.83 (m, 6H).

### Step 2: Synthesis of ((benzyloxy)carbonyl)-L-valyl-L-lysine hydrochloride (IV-2-2-B-4)

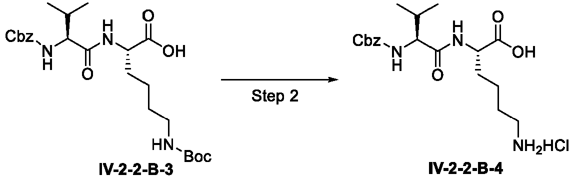

### Method I:

Compound IV-2-2-B-3 (65.0 g, 135.54 mmol) was added to dioxane (520 mL) and stirred for 10 min, then 260 mL of HCl (4 mol/L in dioxane) was added, and the mixture was stirred overnight at room temperature. MTBE (1300 mL) was added to the reaction solution, the reaction system was stirred for 30 min and then filtered, and the filter cake was dried to obtain the target compound IV-2-2-B-4 (52 g, yield: 93%).

### Method II:

Compound IV-2-2-B-3 (50.0 g, 104.26 mmol) was weighed and added to tetrahydrofuran (20 mL), then concentrated hydrochloric acid (17.2 mL, 206.40 mmol) was added, and the mixture was heated and reacted at 60 °C for 1 h. The reaction solution was cooled to room temperature and directly used in the next step.
LCMS (ESI) [M+H]⁺ = 380.42
¹H NMR (400 MHz, DMSO-*d₆*) δ 12.59 (s, 1H), 8.19 (d, *J =* 8.0 Hz, 1H), 7.96 (s, 3H), 7.40 - 7.34 (m, 5H), 8.27 (d, *J =* 8.0 Hz, 1H), 5.06 (s, 2H), 4.22 - 4.17 (m, 1H), 3.96 - 3.92 (m, 1H), 2.80 - 2.77 (m, 2H), 2.04-2.00 (m, 1H), 1.63 - 1.57 (m, 4H), 1.41 - 1.39 (m, 2H), 0.93 - 0.87 (m, 6H).

### Step 3: Synthesis of N-((benzyloxy)carbonyl)-L-propionyl)-N,N-dipropyl-L-lysine (IV-2-2-B)

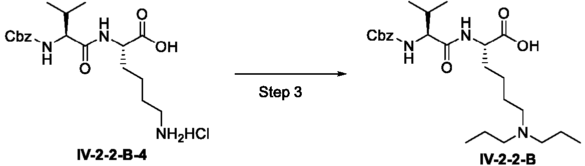

### Method I:

After compound IV-2-2-B-4 (52 g, 125.03 mmol) obtained in method I of step 2 was dissolved in methanol (520 mL) with stirring, sodium cyanoborohydride (15.71 g, 250.06 mmol) was added to the reaction solution, and *n*-propionaldehyde (14.52 g, 250.06 mmol) was added and reacted for 1 h. *n*-propionaldehyde (14.52 g, 250.06 mmol) was added and reacted for 1 h. Water (45 mL, 2.5 mol) was added to the reaction solution, the reaction mixture was stirred for 10 min and then concentrated, and THF (200 mL) was added to the concentrated solution for concentration to remove water. The above operation was repeated once. The residue was dissolved in DCM (250 mL) and filtered to remove the insoluble substances, and the filtrate was concentrated to obtain a crude product of the target compound IV-2-2-B, which was directly used in the next step in 100% yield.

### Method II:

THF (300 mL) was added to the solution obtained in method II of step 2, sodium cyanoborohydride (19.7 g, 312.78 mmol) was added in an ice-water bath, and n-propionaldehyde (24.3 g, 417.04 mmol) was added. The reaction system was heated to room temperature and stirred for 1 h. Hydrochloric acid was added to adjust the pH value to 4-5, followed by stirring for 30 min. The reaction system was concentrated to remove THF, water (200 mL) and DCM (200 mL) were added, and the organic phase was collected. 60.0 g of anhydrous sodium sulfate was added to the aqueous phase, followed by re-extraction with DCM (100 mL). The organic phases were combined, dried over anhydrous sodium sulfate, and filtered to remove the insoluble substances. The residue was concentrated to obtain the target compound (38.0 g, two-step yield: 78.6%).
LCMS (ESI) [M+H]⁺ = 464.44
¹H NMR (400 MHz, DMSO-*d₆*) δ 7.93 (d, *J =* 8.0 Hz, 1H), 7.35 - 7.26 (m, 6H), 5.03 (s, 2H), 4.15 - 4.10 (m, 1H), 3.91 - 3.87 (m, 1H), 2.76 - 2.58 (m, 6H), 2.00 - 1.96 (m, 1H), 1.82 - 1.19 (m, 10H), 0.89 - 0.84(m, 12H).

### Example 3: Synthesis of Intermediate IV-1

### I. Synthesis of IV-1-A

### Route I:

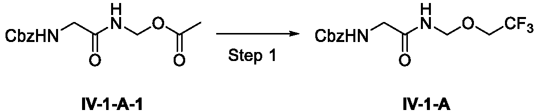

### Method I:

Compound IV-1-A-1 (5 g, 17.84 mmol) was dissolved in trifluoroethanol (30 mL) under argon atmosphere with stirring at 40 °C, then a solution of HCl in DMF (2.1 mL, 0.4 mol/L) was added, and the mixture was stirred at 40 °C for 2.5 h. The reaction solution was cooled to room temperature and diluted with 200 mL of ethyl acetate, and the organic phase was washed with 2% sodium bicarbonate solution (200 mL), extracted, and collected. The organic phase was washed with a saturated aqueous sodium chloride solution (200 mL), dried over anhydrous sodium sulfate, and filtered, and the filtrate was subjected to rotary evaporation to dryness to obtain the target compound IV-1-A (5.5 g, yield: 96.3%).

### Method II:

Compound IV-1-A-1 (5 g, 17.84 mmol) was dissolved in trifluoroethanol (30 mL) under argon atmosphere with stirring at 40 °C, then a solution of HCl in DMF (2.1 mL, 0.4 mol/L) was added, and the mixture was stirred at 40 °C for 2.5 h. The reaction solution was cooled to room temperature and then added to 120 mL of an aqueous sodium bicarbonate solution (1%, W/V), the reaction system was stirred and filtered under vacuum, and the filter cake was dried to obtain the target compound IV-1-A (5.3 g, yield: 92.8%).

¹H NMR (400 MHz, DMSO-*d₆*) δ 8.88 (t, J = 6.5 Hz, 1H), 7.54 (t, J = 6.0 Hz, 1H), 7.40-7.32 (m, 5H), 5.07 (s, 2H), 4.70 (d, J = 6.8 Hz, 2H), 4.03 (q, J = 9.4 Hz, 2H), 3.68 (d, J = 6.1 Hz, 2H).

### Route II:

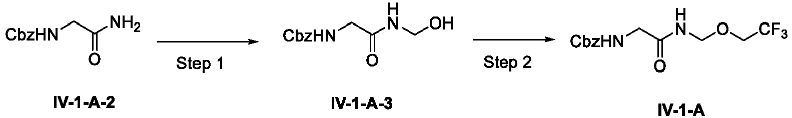

### Step 1: Synthesis of benzyl(2-((hydroxymethyl)amino)-2-oxoethyl)carbamate (IV-1-A-3)

### Method I:

Water (156 mL), an aqueous formaldehyde solution (7.3 g, 90 mmol) and K₂CO₃ (0.69 g, 5 mmol) were added to IV-1-A-2 (10.4 g, 50 mmol), and the mixture was reacted at room temperature for 14 h, filtered, rinsed with water (50 mL) for 2 times, and then dried under vacuum at 40 °C for 4 h to obtain the product (9.23 g, yield: 77.5%).

### Method II:

Water (156 mL), an aqueous formaldehyde solution (6.0 g, 75 mmol) and K₂CO₃ (0.69 g, 5 mmol) were added to IV-1-A-2 (10.4 g, 50 mmol), and the mixture was reacted at room temperature for 14 h, filtered, rinsed with water (50 mL) for 2 times, and then dried under vacuum at 40 °C to obtain the product (7.38 g, yield: 62%).
LCMS (ESI) [M+Na]⁺ = 261.2
¹H NMR (400 MHz, DMSO-*d*₆): δ 8.48 (t, J = 6.1 Hz, 1H), 7.47 (t, J = 6.0 Hz, 1H), 7.44 - 7.25 (m, 5H), 5.60 (t, J = 6.6 Hz, 1H), 5.06 (s, 2H), 4.54 (t, J = 6.4 Hz, 2H), 3.62 (d, J = 6.1 Hz, 2H).

### Step 2: Synthesis of benzyl (2-oxo-2-(((2,2,2-trifluoroethoxy)methyl)amino)ethyl)carbamate (IV-1-A)

### Method I:

Trifluoroethanol (14.4 mL, 6 V) and HCl-DMF (1.25 mL, 0.5 mmol, 0.4 mol/L) were added to compound IV-1-A-3 (2.38 g, 10 mmol), and the mixture was reacted at 40 °C for 3 h. The reaction system was cooled to 20 °C and then added dropwise to 2% NaHCO₃ aqueous solution (58 mL). The reaction system was stirred for 0.5 h, filtered, rinsed with water (12 mL) for 2 times, and dried under vacuum at 40 °C for 2 h to obtain the product (2.61 g, yield: 81.5%, purity: 98%).

### Method II:

Trifluoroethanol (60 mL) and BF₃-Et₂O (0.3 g, 2.1 mmol, 48%) were added to compound IV-1-A-3 (10.0 g, 42 mmol), and the mixture was reacted at 40 °C for 3 h. The reaction system was cooled to 20 °C and then added dropwise to 2% NaHCO₃ aqueous solution (245 mL). The reaction system was stirred for 0.5 h, filtered, rinsed with water (50 mL) for 2 times, and dried under vacuum at 40 °C to obtain the product (10.6 g, yield: 78.8%, purity: 97%).

### Method III:

Trifluoroethanol (60 mL) and HCl-DMF (63 mL, 25.2 mmol, 0.4 mol/L) were added to compound IV-1-A-3 (10.0 g, 42 mmol), and the mixture was reacted at 40 °C for 3 h. The reaction system was cooled to 20 °C and then added dropwise to 2% NaHCO₃ aqueous solution (245 mL). The reaction system was stirred for 0.5 h, filtered, rinsed with water (50 mL) for 2 times, and dried under vacuum at 40 °C to obtain the product (9.5 g, yield: 70.6%, purity: 88%).
LCMS (ESI) [M+Na]⁺ = 343.2
¹H NMR (400 MHz, DMSO-*d*₆): δ 8.91 (t, J = 6.8 Hz, 1H), 7.57 (t, J = 6.1 Hz, 1H), 7.46 - 7.27 (m, 5H), 5.07 (s, 2H), 4.71 (d, J = 6.8 Hz, 2H), 4.04 (q, J = 9.4 Hz, 2H), 3.70 (d, J = 6.1 Hz, 2H).

### II. Synthesis of IV-1-B

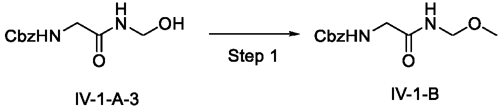

Methanol (120 mL) and a solution of HCl-DMF (10.5 mL, 0.4 mol/L) were added to IV-1-A-3 (20.0 g, 84 mmol), and the mixture was reacted at 40 °C for 4 h. The reaction solution was added dropwise to an aqueous sodium bicarbonate solution (720 mL, 2% wt), and concentrated under reduced pressure at 40 °C in a water bath to remove MeOH, and the residue was extracted with EA (200 mL × 2). The organic phases were combined, washed once with a saturated aqueous sodium chloride solution (100 mL), dried over anhydrous sodium sulfate for 0.5 h, and concentrated under reduced pressure to dryness, and the resulting product was dried under vacuum to obtain IV-1-B (19.4 g, yield: 91.5%).
LCMS (ESI) [M+Na]⁺ = 275.19
¹H NMR (400 MHz, DMSO-*d*₆): δ 8.67 (s, 1H), 7.53 (s, 1H), 7.46-7.25 (m, 5H), 5.07 (s, 2H), 4.50 (s, 2H), 3.67 (s, 2H), 3.19 (s, 3H).

### III. Synthesis of IV-1-C

### Step 1: Preparation of (9H-fluoren-9-yl)methyl (2-oxo-2-(((2,2,2-trifluoroethoxy)methyl)amino)ethyl)carbamate (IV-1-C)

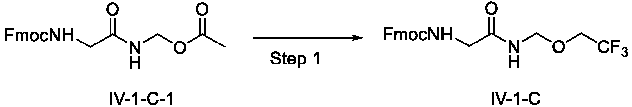

Compound IV-1-C-1 (10.00 g, 27.15 mmol) was dissolved in 2,2,2-trifluoroethanol (60 mL) with stirring at 40 °C, then HCl/DMF (3.4 mL, 0.4 M) was added to the reaction solution, and the reaction solution was stirred at 40 °C for 2.0 h. Ethyl acetate (200 mL) was added to the reaction solution for dilution with stirring, and the reaction system was washed and extracted with 2% sodium bicarbonate solution (200 mL) and saturated brine solution (200 mL). The organic phase was separated, dried over anhydrous sodium sulfate, filtered, and concentrated to obtain the target product (IV-1-C) (10.51 g, yield: 94.8%).

¹H NMR (400 MHz, DMSO-*d₆*) δ 8.87 (t, *J* = 6.8 Hz, 1H), 7.92 (d, *J* = 7.5 Hz, 2H), 7.74 (d, *J =* 7.5 Hz, 2H), 7.62 (t, *J =* 6.3 Hz, 1H), 7.45 (t, *J =* 7.4 Hz, 2H), 7.36 (td, *J =* 7.4, 1.2 Hz, 2H), 4.70 (d, *J =* 6.8 Hz, 2H), 4.33 (d, *J* = 6.9 Hz, 2H), 4.29-4.22 (m, 1H), 4.03 (q, *J* = 9.4 Hz, 2H), 3.68 (t, *J* = 3.1 Hz, 2H).

### IV. Synthesis of IV-1-D

### Step 1: Synthesis of (9H-fluoren-9-yl)methyl (2-(((benzyloxy)methyl)amino)-2-oxoethyl)carbamate (IV-1-D)

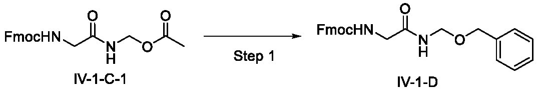

Compound IV-1-C-1 (50.0 g, 0.135 mol) was weighed and dissolved in DMF (300 mL), and benzyl alcohol (17.6 g, 0.163 mol) was added. HCl/EA (4 mol/L, 20.36 mL) was added in one portion with stirring at 25 °C, and the reaction was continued with stirring for 4 h. Ethyl acetate (1.5 L) was added to the reaction solution. The reaction system was washed with 2% sodium bicarbonate (1 L), water (1 L × 2) and brine (1 L), dried over anhydrous sodium sulfate, and concentrated to dryness to obtain a crude product. The crude product was purified by silica gel column chromatography (eluent: EA/PE = 4/6) and dried to obtain the target product IV-1-D (40.0 g, yield: 71%).
LCMS (ESI) [M+H]⁺ = 417.2

### V. Synthesis of IV-1-E

### Step 1: Synthesis of (9H-fluoren-9-yl)methyl (2-((isopropoxymethyl)amino)-2-oxoethyl)carbamate (IV-1-E)

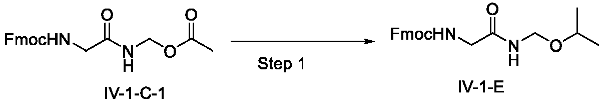

Compound IV-1-C-1(10.00 g, 27.15 mmol) was dissolved in isopropanol (60 mL) with stirring at 40 °C, and then HCl/DMF (3.4 mL, 0.4 M) was added to the reaction solution, followed by stirring at 40 °C for 2.0 h. Ethyl acetate (200 mL) was added to the reaction solution for dilution with stirring, and the reaction system was washed and extracted with 2% sodium bicarbonate solution (200 mL) and saturated brine solution (200 mL). The organic phase was separated, dried over anhydrous sodium sulfate, filtered, and concentrated to obtain the target product (9.50 g, yield: 95.0%).

¹H NMR (400 MHz, DMSO-*d₆*) δ 8.56 (t, *J =* 6.6 Hz, 1H), 7.92 (d, *J =* 7.5 Hz, 2H), 7.75 (d, *J =* 7.4 Hz, 2H), 7.56 (t, *J =* 6.2 Hz, 1H), 7.45 (t, *J =* 7.4 Hz, 2H), 7.36 (t, *J =* 7.4 Hz, 2H), 4.57 (d, *J =* 6.5 Hz, 2H), 4.37-4.21 (m, 3H), 3.77-3.60 (m, 3H), 1.09 (dd, *J* =6.2,1.9 Hz, 6H).

### V. Synthesis of IV-1-F

### Step 1: Synthesis of (9H-fluoren-9-yl)methyl (2-oxo-2-(((2,2,3,3,3-pentafluoropropoxy)methyl)amino)ethyl)carbamate (IV-1-F)

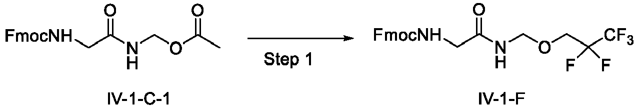

Compound IV-1-C-1 (10.00 g, 27.15 mmol) was dissolved in 2,2,3,3,3-pentafluoro-1-propanol (40 mL) with stirring at 40 °C, then HCl/DMF (3.4 mL, 0.4 M) was added to the reaction solution, and the reaction solution was stirred at 40 °C for 2.0 h. Ethyl acetate (200 mL) was added to the reaction solution for dilution with stirring, and the reaction system was washed and extracted with 2% sodium bicarbonate solution (200 mL) and saturated brine solution (200 mL). The organic phase was separated, dried over anhydrous sodium sulfate, filtered, and concentrated to obtain the target product (8.75 g, yield: 70.3%).

¹H NMR (400 MHz, DMSO-*d₆*) δ 8.86 (t, *J =* 8.0 Hz, 1H), 7.90 (d, *J =* 8.0 Hz, 2H), 7.73-7.71 (*d, J =* 8.0, 2H), 7.60 (t, *J =* 8.0, 2H), 7.44-7.40 (m, 2H), 7.35-7.30(m, 2H), 4.70-4.68 (m, 2H), 4.31-4.20 (m, 2H), 4.25-4.22 (m, 1H), 4.13-4.06 (m, 2H), 3.67-3.65 (m, 2H).

### Example 4: Synthesis of Intermediate IV-2-1

### I. Synthesis of intermediate IV-2-1-A

### Route I:

### Synthesis of 2-amino-N-((2, 2,2-trifluoroethoxy)methyl)acetamide (IV-2-1-A)

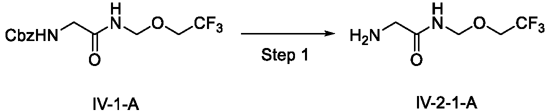

### Method I:

Compound IV-1-A (1.00 g, 3.29 mmol) was dissolved in THF (10 mL), then 10% Pd(OH)₂/C (100 mg) was added, and the mixture was purged with hydrogen and reacted at room temperature for 3 h. DMF (10 mL) was added to the reaction solution. The reaction system was filtered and concentrated to remove THF to obtain a solution of the target compound IV-2-1-A in DMF, which was directly used in the next step in 100% yield.

### Method II:

Compound IV-1-A (1.00 g, 3.29 mmol) was dissolved in THF (5 mL), 5% Pd/C (50 mg) was added, and the mixture was purged with hydrogen and reacted at room temperature for 3 h. DMF (5 mL) was added to the reaction solution. The reaction system was filtered and concentrated to remove THF to obtain a solution of the target compound IV-2-1-A in DMF, which was directly used in the next step in 100% yield.

### Method III:

Compound IV-1-A (15.0 g, 49.35 mmol) was dissolved in THF (75 mL), then 5% Pd/C (750 mg) was added, and the mixture was purged with hydrogen and reacted at room temperature for 3 h. DMF (75 mL) was added to the reaction solution. The reaction system was filtered and concentrated to remove THF to obtain a solution of the target compound IV-2-1-A in DMF, which was directly used in the next step in 100% yield.

The nuclear magnetic hydrogen spectrum of the HOBt salt of compound Ia-A-3 was as follows: ¹H NMR (400 MHz, DMSO-*d₆*) δ 9.18 (br, 1H), 7.79 - 7.71 (m, 1H), 7.49 - 7.41 (m, 1H), 7.25-7.18 (m, 2H), 4.74 (d, J = 4.9 Hz, 2H), 4.06 (q, J = 9.4 Hz, 2H), 3.47 (s, 2H).

### Route II:

### Preparation of 2-amino-N-((2,2,2-trifluoroethoxy)methyl)acetamide (IV-2-1-A)

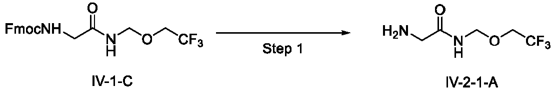

IV-1-C (57.0 g, 139.57 mmol) was dissolved in a solution of DMF (350 mL) and diethylamine (17.5 mL). The mixture was stirred at 25±5 °C for 1 h, and concentrated under reduced pressure at 35 °C for 30 min to obtain a solution of 2-amino-*N*-((2,2,2-trifluoroethoxy)methyl)acetamide (IV-2-1-A) in DMF, which was directly used for the next step in 100% conversion rate.

### II. Synthesis of intermediate IV-2-1-B

### Synthesis of 2-amino-N-(methoxymethyl)acetamide (IV-2-1-B)

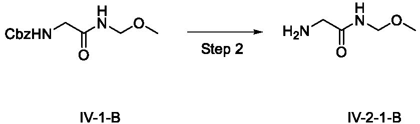

THF (40 mL), MeOH (40 mL) and Pd/C (550 mg, 10%, 55% water) were added to compound IV-1-B (11.0 g, 43.6 mmol), and the mixture was reacted at 30 °C for 7 h under hydrogen atmosphere. After the reaction was completed, DMF (55 mL) was added, the reaction system was concentrated under reduced pressure to remove THF and MeOH, and the residue was directly used in the next step without purification in 100% yield.
LCMS (ESI) [M+H]⁺ = 119.21

### III. Synthesis of intermediate IV-2-1-D

### Synthesis of 2-amino-N-((benzyloxy)methyl)acetamide (IV-2-1-D)

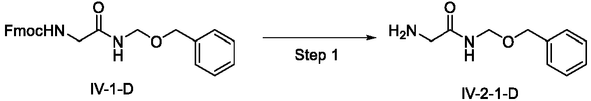

Compound IV-1-D (38.0 g, 0.091 mol) was dissolved in a solution of DMF (240 mL) and diethylamine (12 mL). The mixture was stirred at 25 °C for 1 h, and the reaction solution was concentrated under reduced pressure at 35 °C for 30 min to obtain a solution of IV-2-1-D in DMF, which was directly used in the next step in 100% conversion rate.

### IV. Synthesis of intermediate IV-2-1-E

### Synthesis of 2-amino-N-((isopropoxy)methyl)acetamide (IV-2-1-E)

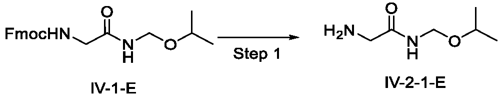

Compound IV-1-E (22.0 g, 59.83 mmol) was dissolved in a solution of DMF (150 mL) and diethylamine (7.5 mL). The mixture was stirred at 25 °C for 1 h. After the reaction was completed, the reaction solution was concentrated under reduced pressure at 35 °C for 30 min to obtain a solution of IV-2-1-E in DMF, which was directly used in the next step in 100% conversion rate.

### V. Synthesis of intermediate IV-2-1-F

### Synthesis of 2-amino-N-((2,2,3,3,3-pentafluoropropoxy)methyl)acetamide (IV-2-1-F)

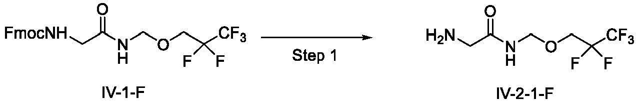

Compound IV-1-F (466.0 mg, 1.00 mmol) was dissolved in a solution of DMF (10 mL) and diethylamine (0.5 mL). The mixture was stirred at 25 °C for 1 h, and the reaction solution was concentrated under reduced pressure at 35 °C for 30 min to obtain a solution of 2-amino-*N*-((2,2,3,3,3-pentafluoropropoxy)methyl)acetamide (IV-2-1-F) in DMF, which was directly used in the next step in 100% conversion rate.

### Example 5: Synthesis of Intermediate IV-2

### I. Synthesis of IV-2-A

### Synthesis of benzyl ((10S,13S)-10-(4-(dipropylamino)butyl)-1,1,1-trifluoro-14-methyl-6,9,12-trioxo-3-oxa-5,8,11-triaza-pentadecan-13-yl)carbamate (IV-2-A)

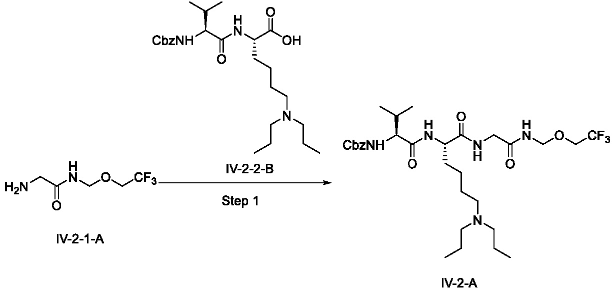

Method I: compound IV-2-2-B (1.52 g, 3.29 mmol) was added to the solution of compound IV-2-1-A (3.29 mmol) in DMF obtained in the above step, the temperature was controlled at -10 °C, and DMTMM (1.16 g, 3.98 mmol) was added and reacted for 1.5 h. EA (50 mL) was added to the reaction system, and the reaction system was washed successively with a saturated aqueous NaHCO₃ solution (15 mL × 3) and saturated brine (15 mL). The organic phase was dried over anhydrous sodium sulfate and filtered under vacuum, and the filtrate was concentrated to dryness. The resulting residue was dissolved in EA (3 mL) and added to n-heptane (30 mL) with stirring to precipitate a solid, which was filtered under vacuum, and the filter cake was the target compound (1.55 g, yield: 74.9%).

Method II: compound IV-2-2-A (33.4 g, 72.3 mmol) was added to the solution of compound IV-2-1-A (139.57 mmol) in DMF obtained in the above step, the temperature was controlled at -15 °C, and DMTMM (23.2 g, 79.6 mmol) was added and reacted for 1.5 h. EA (1000 mL) was added to the reaction system, and the reaction system was washed successively with a saturated aqueous NaHCO₃ solution (300 mL × 3) and saturated brine (300 mL). The organic phase was dried over anhydrous sodium sulfate and filtered under vacuum, and the filtrate was concentrated to dryness. The resulting residue was dissolved in MTBE (60 mL) and added to *n-*heptane (600 mL) with stirring to precipitate a solid, which was filtered under vacuum, and the filter cake was the target compound (31.2 g, yield: 75.4%).

LCMS (ESI) [M+H]⁺ = 632.3.

¹H NMR (400 MHz, DMSO-*d₆*) δ 8.85 - 8.73 (m, 1H), 8.37 - 8.26 (m, 1H), 8.03 (d, J = 7.0 Hz, 1H), 7.40-7.32 (m, 6H), 5.10 - 5.01 (m, 2H), 4.73-4.66 (m, 2H), 4.29 - 4.20 (m, 1H), 4.02 (q, J = 9.4 Hz, 2H), 3.93 (dd, J = 13.8, 6.6 Hz, 1H), 3.85 - 3.67 (m, 2H), 2.38-2.32 (m, 6H), 2.02-1.96 (m, 1H), 1.75 - 1.51 (m, 2H), 1.46 - 1.23 (m, 8H), 0.90-0.82 (m, 12H).

### II. Synthesis of IV-2-B

### Synthesis of benzyl ((9S,12S)-9-(4-(dipropylamino)butyl)-13-methyl-5,8,11-trioxo-2-oxa-4,7,10-triazatetradecan-12-yl)carbamate (IV-2-B)

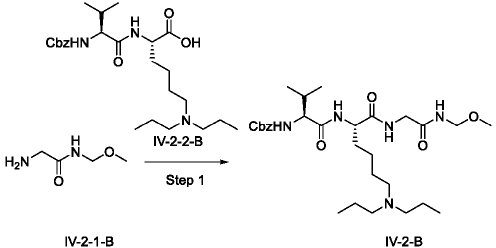

IV-2-2-B (23.6 g, 45.8 mmol, 90% wt) was added to the solution of IV-2-1-B (43.6 mmol) in DMF obtained in the above step, the reaction solution was cooled to -15 °C and the temperature was controlled at -15 °C to - 10 °C, and DMTMM monohydrate (12.9 g, 43.6 mmol) was added and reacted for 3 h. An aqueous NaHCO₃ solution (500 mL, 5% wt) was added to the reaction solution, and the reaction system was extracted with EA (200 mL × 4). The organic phases were combined, washed once with a saturated aqueous sodium chloride solution (200 mL), dried over anhydrous sodium sulfate, and concentrated under reduced pressure to dryness to obtain a crude product, which was purified by column chromatography (MeOH/DCM = 1% - 10%) to obtain IV-2-B (17.5 g, yield: 71.2%).
LCMS (ESI) [M+H]⁺ = 564.46
Formate: ¹H NMR (400 MHz, DMSO-*d₆*) δ 8.57 (t, *J =* 6.6 Hz, 1H), 8.33-8.21 (m, 2H), 8.05 (d, *J =* 7.3 Hz, 1H), 7.50-7.15 (m, 6H), 5.11-4.98 (m, 2H), 4.54-4.37 (m, 2H), 4.31-4.14 (m, 1H), 3.96-3.83 (m, 1H), 3.81-3.57 (m, 2H), 3.15 (s, 3H), 2.49 (s, 6H), 2.05-1.10 (m, 11H), 0.93-0.75 (m, 12H).

### III. Synthesis of IV-2-C

### Synthesis of (9H-fluoren-9-yl)methyl ((10S,13S)-10-(4-(dipropylamino)butyl)-1,1,1-trifluoro-14-methyl-6,9,12-trioxo-3-oxa-5,8,11-triazapentan-13-yl)carbamate (IV-2-C)

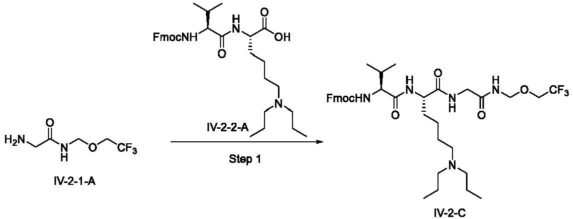

IV-2-2-A (70.0 g, 126.88 mmol) and DMF (350 mL) were added to a solution of IV-2-1-A (139.57 mmol) in DMF. The reaction solution was cooled to -15 °C, DMTMM (42.13 g, 152.26 mmol) was added, and the reaction system was stirred at -10 °C for 2 h. The reaction solution was poured into DCM (2000 mL), and the organic phase was washed with 2% aqueous NaHCO₃ solution (1000 mL × 3), water (1000 mL) and a saturated aqueous NaCl solution (500 mL). The organic phase was dried over anhydrous sodium sulfate and filtered to remove the insoluble substances, and the filtrate was concentrated to obtain a crude product, which was purified by silica gel column chromatography (eluent: 0-7% MeOH/DCM) to obtain (9H-fluoren-9-yl)methyl ((10S,13S)-10-(4-(dipropylamino)butyl)-1,1,1-trifluoro-14-methyl-6,9,12-trioxo-3-oxa-5,8,11-triazapentan-13-yl)carbamate (IV-2-C) (70 g, yield: 81%).
LCMS (ESI) [M+H]⁺ = 720.5
¹H NMR (400 MHz, DMSO-*d₆*) δ 8.78 (t, *J =* 8.0 Hz, 1H), 8.28 (t, *J =* 8.0 Hz, 1H), 8.02 (d, *J =* 4.0 Hz, 1H), 7.90-7.88 (m, 2H), 7.72 (t, *J =* 8.0 Hz, 2H), 7.46-7.39 (m, 3H), 7.34-7.30(m, 2H), 4.71-4.62 (m, 2H), 4.30-4.24 (m, 4H), 4.03-3.96 (m, 2H), 3.90-3.86 (m, 1H), 3.80-3.66 (m, 2H), 2.35-2.15 (m, 6H), 1.99-1.94 (m, 1H), 1.66-1.63 (m, 1H), 1.56-1.53 (m, 1H), 1.36-1.26 (m, 8H), 0.89-0.77 (m, 12H).

### IV. Synthesis of IV-2-D

### Synthesis of (9H-fluoren-9-yl)methyl ((9S,12S)-9-(4-(dipropylamino)butyl)-13-methyl-5,8,11-trioxo-1-phenyl-2-oxa-4,7,10-triazatetetradecan-12-yl)carbamate (IV-2-D)

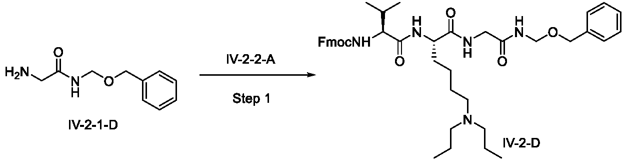

IV-2-2-A (49.3 g, 0.089 mmol) was added to a solution of IV-2-1-D (0.091 mol) in DMF, the reaction solution was cooled to -15 °C, and DMTMM (26.9 g, 0.091 mol) was added and stirred at -15 °C for 2 h. The reaction solution was poured into DCM (720 mL) and washed successively with 2% aqueous NaHCO₃ solution (500 mL × 2) and a saturated aqueous NaCl solution (500 mL). The organic phase was concentrated and the crude product was purified by silica gel column chromatography (eluent: 0-7% MeOH/DCM) to obtain (9*H*-fluoren-9-yl)methyl ((9*S*,12*S*)-9-(4-(dipropylamino)butyl)-13-methyl-5,8,11-trioxo-1-phenyl-2-oxa-4,7,10-triazatecan-12-yl)carbamate (IV-2-D) (55 g, yield: 85%).
LCMS (ESI) [M+H]⁺ = 728.6
¹H NMR (400 MHz, DMSO-*d₆*) δ 8.69 (t, *J =* 6.8 Hz, 1H), 8.27 (t, *J* = 5.8 Hz, 1H), 8.03 (d, *J* = 7.2 Hz, 1H), 7.93-7.87 (m, 2H), 7.75 (t, *J =* 7.0 Hz, 2H), 7.48-7.30 (m, 10H), 4.71-4.56 (m, 2H), 4.47 (s, 2H), 4.30-4.25 (m, 4H), 3.93-3.69 (m, 3H), 2.32-2.27 (m, 6H), 2.03-1.98 (m, 1H), 1.71-1.55 (m, 2H), 1.40-1.30 (m, 8H), 0.92-0.80 (m, 12H).

### V. Synthesis of IV-2-E

### Synthesis of (9H-fluoren-9-yl)methyl ((10S,13S)-10-(4-(dipropylamino)butyl)-2,14-dimethyl-6,9,12-trioxo-3-oxa-5,8,11-triazapentan-13-yl)carbamate (IV-2-E)

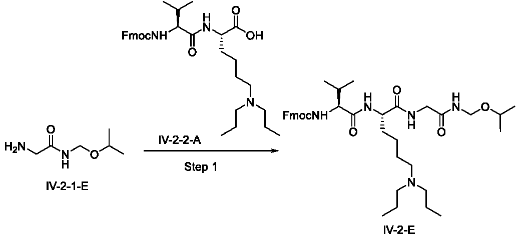

IV-2-2-A (30.0 g, 54.38 mol) was added to a solution of IV-2-1-E (59.83 mmol) in DMF. The reaction solution was cooled to -15 °C, and DMTMM (18.0 g, 65.26 mmol) was added and stirred at -10 °C for 2 h. The reaction solution was poured into DCM (1 L). The organic phase was washed successively with 2% aqueous NaHCO₃ solution (500 mL × 3), water (500 mL) and a saturated aqueous NaCl solution (500 mL). The organic phase was concentrated and the crude product was purified by silica gel column chromatography (eluent: 0-7% MeOH/DCM) to obtain the target product IV-2-E (30.0 g, yield: 81%).
LCMS (ESI) [M+H]⁺ = 680.5
¹H NMR (400 MHz, DMSO-*d₆*) δ 8.54 (t, *J =* 8.0 Hz, 1H), 8.27 (t, *J =* 8.0 Hz, 1H), 8.08 (d, *J =* 8.0 Hz, 1H), 7.90-7.88 (m, 2H), 7.75-7.73 (m, 2H), 7.46-7.40 (m, 3H), 7.34-7.30 (m, 2H), 4.56-4.52 (m, 2H), 4.34-4.19 (m, 4H), 3.91-3.87 (m, 1H), 3.71-3.63 (m, 3H), 3.00-2.75 (m, 6H), 2.02-1.97 (m, 1H), 1.75-1.54 (m, 8H), 1.37-1.24 (m, 2H), 1.06-1.04 (m, 6H), 0.93-0.77 (m, 12H).

### VI. Synthesis of IV-2-F

### Synthesis of (9H-fluoren-9-yl)methyl ((11S,14S)-11-(4-(dipropylamino)butyl)-1,1,1,2,2-pentafluoro-15-methyl-7,10,13-trioxo-4-oxa-6,9,12-triazahexadec-14-yl)carbamate (IV-2-F)

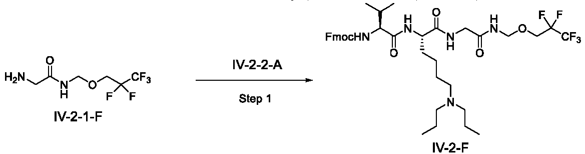

IV-2-2-A (500.0 mg, 0.91 mmol) was added to a solution of IV-2-1-F (1.00 mmol). The reaction solution was cooled to -15 °C, and DMTMM (300.0 mg, 1.09 mmol) was added and stirred at -10 C for 2 h. The reaction solution was poured into DCM (50 mL). The organic phase was washed successively with 2% aqueous NaHCO₃ solution (20 mL x 3), water (10 mL) and a saturated aqueous NaCl solution (10 mL). The organic phase was concentrated and the crude product was purified by silica gel column chromatography (eluent: 0-7% MeOH/DCM) to obtain the target product (IV-2-F) (440.0 mg, yield: 67%).
LCMS (ESI) [M+H]⁺ = 770.48
¹H NMR (400 MHz, DMSO-*d₆*) δ 8.79 (t, *J =* 8.0 Hz, 1H), 8.27 (t, *J =* 4.0 Hz, 1H), 8.00 (d, *J =* 8.0 Hz, 1H), 7.90-7.88 (m, 2H), 7.73-7.70 (m, 2H), 7.45-7.39 (m, 3H), 7.33-7.30 (m, 2H), 4.71-4.63 (m, 2H), 4.33-4.22 (m, 4H), 4.11-4.04 (m, 2H), 3.90-3.86 (m, 1H), 3.79-3.67 (m, 2H), 2.30-2.20 (m, 6H), 2.01-1.94 (m, 1H), 1.66-1.52 (m, 2H), 1.35-1.23 (m, 8H), 0.86-0.76 (m, 12H).

### VII. Synthesis of IV-2-G

### Synthesis of (9H-fluoren-9-yl)methyl ((S)-1-((S)-6-(dipropylamino)-1-((2-((hydroxymethyl)amino)-2-oxoethyl)amino)-1-oxohexan-2-yl)amino)-3-methyl-1-oxobutan-2-yl)carbamate (IV-2-G)

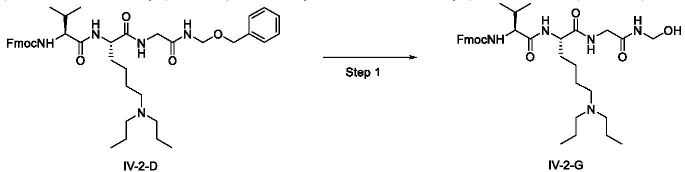

IV-2-D (73 mg, 0.10 mmol) was dissolved in tetrahydrofuran (1.4 mL), 10% palladium on carbon (28 mg) and HCl/EA (4 mol/L, 25 µL) were added sequentially, and the reaction system was purged with hydrogen for three times, and stirred at 25 °C for 12 h under hydrogen atmosphere. The reaction solution was filtered to remove the insoluble substances, and the filtrate was concentrated and purified by prep-HPLC (chromatographic conditions: acetonitrile/0.05% FA aqueous solution: 5%-40%) to obtain a formate of IV-3-G (345 mg, yield: 54%).
LCMS (ESI) [M+H]⁺ = 638.4
¹H NMR (400 MHz, DMSO-*d₆*) δ 9.09(s, 1H), 8.47-8.46 (m, 1H), 8.24-8.23 (m, 1H), 8.12-8.08 (m, 1H), 7.95-7.72 (m, 2H), 7.78-7.75 (m, 2H), 7.52-7.33 (m, 5H), 4.58-4.50 (m, 3H), 4.36-4.31 (m, 2H), 4.27-4.21 (m, 2H), 3.92-3.89 (m, 1H), 3.72-3.71 (m, 2H), 2.99-2.96 (m, 6H), 2.03-1.98 (m, 1H), 1.74-1.72 (m, 1H), 1.60-1.58 (m, 7H), 1.33 (s, 2H), 0.89-0.90 (m, 12H).

### Example 6: Synthesis of X-3-1

### I. Synthesis of X-3-1-A

### Route I: synthesis of (S)-2-((S)-2-amino-3-methylbutanamido)-6-(dipropylamino)-N-(2-oxo-2-(((2,2,2-trifluoroethoxy)methyl)amino)ethyl)hexanamide (X-3-1-A)

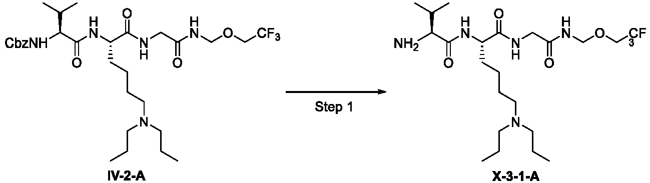

### Method I:

Compound IV-2-A (3.4 g, 5.4 mmol) was dissolved in 34 mL of MeOH, 680 mg of 10% Pd(OH)₂/C was added, and the reaction system was purged with hydrogen and reacted overnight. Then 680 mg of a catalyst 10% Pd(OH)₂/C was added and reacted for 24 h. The reaction solution was filtered, and the filtrate was concentrated under reduced pressure to obtain the target compound X-3-1-A (2.5 g, yield: 93.5%).

### Method II:

Compound IV-2-A (4 g, 6.3 mmol) was dissolved in 100 mL of methanol, and a program was set by using a fully automatic hydrogenation microreactor and using palladium hydroxide loaded by aluminum oxide as a catalyst: the temperature was 50 °C, the pressure was 2.5 Mpa, the flow rate of the starting material solution was 0.3 mL/min, and the flow rate of hydrogen was 30 mL/min. The effluent solution was concentrated under reduced pressure to obtain the target compound X-3-1-A (3.0 g, yield: 95.1%).

LCMS (ESI) [M+H]⁺ = 498.3.

¹H NMR (400 MHz, DMSO-*d₆*) δ 8.89 - 8.77 (m, 1H), 8.42 - 8.30 (m, 1H), 8.14 (s, 1H), 4.70 (p, J = 10.3 Hz, 2H), 4.28 (s, 1H), 4.02 (q, J = 9.4 Hz, 2H), 3.80 (dd, J = 15.7, 4.9 Hz, 1H), 3.75 - 3.67 (m, 1H), 3.20 (brs, 2H), 3.11 (d, J = 4.8 Hz, 1H), 2.35 (dd, J = 14.4, 7.3 Hz, 6H), 2.04 - 1.90 (m, 1H), 1.76 - 1.51 (m, 2H), 1.45 - 1.20 (m, 8H), 0.95 - 0.77 (m, 12H).

### Route II: synthesis of (S)-2-((S)-2-amino-3-methylbutanamido)-6-(dipropylamino)-N-(2-oxo-2-(((2,2,2-trifluoroethoxy)methyl)amino)ethyl)hexanamide (X-3-1-A)

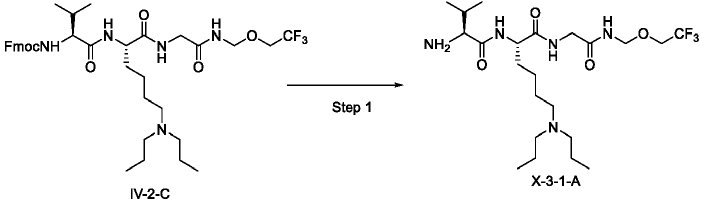

Compound IV-2-C (1.7 g, 2.4mmol) was dissolved in DMF (10 mL), DEA (0.5 mL) was added, and the reaction solution was stirred at 25 °C for 1 h. Tetrahydrofuran (15 mL) was added to the reaction solution and the reaction system was concentrated under reduced pressure. The above operation was repeated for three times to obtain a solution of the target compound X-3-1-A in DMF, which was directly used in the next step in 100% conversion rate.
LCMS (ESI) [M+H]⁺ = 498.3

### II. Synthesis of X-3-1-B

### Synthesis of (S)-2-((S)-2-amino-3-methylbutanamido)-6-(dipropylamino)-N-(2-((2-methoxymethyl)amino)-2-oxoethyl)hexanamide (X-3-1-B)

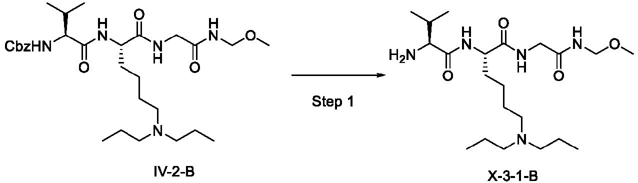

MeOH (70 mL) and Pd/C (2.1 g, 10%, 55% water) were added to compound IV-2-B (7.0 g, 12.4 mmol), and the mixture was reacted at 30 °C for 48 h. The reaction was monitored by LCMS. After the reaction was completed, the reaction system was concentrated to remove MeOH, 63 mL of ACN was added, and the reaction system was directly used in the next step without purification (yield: 100%).
LCMS (ESI) [M+H]⁺ = 430.51
¹H NMR (400 MHz, DMSO-*d₆*) δ 8.59 (t, *J =* 6.6 Hz, 1H), 8.33 (t, *J* = 5.9 Hz, 1H), 8.21 (d, *J* = 7.1 Hz, 1H), 4.52-4.36 (m, 2H), 4.32-4.13 (m, 1H), 3.80-3.57 (m, 2H), 3.18-3.03 (m, 4H), 2.44-2.23 (m, 6H), 2.06-1.91 (m, 1H), 1.76-1.47 (m, 2H), 1.45-1.13 (m, 8H), 0.96-0.71 (m, 12H).

### Example 7: Synthesis of X-3

### I. Synthesis of X-3a

### Synthesis of N-((10S,13S)-10-(4-(dipropylamino)butyl)-1,1,1-trifluoro-14-methyl-6,9,12-trioxo-3-oxa-5,8,11-triaza-pentadecan-13-yl)-6-(2-(methylsulfonyl)pyrimidin-5-yl)hex-5-ynamide (X-3a)

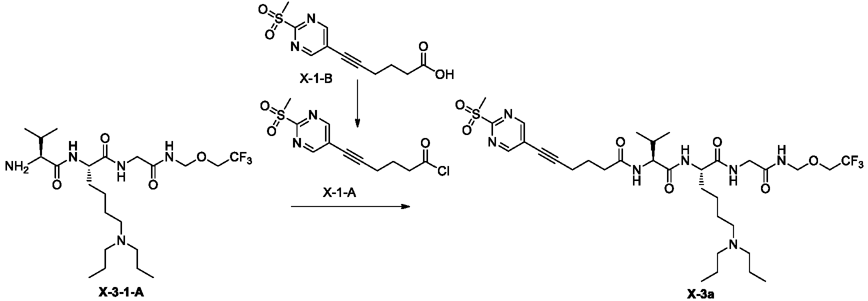

### Method I:

X-1-B (2.95 g, 11.0 mmol), DCM (20 mL) and thionyl chloride (3.93 g, 33.0 mmol) were added sequentially to a reaction flask under argon atmosphere, and the mixture was stirred at room temperature for 3 h, and concentrated by rotary evaporation under reduced pressure to remove the solvent to obtain X-1-A, which was directly used in the next step (conversion rate: 100%).

Compound X-3-1-A (5.0 g, 10.0 mmol) was dissolved in 35 mL of acetonitrile, the mixture was cooled to 10 °C, and DIPEA (0.26 g, 2.0 mmol) was added, followed by dropwise addition of a solution of compound X-1-A (11.0 mmol) in acetonitrile (15 mL). The reaction was continued for 1 h, then methyl *tert-butyl* ether (500 mL) was added to the reaction solution to precipitate a solid, which was filtered under vacuum, and the filter cake was dried to obtain a hydrochloride of the target compound (7.13 g, yield: 91.0%).

### Method II:

X-1-B (32.2 g, 120 mmol), DCM (220 mL) and thionyl chloride (42.9 g, 360 mmol) were added sequentially to a reaction flask under argon atmosphere, and the mixture was stirred at room temperature for 3 h, and concentrated by rotary evaporation under reduced pressure to remove the solvent to obtain X-1-A, which was directly used in the next step (conversion rate: 100%).

Compound X-3-1-A (50.0 g, 100 mmol) was dissolved in 350 mL of acetonitrile, the mixture was cooled to 10 °C, and DIPEA (3.25 g, 25 mmol) was added, followed by dropwise addition of a solution of compound X-1-A (prepared from acid and thionyl chloride, prepared for immediate use) (120 mmol) in acetonitrile (150 mL). The reaction was continued for 1 h, then 2-methyltetrahydrofuran (2.1 L) was added to the reaction solution to precipitate a solid, which was filtered under vacuum, and the filter cake was dried to obtain a hydrochloride of the target compound (71.8 g, yield: 91.6%).
LCMS (ESI) [M+H]⁺ = 748.4
¹H NMR (400 MHz, DMSO-*d₆*) δ 9.15 (s, 2H), 8.78 (t, J = 6.8 Hz, 1H), 8.24 (t, J = 5.8 Hz, 1H), 8.05 (d, J = 7.3 Hz, 1H), 7.95 (d, J = 8.6 Hz, 1H), 4.75-4.66 (m, 2H), 4.24-4.19 (m, 2H), 4.03 (q, J = 9.4 Hz, 2H), 3.78-3.76 (m, 2H), 3.44 (s, 3H), 2.58 (t, J = 7.1 Hz, 2H), 2.46-2.29 (m, 8H), 2.03-1.95 (m, 1H), 1.89-1.81 (m, 2H), 1.73-1.66 (m, 1H), 1.62-1.56 (m, 1H), 1.41-1.36 (m, 6H), 1.31-1.25 (m, 2H), 0.89-0.83 (m, 12H).

### II. Synthesis of X-3b

### Synthesis of N-((9S,12S)-9-(4-(dipropylamino)butyl)-13-methyl-5,8,11-trioxo-2-oxa-4,7,10-triazatetradecan-12-yl)-6-(2-(methylsulfonyl)pyrimidin-5-yl)hex-5-ynamide (X-3b)

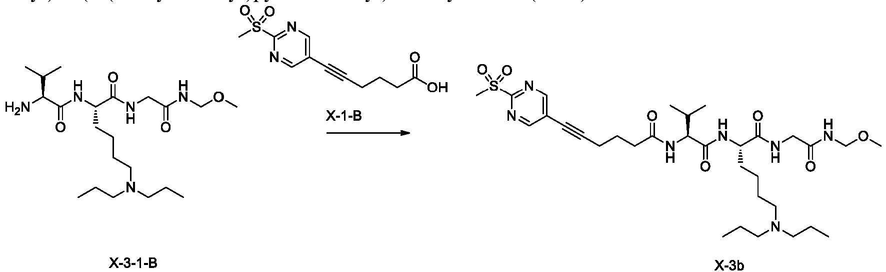

DIPEA (321 mg, 2.48 mmol) was added to a solution of compound X-3-1-B (12.4 mmol) in ACN for later use. Compound X-1-B (3.66 g, 13.6 mmol) was added to dichloromethane (40 mL), and thionyl chloride (4.85 g, 40.8 mmol) was added. The mixture was stirred at 30 °C for 3.0 h, and concentrated under reduced pressure to dryness in a water bath at 40 °C, then ACN (7 mL) was added for dissolution, and the temperature was controlled at 10 °C to 15 °C. The resulting solution was added dropwise to the prepared solution of compound X-3-1-B in ACN. After 2 h of reaction, MTBE (490 mL) was added, and the reaction system was filtered and dried to obtain the compound (5.40 g, 7.5 mmol, yield: 60.5%).
LCMS (ESI) [M+H]⁺ = 680.42
¹H NMR (400 MHz, DMSO-*d₆*) δ 9.12 (s, 2H), 8.51 (t, *J =* 6.6 Hz, 1H), 8.27 (s, 1H), 8.19 (t, *J =* 5.9 Hz, 1H), 8.04 (d, *J =* 7.3 Hz, 1H), 7.95 (d, *J =* 8.6 Hz, 1H), 4.57-4.35 (m, 2H), 4.25-4.04 (m, 2H), 3.82-3.62 (m, 2H), 3.41 (s, 3H), 3.14 (s, 3H), 2.54 (t, *J* = 7.1 Hz, 2H), 2.43-2.18 (m, 8H), 2.02-1.88 (m, 1H), 1.87-1.73 (m, 2H), 1.72-1.45 (m, 2H), 1.43-1.16 (m, 8H), 0.92-0.73 (m, 12H).

### III. Synthesis of X-3a

### Synthesis of N-((10S,13S)-10-(4-(dipropylamino)butyl)-1,1,1-trifluoro-14-methyl-6,9,12-trioxo-3-oxa-5,8,11-triaza-pentadecan-13-yl)-6-(2-(methylsulfonyl)pyrimidin-5-yl)hex-5-ynamide (X-3a)

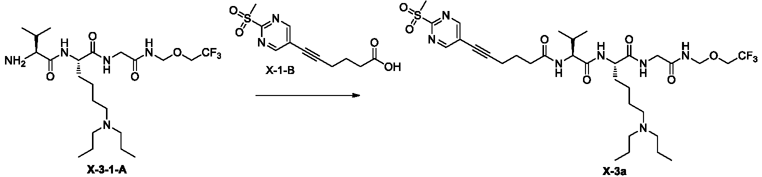

X-1-B (772 mg, 2.9 mmol) and DMTMM (849 mg, 2.9 mmol) were added sequentially to the solution of X-3-1-A in DMF obtained in step 1 and stirred at 25 °C for 1 h. The reaction solution was purified by prep-HPLC and lyophilized to obtain *N*-((10*S*,13*S*)-10-(4-(dipropylamino)butyl)-1,1,1-trifluoro-14-methyl-6,9,12-trioxo-3-oxa-5,8,11-triazapentadecan-13-yl)-6-(2-(methylsulfonyl)pyrimidin-5-yl)hex-5-ynamide (X-3a) (1.3 g, yield: 76.5%).
LCMS (ESI) [M+H]⁺ = 748.4
¹H NMR (400 MHz, DMSO-*d₆*) δ 9.15 (s, 2H), 8.78 (t, *J =* 6.8 Hz, 1H), 8.24 (t, *J =* 5.8 Hz, 1H), 8.05 (d, *J =* 7.3 Hz, 1H), 7.95 (d, *J =* 8.6 Hz, 1H), 4.75-4.66 (m, 2H), 4.24-4.19 (m, 2H), 4.03 (q, *J =* 9.4 Hz, 2H), 3.78-3.76 (m, 2H), 3.44 (s, 3H), 2.58 (t, *J =* 7.1 Hz, 2H), 2.46-2.29 (m, 8H), 2.03-1.95 (m, 1H), 1.89-1.81 (m, 2H), 1.73-1.66 (m, 1H), 1.62-1.56 (m, 1H), 1.41-1.36 (m, 6H), 1.31-1.25 (m, 2H), 0.89-0.83 (m, 12H).

### Example 8: Synthesis of IIa

### Route I:

### Synthesis of N-((11S,14S)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (IIa)

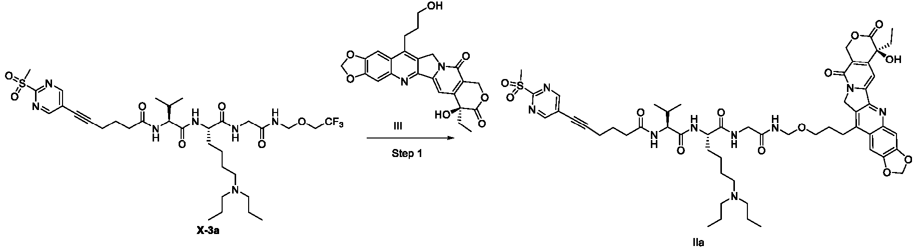

Method 1: compound III (2.0 g, 4.4 mmol) was dissolved in dry DMF (10 mL) at room temperature, a solution of HCl in DMF (3.3 mL, 4.02 mol/L) was added, and the mixture was stirred until dissolution. The hydrochloride of X-3a (6.97 g, 8.8 mmol) obtained in step 8 was added, and the mixture was stirred at room temperature for 3 h under nitrogen atmosphere. DCM (72 mL) was added to the reaction solution, and an acetonitrile/water mixed solution (72 mL, v:v = 30:70) was added, followed by liquid separation, and the aqueous phase and the organic phase were collected separately. The organic phase was washed with 2% aqueous sodium bicarbonate solution (72 mL); the aqueous phases were combined twice and extracted with DCM (72 mL × 2). The organic phases were combined for three times, washed successively with pure water (72 mL) and saturated brine (72 mL), dried over anhydrous sodium sulfate, and filtered. The filtrate was left to stand at -20 °C for 12 h and filtered to remove the insoluble substances, and the filtrate was concentrated to obtain a crude product. The crude product was separated and purified by preparative HPLC to obtain a formate of the target product IIa (2.5 g, yield: 51.2%).
LCMS (ESI) [M+H]⁺ = 1098.7
¹H NMR (400 MHz, DMSO-*d₆*) δ 9.10 (s, 2H), 8.59 (t, J = 6.6 Hz, 1H), 8.25 - 8.14 (m, 2H), 8.02 (d, J = 7.4 Hz, 1H), 7.93 (d, J = 8.5 Hz, 1H), 7.58 (s, 1H), 7.50 (s, 1H), 7.24 (s, 1H), 6.28 (s, 2H), 5.42 (d, J = 2.2 Hz, 2H), 5.23 (s, 2H), 4.66 - 4.52 (m, 2H), 4.26 - 4.11 (m, 2H), 3.80 - 3.64 (m, 2H), 3.49 (t, J = 6.0 Hz, 2H), 3.40 (s, 3H), 3.15-3.06 (m, 2H), 2.54 (d, J = 7.3 Hz, 2H), 2.44 - 2.25 (m, 8H), 1.99 - 1.77 (m, 7H), 1.73 - 1.45 (m, 2H), 1.39 - 1.20 (m, 8H), 0.89 - 0.77 (m, 15H).

Method 2: 200 mL of toluene was added to the solid of compound III (10.0 g, 22.2 mmol) and the hydrochloride of X-3a (35.0 g, 44.4mmol), and the mixture was stirred at reflux by using a water separator at 110 °C to remove water for 2.0 h; the reaction system was subjected to rotary evaporation to remove toluene; 60 mL of DMF was added, then boron trifluoride etherate (9.5 g, 66.6 mmol) was added, and the mixture was stirred at 30 °C for 16 h. 360 mL of DCM was added to the reaction solution, and stirred and diluted. The reaction system was washed with (2% sodium bicarbonate:saturated brine = 7:3) (300 mL × 2) and (water:saturated brine = 7:3) (300 mL). The organic phase was dried, filtered and concentrated to dryness to obtain a crude product, and the crude product was separated and purified by prep-HPLC to obtain a formate of the target product IIa (16.7 g, yield: 68.3%).
LCMS (ESI) [M+H]⁺ = 1098.7
¹H NMR (400 MHz, DMSO-*d₆*) δ 9.09 (s, 2H), 8.57 (t, J = 6.6 Hz, 1H), 8.22-8.12 (m, 2H), 7.99 (d, J = 7.4 Hz, 1H), 7.91 (d, J = 8.6 Hz, 1H), 7.57 (s, 1H), 7.49 (s, 1H), 7.24 (s, 1H), 6.45 (s, 1H), 6.28 (s, 2H), 5.42 (d, J = 3.1 Hz, 2H), 5.23 (s, 2H), 4.58 (q, J = 10.2, 8.3 Hz, 2H), 4.26-4.11 (m, 2H), 3.81-3.64 (m, 2H), 3.50 (t, J = 6.1 Hz, 2H), 3.40 (s, 3H), 3.10 (t, J = 8.1 Hz, 2H), 2.54 (d, J = 7.1 Hz, 2H), 2.42-2.25 (m, 8H), 2.00-1.74 (m, 7H), 1.70-1.48 (m, 2H), 1.40-1.22 (m, 8H), 0.91 -0.74 (m, 15H).

Method 3: 200 mL of toluene was added to the solid of compound III (10.0 g, 22.2 mmol) and the hydrochloride of X-3a (35.0 g, 44.4 mmol), and the mixture was stirred at reflux by using a water separator at 110 °C to remove water for 2.0 h; the reaction system was subjected to rotary evaporation to remove toluene; 60 mL of NMP was added, and the mixture was stirred at 120 °C for 5 h. The reaction solution was cooled to room temperature and added dropwise to 360 mL of EA, and the reaction system was stirred for 1 h and filtered under vacuum. The filter cake was purified by column chromatography (MeOH/DCM = 1%-20%) to obtain a hydrochloride of the target product I (16.4 g, yield: 67.2%).
LCMS (ESI) [M+H]⁺ = 1098.7
¹H NMR (400 MHz, DMSO-*d₆*) δ 9.81 (s, 1H), 9.11 (s, 2H), 8.65 (t, J = 6.6 Hz, 1H), 8.23 (t, J = 5.8 Hz, 1H), 8.10 (d, J = 7.4 Hz, 1H), 7.97 (d, J = 8.5 Hz, 1H), 7.58 (s, 1H), 7.50 (s, 1H), 7.24 (s, 1H), 6.49 (s, 1H), 6.28 (d, J = 1.8 Hz, 2H), 5.42 (s, 2H), 5.22 (s, 2H), 4.65 - 4.52 (m, 2H), 4.25 (q, J = 7.3 Hz, 1H), 4.15 (dd, J = 8.5, 6.8 Hz, 1H), 3.74 (d, J = 5.9 Hz, 2H), 3.49 (t, J = 6.1 Hz, 2H), 3.40 (s, 3H), 3.13 - 3.03 (m, 2H), 2.95 (dt, J = 11.5, 5.0 Hz, 6H), 2.54 (t, J = 7.0 Hz, 2H), 2.36 (dq, J = 24.7, 7.4 Hz, 2H), 2.04 - 1.54 (m, 15H), 1.37 - 1.25 (m, 2H), 0.94 - 0.72 (m, 15H).

### Route II:

### Synthesis of N-((11S,14S)-11-(4-(dipropylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecan-14-yl)-6-(2-(methylsulfonyl)pyrimidin-5-yl)hex-5-ynamide (IIa)

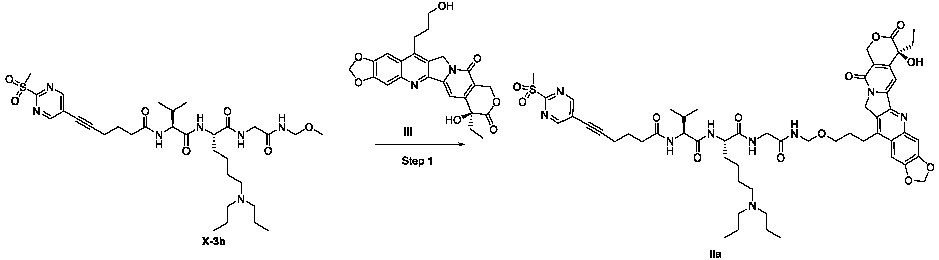

DMF (1.35 mL), compound III (300 mg, 90% purity, 0.6 mmol) and HCl-DMF (4 mol/L, 0.45 mL) were added to X-3b (816 mg, 1.2 mmol), and the mixture was reacted at 30 °C for 3 h. DCM (12 mL) was added to the reaction solution, and an acetonitrile/water mixed solution (12 mL, v:v = 30:70) was added, followed by liquid separation, and the aqueous phase and the organic phase were collected separately. The organic phase was washed with 2% aqueous sodium bicarbonate solution (12 mL); the aqueous phases were combined twice and extracted with DCM (12 mL × 2). The organic phases were combined for three times, washed successively with pure water (12 mL) and saturated brine (12 mL), dried over anhydrous sodium sulfate, and filtered. The filtrate was concentrated to obtain the crude product. The crude product was separated and purified by preparative HPLC to obtain a formate of the target product IIa (347 mg, yield: 50.6%).
LCMS (ESI) [M+H]⁺ = 1098.7
¹H NMR (400 MHz, DMSO-*d₆*) δ 9.09 (s, 2H), 8.57 (t, *J =* 6.6 Hz, 1H), 8.22-8.12 (m, 2H), 7.99 (d, *J =* 7.4 Hz, 1H), 7.91 (d, *J =* 8.6 Hz, 1H), 7.57 (s, 1H), 7.49 (s, 1H), 7.24 (s, 1H), 6.45 (s, 1H), 6.28 (s, 2H), 5.42 (d, *J* = 3.1 Hz, 2H), 5.23 (s, 2H), 4.58 (q, *J =* 10.2, 8.3 Hz, 2H), 4.26-4.11 (m, 2H), 3.81-3.64 (m, 2H), 3.50 (t, *J =* 6.1 Hz, 2H), 3.40 (s, 3H), 3.10 (t, *J =* 8.1 Hz, 2H), 2.54 (d, *J =* 7.1 Hz, 2H), 2.42-2.25 (m, 8H), 2.00-1.74 (m, 7H), 1.70-1.48 (m, 2H), 1.40-1.22 (m, 8H), 0.91-0.74 (m, 15H).

### Synthesis of Other Linkers and Drug Linker Conjugates

### Example 1: Synthesis of (S)-1-ethyl-4-(3-methyl-2-(6-(2-(methylsulfonyl)pyrimidin-5-yl)hex-5-ynamido)butanamido)-N-(2-oxo-2-(((2,2,2-trifluoroethoxy)methyl)amino)ethyl)piperidine-4-carboxamide (L1)

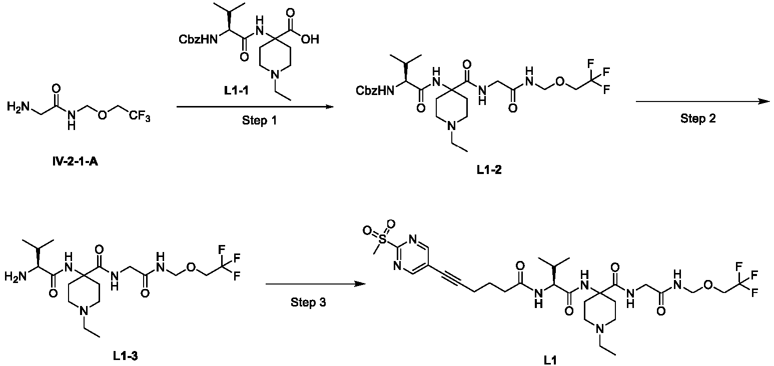

### Step 1:

To a solution of IV-2-1-A (495.85 mg, 2.66 mmol) in DMF (3 mL) was added L1-1 (900 mg, 2.22 mmol). The reaction solution was cooled to -15 °C, DMTMM (785.19 mg, 2.66 mmol) was added, and the mixture was stirred at -10 °C for 2 h. The reaction solution was poured into DCM (200 mL), and the organic phase was washed successively with 2% aqueous NaHCO₃ solution (100 mL × 3), water (100 mL) and a saturated aqueous NaCl solution (50 mL). The organic phase was dried over anhydrous Na₂SO₄ and filtered, the filtrate was concentrated to obtain a crude product, and the crude product was purified by silica gel column chromatography (eluent: 0-7% MeOH/DCM) to obtain the target compound L1-2 (900 mg).

LCMS (ESI) [M+H]⁺ = 574.6.

### Step 2:

Compound L1-2 (900 mg, 1.57 mmol) was dissolved in MeOH (10 mL), Pd(OH)₂/C (270 mg) was added, and the mixture was stirred at 25 °C overnight under hydrogen atmosphere, and filtered, and the filtrate was concentrated to obtain the target compound L1-3 (620 mg).

LCMS (ESI) [M+H]⁺ = 440.2.

### Step 3:

L1-3 (200 mg, 0.45 mmol) was dissolved in DMF (2 mL) at 25 °C, then 6-(2-(methylsulfonyl)pyrimidin-5-yl)hex-5-ynoic acid (121 mg, 0.45 mmol) and DMTMM (133 mg, 0.45 mmol) were added sequentially, and the mixture was stirred for 1 h. The reaction solution was purified by preparative HPLC to obtain the target compound L1 (180 mg).

LCMS (ESI) [M+H]⁺ = 690.4.

¹H NMR (400 MHz, DMSO-*d₆*) δ 9.11 (s, 2H), 8.43 (s, 1H), 8.37 (t, *J* = 6.8 Hz, 1H), 8.18-8.16 (m, 2H), 8.01 (t, *J* = 6.0 Hz, 1H), 4.71 (dd, *J* = 10.3, 7.1 Hz, 1H), 4.60 (dd, *J* = 10.3, 6.4 Hz, 1H), 4.07-4.11 (m, 1H), 4.03-3.95 (m, 2H), 3.76-3.57 (m, 1H), 3.41 (s, 3H), 2.77-2.67 (m, 2H), 2.58-2.50 (m, 2H), 2.41-2.26 (m, 5H), 2.19-2.03 (m, 3H), 1.99-1.89 (m, 1H), 1.90-1.70 (m, 4H), 0.99 (t, *J =* 7.1 Hz, 3H), 0.96-0.90 (m, 6H).

### Example 2: Synthesis of (S)-2-((S)-2-(6-(2,5-dioxo-2,5-dihydro-1H-pyrrol-1-yl)hexanamido)-3-methylbutanamido)-6-(dipropylamino)-N-(2-oxo-2-(((2,2,2-trifluoroethoxy)methyl)amino)ethyl)hexanamide (L2)

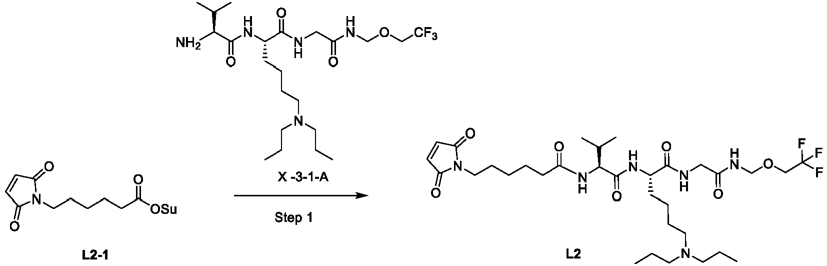

L2-1 (300 mg, 0.97 mmol) and X-3-1-A (531 mg, 1.07 mmol) were added to a reaction flask, then DMF (5 mL) was added for dissolution, and DIPEA (151 mg, 1.16 mmol) was added to the mixture at room temperature. The reaction was continued for 3 h at room temperature. The reaction solution was extracted twice with water (50 mL) and DCM (20 mL), and the organic phases were combined. The organic phase was dried over anhydrous Na₂SO₄ and filtered, and the filtrate was concentrated to bring the volume to 5 mL, and purified by silica gel column chromatography (MeOH:DCM = 0-15%) to obtain the target compound L2 (300 mg).
LCMS (ESI) [M+H]⁺ = 691.6;
¹H NMR (400 MHz, DMSO-*d₆*) δ 8.82 (t, *J =* 6.8 Hz, 1H), 8.25 (t, *J =* 5.8 Hz, 1H), 8.06 (t, *J =* 9.9 Hz, 1H), 7.80 (t, *J = 10.7* Hz, 1H), 7.03 (s, 2H), 4.75-4.65 (m, 2H), 4.31-4.22 (m, 1H), 4.13 (dd, *J =* 15.4, 7.3 Hz, 1H), 4.02 (q, *J =* 9.4 Hz, 2H), 3.82-3.71 (m, 2H), 3.40 (t, *J =* 7.1 Hz, 2H), 3.06-2.98 (m, 6H), 2.26-2.06 (m, 2H), 2.03-1.90 (m, 1H), 1.68-1.60 (m, 8H), 1.53-1.48 (m, 3H), 1.37-1.26 (m, 3H), 1.24-1.18 (m, 2H), 0.93 (t, *J* = 7.3 Hz, 6H), 0.85 (t, *J=* 7.0 Hz, 6H).

### Example 3: Synthesis of (S)-4-(2-(6-(2,5-dioxo-2,5-dihydro-1H-pyrrol-1-yl)hexanamido)-3-methylbutanamido)-1-ethyl-N-(2-oxo-2-(((2,2,2-trifluoroethoxy)methyl)amino)ethyl)piperidine-4-carboxamide (L3)

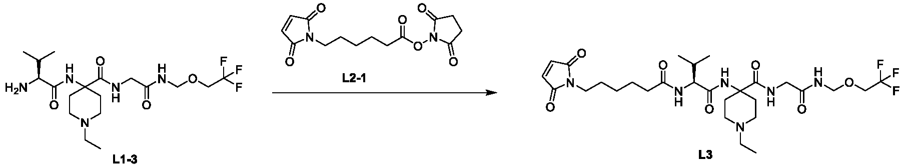

L1-3 (200 mg, 0.46 mmol) was dissolved in DMF (2 mL) at 25 °C, then L2-1 (170 mg, 0.55 mmol) and DIPEA (71 mg, 0.55 mmol) were added sequentially, and after the addition was completed, the mixture was stirred at room temperature for 1 h. The reaction was monitored by LC-MS. The reaction solution was purified by preparative high-performance liquid chromatography (acetonitrile:H₂O containing 0.05% FA = 5%-50%) to obtain the target compound INT4 (200 mg).

LCMS (ESI) [M+H]⁺ = 633.45.

¹H NMR (400 MHz, DMSO-*d₆*) δ 8.42 (s, 1H), 8.35 (t, *J =* 6.8 Hz, 1H), 8.22 (s, 1H), 8.13-8.02 (m, 2H), 7.03 (s, 2H), 4.73 (dd, *J* = 10.3, 7.1 Hz, 1H), 4.62 (dd, *J* = 10.3, 6.4 Hz, 1H), 4.10-3.98 (m, 3H), 3.76-3.70 (m, 2H), 3.63-3.58 (m, 1H), 3.41-3.37 (m, 2H), 2.74- 2.71 (m, 2H), 2.36 (q, *J =* 7.2 Hz, 2H), 2.30-2.04 (m, 6H), 1.98-1.93 (m, 1H), 1.86-1.84 (m, 1H), 1.53-1.43 (m, 4H), 1.25-1.14 (m, 2H), 1.02 (t, *J =* 7.1 Hz, 3H), 0.95-0.92 (m, 6H).

### Example 4: Synthesis of (S)-6-(dimethylamino)-2-((S)-3-methyl-2-(6-(4-(2-(methylsulfonyl)pyrimidin-5-yl)-1H-1,2,3-triazol-1-yl)hexanamido)butanamido)-N-(2-oxo-2-(((2,2,2-trifluoroethoxy)methyl)amino)ethyl)hexanamide (L4)

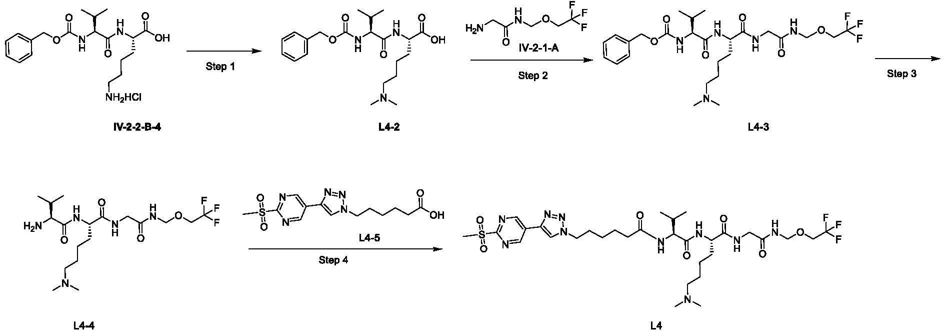

### Step 1:

Compound IV-2-2-B-4 (20 g, 48.09 mmol) was dissolved in tetrahydrofuran (200 mL), the mixture was cooled to 5 °C with stirring, and sodium cyanoborohydride (9.07 g, 144.27 mmol) was added to the solution. The mixture was heated to 10 °C, then an aqueous formaldehyde solution (15.61 g, 192.36 mmol) was added, and the reaction solution was heated to room temperature and stirred for 2 h. The reaction was monitored by HPLC. Concentrated hydrochloric acid (12 mL) and water (20 mL) were added to the reaction solution to quench the reaction, and the reaction solution was stirred at 30 °C overnight. The reaction solution was concentrated under reduced pressure until no droplets dropped, then water (200 mL), dichloromethane (200 mL) and anhydrous sodium sulfate (60 g) were added, and the mixture was stirred and dissolved at 40 °C, and then left to stand, followed by liquid separation. The aqueous phase was extracted with dichloromethane (200 mL), and the pH value of the aqueous phase was adjusted to 7. Dichloromethane (200 mL), a saturated aqueous sodium chloride solution (100 mL) and anhydrous sodium sulfate (90 g) were sequentially added and dissolved completely, and the reaction system was left to stand, followed by liquid separation. Dichloromethane (200 mL) was added to the aqueous phase for extraction, and the organic phases were combined, dried over anhydrous sodium sulfate, filtered, and concentrated under reduced pressure to obtain the target compound L4-2 (16 g). LCMS (ESI) [M+H]⁺ = 408.54.

### Step 2:

Compound L4-2 (16 g, 28.67 mmol) was dissolved in a reaction system of compound IV-2-1-A (4.85 g, 26.06 mmol), the reaction system was cooled to -12 °C, then DMTMM (7.21 g, 26.06 mmol) was added, and the temperature was kept at -12 °C for reaction for 2 h. The reaction was monitored by HPLC. EA (425 mL) was added to the reaction solution, filtration was carried out, and the filtrate was washed with 2% sodium bicarbonate aqueous solution (200 mL × 2). The organic phase was concentrated under reduced pressure until no droplets dropped, acetonitrile (30 mL) was added to the crude product for dissolution, and the crude product was added dropwise to an aqueous citric acid (12.5 g, 65.15 mmol) solution (300 mL) at 5-10 °C. The mixture was stirred for 30 min and filtered, the pH value of the filtrate was adjusted to 10 by adding sodium carbonate, and then DCM (300 mL) was added for extraction. The organic phase was concentrated under reduced pressure until no droplets dropped, methyl *tert-butyl* ether (45 mL) was added for dissolution, and the reaction solution was dropped into n-heptane (900 mL) to precipitate a large amount of solid, which was filtered. The filter cake was dried to obtain the target compound L4-3 (4.3 g).

LCMS (ESI) [M+H]⁺ = 576.38.

### Step 3:

Compound L4-3 (800 mg, 1.04 mmol) was dissolved in methanol (8 mL), palladium hydroxide (0.15 g, 1.04 mmol) was added under nitrogen atmosphere, and the mixture was purged with hydrogen for three times and stirred at room temperature overnight under hydrogen atmosphere. The reaction was monitored by LCMS. The reaction solution was directly filtered. The filtrate was concentrated under reduced pressure to obtain the target compound L4-4 (460 mg).

LCMS (ESI) [M+H]⁺ = 442.5.

### Step 4:

Compound L4-4 (460 mg, 1.05 mmol) was dissolved in DMF (5 mL), then compound L4-5 (0.36 g, 1.05 mmol) was added, and the mixture was cooled to 5 °C with stirring. DMTMM (0.29 g, 1.05 mmol) was added in one portion, and the mixture was reacted at -12 °C for 2 h. The reaction was monitored by LCMS. The reaction solution was directly purified by preparative high-performance liquid chromatography (acetonitrile:H₂O containing 0.05% FA = 5%-50%) to obtain the target compound L4 (400 mg).

LCMS (ESI) [M+H]⁺ = 763.53.

¹H NMR (400 MHz, DMSO-*d₆*) δ 9.48 (s, 2H), 8.94 (s, 1H), 8.76 (t, *J =* 6.8 Hz, 1H), 8.29-8.15 (m, 2H), 8.00 (d, *J =* 7.3 Hz, 1H), 7.82 (d, *J =* 8.7 Hz, 1H), 4.74-4.57 (m, 2H), 4.47 (t, *J =* 7.0 Hz, 2H), 4.24-4.09 (m, 2H), 3.99 (q, *J =* 9.4 Hz, 2H), 3.82-3.63 (m, 2H), 3.44 (s, 3H), 2.27-2.08 (m, 10H), 1.92 (m, 2H), 1.73-1.47 (m, 4H), 1.43-1.19 (m, 6H), 0.80 (t, *J=* 7.3 Hz, 6H).

### Example 5: Synthesis of (S)-2-((S)-2-(2,2-dimethyl-4-(4-(2-(methylsulfonyl)pyrimidin-5-yl)-1H-1,2,3-triazol-1-yl)butanamido)-3-methylbutanamido)-6-(dimethylamino)-N-(2-oxo-2-(((2,2,2-trifluoroethoxy)methyl)amino)ethyl)hexanamide (L5)

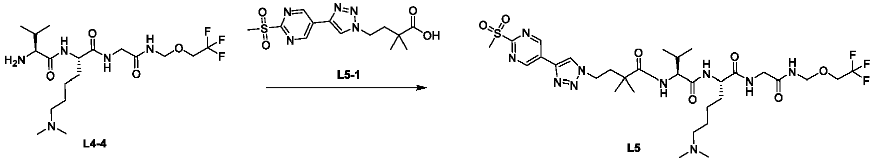

Compound L4-4 (300 mg, 0.68 mmol) was dissolved in DMF (5 mL), then compound L5-1 (0.23 g, 0.68 mmol) was added, and the mixture was cooled to -12 °C. DMTMM (0.19 g, 0.68 mmol) was added, and the temperature was maintained at -12 °C and the reaction was continued for 1 h. The reaction was monitored by LCMS. The reaction solution was directly purified by preparative high-performance liquid chromatography (acetonitrile:H₂O containing 0.05% FA = 5%-50%) to obtain the target compound L5 (170 mg).

LCMS (ESI) [M+H]⁺ = 763.55.

¹H NMR (400 MHz, DMSO-*d₆*) δ 9.51 (s, 2H), 8.97 (s, 1H), 8.81 (t, *J* = 6.9 Hz, 1H), 8.32 (t, *J* = 5.9 Hz, 1H), 8.24 (s, 1H), 8.05 (d, *J =* 7.3 Hz, 1H), 7.45 (d, *J =* 8.5 Hz, 1H), 4.68 (m, 2H), 4.51 - 4.38 (m, 2H), 4.29 (q, *J* = 7.2 Hz, 1H), 4.17 (t, *J =* 8.2 Hz, 1H), 4.01 (q, *J* = 9.4 Hz, 2H), 3.75 (t, *J =* 5.0 Hz, 2H), 3.47 (s, 3H), 2.29-2.01 (m, 10H), 1.78-1.51 (m, 2H), 1.46-1.18 (m, 10H), 0.90 (t, *J =* 6.7 Hz, 6H).

### Example 6: Synthesis of (S)-N-(10-benzyl-1,1,1-trifluoro-6,9,12,15-tetraoxo-3-oxa-5,8,11,14-tetrazahexadecan-16-yl)-6-(2,5-dioxo-2,5-dihydro-1H-pyrrol-1-yl)hexanamide (L6)

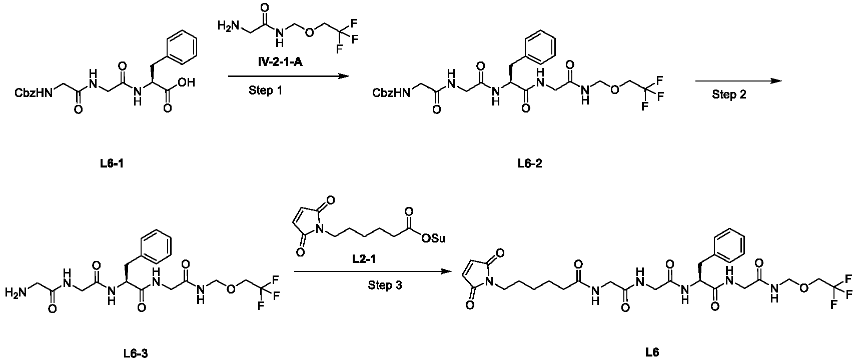

### Step 1:

Compound L6-1 (225.22 mg, 1.21 mmol) was weighed and added to a single-necked flask, anhydrous DMF (5 mL) was added and stirred at room temperature for 5 min for complete dissolution, and then compound IV-2-1-A (500 mg, 1.21 mmol) was added. The mixture was stirred at room temperature for 5 min and then cooled to -10 °C and stirred for 5 min, then DMTMM (392.25 mg, 1.33 mmol) was added to the reaction solution, and the reaction was continued at -10 °C for 1 h. The reaction was monitored by LCMS. The reaction solution was dissolved in DCM (30 mL), a saturated aqueous NaHCO₃ solution (30 mL) was added and stirred for 5 min, and the reaction system was left to stand, followed by liquid separation. The aqueous phase was extracted with DCM (20 mL), and the organic phases were combined, washed with water (50 mL), concentrated under reduced pressure to bring the volume to 10 mL, and directly purified by column chromatography (wet loading, MeOH:DCM = 0-10%) to obtain the target compound L6-2 (300 mg).

LCMS (ESI) [M+Na]⁺ = 604.28.

### Step 2:

Compound L6-2 (300 mg, 0.52 mmol) was dissolved in methanol (10 mL), Pd/C (73.02 mg, 0.052 mmol) was added under nitrogen atmosphere, and the mixture was purged with hydrogen for 3 times and reacted overnight under hydrogen atmosphere. The reaction was monitored by LCMS. The reaction solution was filtered, and the filtrate was concentrated under reduced pressure to obtain the target compound L6-3 (210 mg).

LCMS (ESI) [M+H]⁺ = 448.28.

### Step 3:

Compound L6-3 (210 mg, 0.47 mmol) and DIPEA (72.89 mg, 0.56 mmol) were dissolved in DMF (2 mL), then L2-1 (144.9 mg, 0.47 mmol) was added, and the mixture was stirred at room temperature for 3 h. The reaction was monitored by LCMS. The reaction solution was directly purified by preparative high-performance liquid chromatography (acetonitrile:H₂O containing 0.05% FA = 5%-50%) to obtain the target compound L6 (200 mg).

LCMS (ESI) [M+Na]⁺ = 663.41.

¹H NMR (400 MHz, DMSO-*d₆*) δ 8.71 (t, *J =* 6.7 Hz, 1H), 8.33 (t, *J =* 5.8 Hz, 1H), 8.14 (d, *J =* 8.0 Hz, 1H), 8.08 (t, *J =* 5.5 Hz, 1H), 8.01 (t, *J =* 5.5 Hz, 1H), 7.31-7.18 (m, 5H), 7.02 (s, 2H), 4.71 (d, *J =* 6.8 Hz, 2H), 4.56-4.50 (m, 1H), 4.04 (q, *J =* 9.4 Hz, 2H), 3.83-3.59 (m, 6H), 3.40 (t, *J =* 7.1 Hz, 2H), 3.12-3.06 (m, 1H), 2.88-2.80 (m, 1H), 2.14 (t, *J =* 7.4 Hz, 2H), 1.57-1.45 (m, 4H), 1.28-1.18 (m, 2H).

### Example 7: Synthesis of (S)-2-((S)-2-(6-(2,5-dioxo-2,5-dihydro-1H-pyrrol-1-yl)hexanamido)-3-methylbutanamido)-6-(dipropylamino)-N-(2-(((3-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)amino)-2-oxoethyl)hexanamide (IIb)

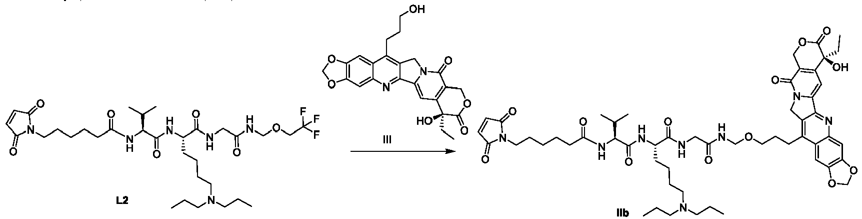

Compound L2 (10 mg, 0.022 mmol) and compound III (30 mg, 0.044 mmol) were dissolved in DMF (0.2 mL), and HCl/DMF (1 N, 0.11 mL) was added. The reaction solution was stirred at room temperature for 3 h. The reaction solution was directly purified by preparative high-performance liquid chromatography to obtain the target compound IIb (8 mg).
LCMS (ESI) [M/2+H]⁺ = 521.7;
¹H NMR (400 MHz, DMSO-*d₆*) δ 8.59 (t, *J =* 6.4 Hz, 1H), 8.23 (s, 1H), 8.17 (t, *J =* 5.7 Hz, 1H), 7.95 (t, *J =* 8.0 Hz, 1H), 7.81 (d, *J =* 8.5 Hz, 1H), 7.61 (s, 1H), 7.53 (s, 1H), 7.27 (s, 1H), 7.01 (s, 2H), 6.50 (s, 1H), 6.31 (s, 2H), 5.50 - 5.40 (m, 2H), 5.26 (s, 2H), 4.66-4.60 (m, 2H), 4.22 (dd, *J =* 13.4, 7.5 Hz, 1H), 4.14 (t, *J =* 7.8 Hz, 1H), 3.80-3.70 (m, 3H), 3.54- 3.51 (m, 2H), 3.16 - 3.11 (m, 2H), 2.38 - 2.32 (m, 6H), 2.22 - 2.06 (m, 2H), 1.98 - 1.85 (m, 5H), 1.72- 1.65 (m, 1H), 1.55- 1.52 (m, 1H), 1.51-1.45 (m, 4H), 1.40 - 1.35 (m, 7H), 1.29 - 1.17 (m, 4H), 0.90 (t, *J =* 7.3 Hz, 3H), 0.84- 0.80 (m, 12H).

### Preparation of ADCs

### Example 1: Preparation of ADC-01

The heavy chain amino acid sequence (SEQ ID NO: 1) of 2E3-02 was subjected to codon optimization and gene synthesis (Sangon Biotech (Shanghai) Co., Ltd.), and then was constructed into a PTT5 vector and named PPT5-02-CH; the light chain amino acid sequence (SEQ ID NO: 2) of 2E3-02 was subjected to codon optimization and gene synthesis (Sangon Biotech (Shanghai) Co., Ltd.), and then was constructed into a PTT5 vector and named PTT5-02-CL; the anti-B7H3 antibody expression plasmid PTT5-02-CH/PTT5-02-CL was co-transfected into HEK293F cells (ATCC) by using a PEI max reagent and expressed in a shaker at 37 °C with 5% CO₂ for 7 days. The supernatant was collected and purified by ProA magnetic beads to obtain the anti-B7H3 antibody 2E3-02 (the heavy chain sequence thereof was set forth in SEQ ID NO: 1 and the light chain sequence thereof was set forth in SEQ ID NO: 2).

To 25 mL of the 2E3-02 antibody (anti-B7H3, at a concentration of 28.5 mg/mL, 20 mM acetic acid buffer) was added 25 mL of 20 mM acetic acid buffer for dilution, and then 1 mL of an aqueous solution containing 0.25 M EDTA was added and mixed well. The pH of the sample was adjusted to 7.6 with 0.5 M aqueous disodium hydrogen phosphate solution, and then a 4.5-fold equivalent (relative to the antibody) of 20 mM TCEP (tris(2-carboxyethyl)phosphine hydrochloride) was added and mixed well. The mixture was reacted at room temperature for 90 min. Finally, a 10-fold equivalent (relative to the antibody) of IIa dissolved in DMSO was added and mixed well. The mixture was reacted at room temperature for 2 h. After the reaction was completed, the sample was exchanged into 10 mM histidine buffer with a pH of 5.5 using a 30 KDa ultrafiltration tube to remove low-molecular-weight substances, and finally, the sample was concentrated to obtain a solution of the ADC composition (ADC-01) containing the anti-B7H3 antibody, whose DAR value was determined to be 7.98 by mass spectrometry.

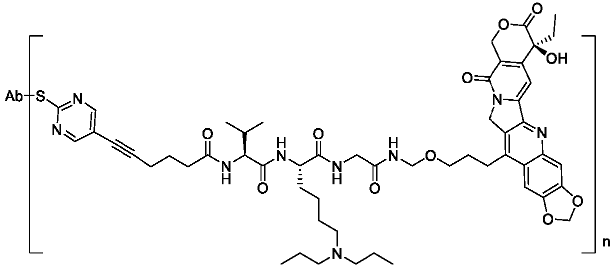

### Ab was the B7H3 antibody 2E3-02.

The DAR value was determined as follows:
Chromatography conditions:
chromatographic column: PLRP-S, 2.1 × 50 mm, 5 µm;
mobile phase A: 0.1% FA/H₂O, mobile phase B: 0.1% FA/ACN;
column temperature: 30 °C; sample chamber temperature: 8 °C; flow rate: 0.6 mL/min; injection volume: 2 µL

| | | | | | |
|---|---|---|---|---|---|
| Time (min) | 1 | 5 | 5.1 | 7 | 10 |
| Mobile phase A | 90 | 40 | 10 | 90 | 90 |
| Mobile phase B | 10 | 60 | 90 | 10 | 10 |

Sample treatment: to 50 µg of the ADC-01 sample was added 2 µL of 1M DTT, and ultrapure water was added to bring the volume to 50 µL, such that the solution was diluted to a concentration of about 1.0 mg/mL. The solution was well mixed and reduced at room temperature for 30 min.

LC/MS model: Agilent 1290-6545XT Q-TOF.

Conditions for mass spectrometry: gas temp: 320 °C, drying gas: nebulizer: 35 psi; sheath gas temp: 350 °C; sheath gas flow: 11 L/min; m/z: 500-3000.

The results are as follows:

| Peptide chain | | mAb | DAR1 | DAR2 | DAR3 |
|---|---|---|---|---|---|
| LC | Theoretical value | 23245 | 24262.4 | 25279.8 | 26297.2 |
| | Measured value | 23247.41 | 24265.49 | Undetectable | Undetectable |
| HC | Theoretical value | 50367 | 51384.4 | 52401.8 | 53419.2 |
| | Measured value | Undetectable | Undetectable | Undetectable | 53426.64 |

In the table, mAb represents an unconjugated antibody; LC represents an antibody light chain; HC represents an antibody heavy chain; DAR1 represents a conjugate comprising a light chain or a heavy chain conjugated to 1 toxin molecule; DAR2 represents a conjugate comprising a light chain or a heavy chain conjugated to 2 toxin molecules; DAR3 represents a conjugate comprising a light chain or a heavy chain conjugated to 3 toxin molecules; wherein the theoretical molecular weight of the monoclonal antibody is calculated by G0F glycoform. In the following text, mAb, LC, HC, DAR1, DAR2, and DAR3 are as described above.

It was determined that the 2E3-02 antibody light chain was conjugated to 0-1 toxin molecule (the proportions of LC and DAR1 were 1.0% and 99.0%, respectively), and the antibody heavy chain was conjugated to 0-3 toxin molecules (the proportions of mAb, DAR1, DAR2, and DAR3 were 0%, 0%, 0%, and 100%, respectively). Therefore, the drug-to-antibody ratio (DAR value) of ADC-01 was calculated to be 7.98.

### ADC Bioactivity Assay

### Example 1: ADC Assay for Efficacy on NCI-HT29 Xenograft

### 1. Materials

Test compound: ADC-01
Experimental cells: NCI-HT29 cells, purchased from ATCC;
Experimental animals: Balb/c nu nude mice, female, 5-6 weeks old, purchased from Vital River Laboratory Animal Technology Co., Ltd.

### 2. Experimental schemes

### 2.1. Cell treatment

NCI-HT29 cells were cultured in a 1640 medium containing 10% FBS in a culture dish with a diameter of 15 cm and digested with trypsin-EDTA when the confluence reached about 80%-90%. The cells were washed twice with PBS, centrifuged, resuspended in pre-cooled PBS and counted on a cell counter. The cells were diluted with PBS to bring the cell concentration to 5 × 10⁷ cells/mL.

### 2.2. Tumor cell transplantation

Balb/c nu mice were acclimated to the laboratory environment for 2-5 days, and subcutaneously inoculated with NCI-HT29 cells in the right flank in an inoculation amount of 5 × 10⁶ cells/mouse with an inoculation volume of 0.2 mL (containing 50% Matrigel). When the tumor grew to about 250 mm³, the experiment was conducted.

### 2.3. Animal administration and detection

The enrolled tumor-bearing nude mice were administered according to the following schemes:

| No. | Group | Number of animals | Administration dose | Route and period of administration |
|---|---|---|---|---|
| 1 | ADC-01 | 5 | 3 mg/kg | i.v., QW×3 |

After the period of administration was over, the mice were observed for 1-2 weeks. After the last tumor volume measurement, the tumor was separated, accurately weighed and photographed for recording.

### 2.4. Measurement of tumor volume and body weight:

The tumor volume and the body weight were measured 2 times per week, and TGI% was calculated.

Tumor volume (V) was calculated using the formula: V = 1/2 × L_{long} × Lₛₕₒᵣₜ.²

### 3. Experimental results

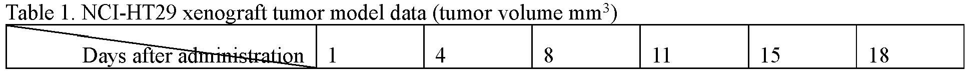

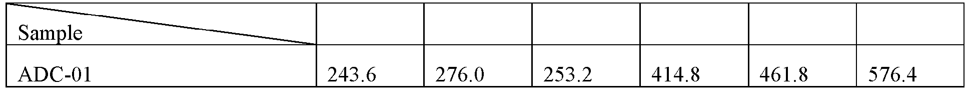

Conclusion: as shown in Table 1, the ADC of the present application has a very strong tumor-inhibiting effect. During the administration, there was no significant weight loss and no significant drug toxicity in each group of animals.

The present invention is not limited to the aforementioned embodiments. The present invention extends to any new features or any new combination disclosed in this specification, as well as to the steps of any new method or process or any new combination disclosed.

## Claims

1. A preparation method for a compound of formula II or a salt thereof, comprising step i) reacting a compound of formula III or a salt thereof with a compound of formula X-3 or a salt thereof to obtain the compound of formula II or the salt thereof, 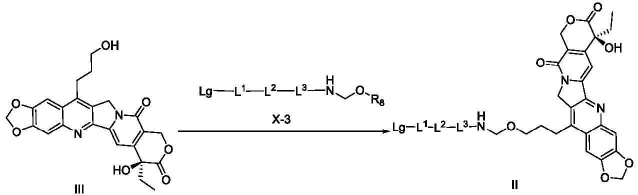
wherein, L¹ is selected from: 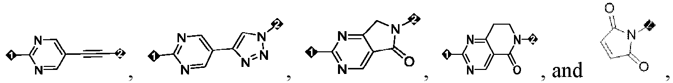 wherein position 1 is attached to Lg, and position 2 is attached to L²;
Lg is a leaving group when reacting with an antibody; Lg is selected from halogen, a sulfone group, a tertiary amine salt group (Me₃N⁺ or Et₃N⁺), a diazonium salt group, -OMs, MeSO₂-, and CF₃SO₃-; preferably, Lg is selected from F, Cl, and MeSO₂-; more preferably, Lg is MeSO₂-;
when L¹ is 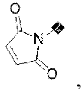 Lg is absent;
L² is selected from 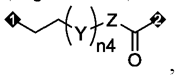 wherein position 1 is attached to L¹, and position 2 is attached to L³; n4 is selected from any integer between 0 and 10;
Y is selected from -CH₂- and -OCH₂CH₂-;
Z is selected from CR^{m}Rⁿ and NR^{m};
R^{m} and Rⁿ are each independently selected from H, deuterium, C1-4 alkyl, C2-4 alkenyl, C2-4 alkynyl, C3-6 cycloalkyl, and 3-6 membered heterocyclyl,
or R^{m} and Rⁿ, together with the carbon atom to which they are both attached, form a 3-6 membered carbocyclic ring or a 3-6 membered heterocyclic ring;
L³ is selected from Val-Ala, AA¹-Val-Ala, Val-AA¹-Gly, Ala-AA'-Gly, Gly-AA¹-Gly, Val-AA¹-Ala, Val-AA¹-Val, Ala-AA¹-Ala, Ala-AA¹-Val, Gly-AA¹-Ala, Gly-AA¹-Val, Ala-Ala-Ala, Ala-Ala-Asn, Gly-Gly-Phe-Gly, and Gly-Gly-Val-Ala;
the structure of the amino acid residues represented by AA¹ is shown as follows: 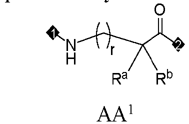
wherein,
R^{a} and R^{b} are each independently selected from H and 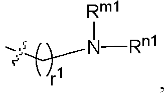 and R^{a} and R^{b} are not both H;
or R^{a} and R^{b}, together with the carbon atom to which they are both attached, form a 4-10 membered heterocyclic ring, and the 4-10 membered heterocyclic ring is optionally substituted with one or more R⁰; r is 0, and r¹ is 4;
R^{m1} and Rⁿ¹ are each independently selected from H, C1-6 alkyl, and C3-6 cycloalkyl;
or R^{m1} and Rⁿ¹, together with the nitrogen atom to which they are both attached, form a 4-10 membered heterocyclic ring, and the 4-10 membered heterocyclic ring is optionally substituted with one or more R^{0'}; R⁰ and R^{0'} are each independently selected from C1-6 alkyl, C3-6 cycloalkyl, -NR^{m2}Rⁿ², and 4-10 membered heterocyclyl optionally substituted with C1-6 alkyl;
R^{m2} and Rⁿ² are each independently selected from H and C1-6 alkyl;
R₈ is selected from hydrogen, C1-30 alkyl, C3-7 cycloalkyl, 3-20 membered heterocyclyl, C1-6 alkyl-C3-6 cycloalkyl, C1-6 alkyl-4-6 membered heterocyclyl, C1-6 alkyl-C5-10 heteroaryl, and C1-6 alkyl-C6-10 aryl, and the alkyl, cycloalkyl, heterocyclyl, or aryl is optionally substituted with one or more R^{x};
R^{x} is selected from hydrogen, deuterium, fluoro, chloro, bromo, methyl, methoxy, amino, dimethylamino, nitro, cyano, and azido.

2. The preparation method for a compound of formula II or a salt thereof according to claim 1, wherein one or more of the following conditions are met:
(1) L¹ is selected from 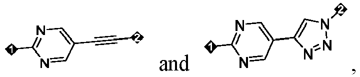 wherein position 1 is attached to Lg, and position 2 is attached to L²; preferably, L¹ is 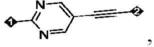 wherein position 1 is attached to Lg, and position 2 is attached to L²;
or L¹ is selected from 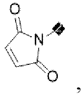 and position 2 is attached to L²;
(2) Y is -CH₂-;
(3) n4 is selected from 0, 1, 2, and 3;
(4) Z is selected from -CH₂-, -C(CH₃)₂-, -N(CH₃)-, and -NH-;
(5) R^{m} and Rⁿ are each independently selected from H, C1-4 alkyl, C2-4 alkenyl, C2-4 alkynyl, C3-6 cycloalkyl, and 3-6 membered heterocyclyl; or R^{m} and Rⁿ, together with the carbon atom to which they are both attached, form a 3-6 membered carbocyclic ring or a 3-6 membered heterocyclic ring;
preferably, R^{m} and Rⁿ are each independently selected from H and Me;
(6) L³ is selected from Val-AA¹-Gly, Ala-AA'-Gly, Gly-AA¹-Gly, Val-AA¹-Ala, Val-AA¹-Val, Ala-AA¹-Ala, Ala-AA¹-Val, Gly-AA¹-Ala, Gly-AA¹-Val, Ala-Ala-Ala, Ala-Ala-Asn, and Gly-Gly-Phe-Gly;
preferably, L³ is selected from Ala-Ala-Ala, Ala-Ala-Asn, Val-AA¹-Gly, and Gly-Gly-Phe-Gly; further preferably, L³ is Val-AA¹-Gly;
(7) one of R^{a} and R^{b} is H, and the other is 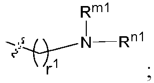 or R^{a} and R^{b}, together with the carbon atom to which they are both attached, form a 5-6 membered heterocyclic ring substituted with R⁰ (preferably, R^{a} and R^{b}, together with the carbon atom to which they are both attached, form a piperidine ring or a piperazine ring substituted with R⁰; more preferably, R^{a} and R^{b}, together with the carbon atom to which they are both attached, form a piperidine ring substituted with R⁰; further preferably, R^{a} and R^{b}, together with the carbon atom to which they are both attached, form 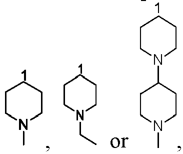 wherein the carbon atom at position 1 is the carbon atom to which R^{a} and R^{b} are both attached);
(8) R^{m1} and Rⁿ¹ are each independently selected from H and C1-6 alkyl (preferably, R^{m1} and Rⁿ¹ are each independently selected from H, methyl, ethyl, *n*-propyl, and *n*-butyl); or R^{m1} and Rⁿ¹, together with the nitrogen atom to which they are both attached, form a 5-6 membered heterocyclic ring optionally substituted with R^{0'} (preferably, R^{m1} and Rⁿ¹, together with the nitrogen atom to which they are both attached, form a piperidine ring or a piperazine ring optionally substituted with R^{0'}; more preferably, R^{m1} and Rⁿ¹, together with the nitrogen atom to which they are both attached, form 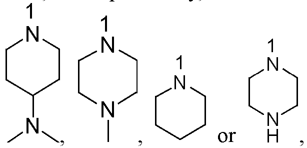 wherein the nitrogen atom at position 1 is the nitrogen atom to which R^{m1} and Rⁿ¹ are both attached);
(9) R⁰ and R^{0'} are each independently selected from C1-6 alkyl, -NR^{m2}Rⁿ², and 5-6 membered heterocyclyl optionally substituted with C1-6 alkyl;
preferably, R⁰ is selected from C1-6 alkyl and 5-6 membered heterocyclyl substituted with C1-6 alkyl, and the 5-6 membered heterocyclyl is selected from piperidinyl and piperazinyl; more preferably, R⁰ is selected from methyl, ethyl, and 5-6 membered heterocyclyl substituted with methyl, and the 5-6 membered heterocyclyl is piperidinyl; further preferably, R⁰ is selected from methyl, ethyl, and 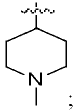
preferably, R^{0'} is selected from C1-6 alkyl and -NR^{m2}Rⁿ²; more preferably, R^{0'} is selected from methyl and - NR^{m2}Rⁿ²;
(10) R^{m2} and Rⁿ² are methyl;
(11) R₈ is selected from hydrogen, C1-30 alkyl (e.g., C1-6 alkyl), and C1-6 alkyl-C6-10 aryl, and the alkyl or aryl is optionally substituted with one or more R^{x}; and R^{x} is selected from hydrogen, fluoro, methyl, and methoxy; preferably, R₈ is selected from hydrogen, methyl, ethyl, isopropyl, *tert*-butyl, 2,2,2-trifluoroethyl, 2,2,3,3,3-pentafluoro-1-propyl, and benzyl; more preferably, R₈ is selected from hydrogen, isopropyl, *tert-*butyl, 2,2,2-trifluoroethyl, 2,2,3,3,3-pentafluoro-1-propyl, and benzyl.

3. The preparation method for a compound of formula II or a salt thereof according to claim 1, wherein one or more of the following conditions are met:
(1) when L¹-L² is selected from 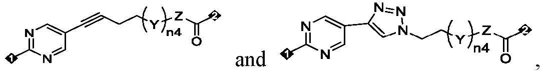 position 1 is attached to Lg, and position 2 is attached to L³; or L¹-L² is selected from 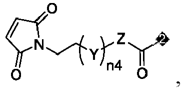 wherein position 2 is attached to L³; wherein L² is preferably selected from 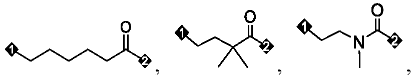 and 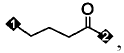 wherein position 1 is attached to L¹, and position 2 is attached to L³; L² is more preferably selected from 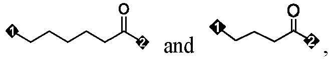 wherein position 1 is attached to L¹, and position 2 is attached to L³; L² is further preferably 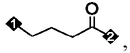 wherein position 1 is attached to L¹, and position 2 is attached to L³;
preferably, L¹-L² is selected from 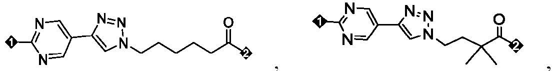 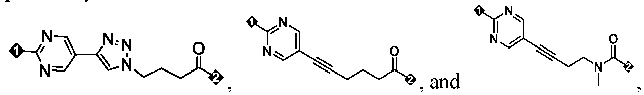 wherein position 1 is attached to Lg, and position 2 is attached to L³; or selected from 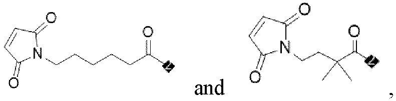 wherein position 2 is attached to L³; more preferably, L¹-L² is selected from 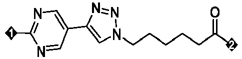 and 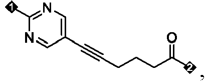 wherein position 1 is attached to Lg, and position 2 is attached to L³; or selected from 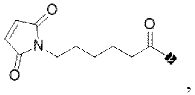 wherein position 2 is attached to L³; for example, L¹-L² is 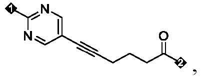 wherein position 1 is attached to Lg, and position 2 is attached to L³;
(2) L³ is selected from 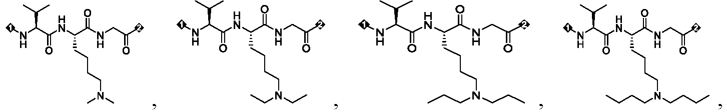 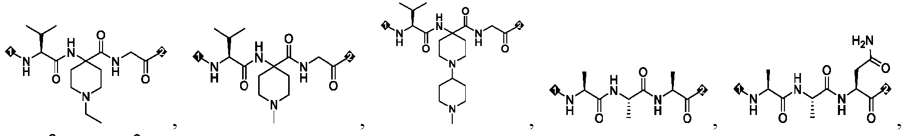 and 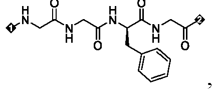 wherein position 1 is attached to L², and position 2 is attached to NH;
preferably, L³ is 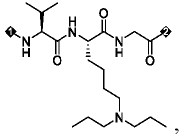 wherein position 1 is attached to L², and position 2 is attached to NH.

4. The preparation method for a compound of formula II or a salt thereof according to claim 1, wherein one or more of the following conditions are met:
(1) in step i), the compound of formula III or the salt thereof is reacted with the compound of formula X-3 or the salt thereof after azeotropic dehydration to obtain the compound of formula II or the salt thereof; preferably, in step i), the azeotropic solvent is selected from toluene, xylene, chloroform, acetonitrile, ethyl acetate, and dichloroethane, preferably toluene and xylene;
(2) in step i), the compound of formula III or the salt thereof is reacted with the compound of formula X-3 or the salt thereof without the addition of an acid to obtain the compound of formula II or the salt thereof, wherein the reaction temperature is selected from 110 °C to 150 °C, preferably 120 °C to 130 °C;
(3) in step i), the compound of formula III or the salt thereof is reacted with the compound of formula X-3 or the salt thereof under an acidic condition to obtain the compound of formula II or the salt thereof; the reaction temperature is preferably selected from 0 °C to 80 °C; more preferably 15 °C to 40 °C; further preferably 20 °C to 35 °C;
preferably, in step i), the acid is a protic or aprotic acid, for example, hydrogen chloride, hydrobromic acid, sulfuric acid, boron trifluoride etherate, p-toluenesulfonic acid, pyridinium p-toluenesulfonate, zinc acetate, aluminum trichloride (AlCl₃), ferric trichloride (FeCl₃), ytterbium(III) trifluoromethanesulfonate (Yb(OTf)₃), triethylamine hydrochloride, boron trifluoride acetonitrile, boron trifluoride tetrahydrofuran, pyridine hydrochloride, and pyridine hydrobromide; preferably, hydrogen chloride, hydrobromic acid, sulfuric acid, trifluoroacetic acid, *p*-toluenesulfonic acid, pyridinium *p*-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); for another example, hydrogen chloride or boron trifluoride etherate; more preferably, hydrogen chloride or sulfuric acid; most preferably, boron trifluoride etherate;
preferably, in step i), a molar ratio of the compound of formula III or the salt thereof to the acid selected for the reaction is selected from 1:0.2-1:6, preferably 1:2-1:5, for example, 1:2.5, 1:3, or 1:5;
(4) in step i), a molar ratio of the compound of formula III or the salt thereof to the compound of formula X-3 or the salt thereof is selected from 1:1-1:5, preferably 1:2-1:3;
(5) in step i), a reaction solvent is selected from one or any combination of ethers, nitriles, amides, sulfones or water; preferably, amides or sulfones; more preferably, *N,N*-dimethylformamide (DMF), *N,N-*dimethylacetamide (DMAc), *N*-methylpyrrolidone (NMP), or dimethylsulfoxide (DMSO); further preferably, *N,N*-dimethylformamide; preferably, in step i), a mass-to-volume ratio (g/mL) of the compound of formula III or the salt thereof to the selected solvent is selected from 1:2-1:10, preferably 1:2.5-1:7.

5. The preparation method for a compound of formula II or a salt thereof according to claim 1, wherein the preparation method for the compound of formula II or the salt thereof further comprises a preparation method for the compound of formula X-3 or the salt thereof, comprising the following steps:
m-1) removing a protecting group PG₂ on an amino group from a compound of formula IV-2 or a salt thereof to obtain a compound of formula X-3-1 or a salt thereof; and 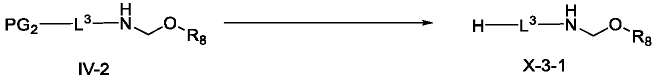
m-2) subjecting the compound of formula X-3-1 or the salt thereof and a compound of formula X-1 to a condensation reaction to obtain the compound of formula X-3 or the salt thereof; 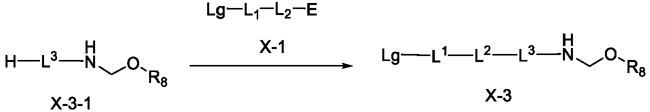
wherein E is selected from hydroxy, halogen, activated hydroxy, such as hydroxy, chlorine, bromine, 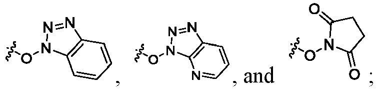
the PG₂ is selected from an amino protecting group; preferably, the PG₂ is selected from benzyloxycarbonyl (Cbz), *tert*-butoxycarbonyl (Boc), 9-fluorenylmethoxycarbonyl (Fmoc), allyloxycarbonyl (Alloc), trimethylsilylethoxycarbonyl (Teoc), methoxycarbonyl, or ethoxycarbonyl; further preferably, the PG₂ is 9-fluorenylmethoxycarbonyl (Fmoc), or the PG₂ is benzyloxycarbonyl (Cbz);
Lg, L¹, L², L³, and R₈ are as defined in any one of claims 1 to 3.

6. The preparation method for a compound of formula II or a salt thereof according to claim 5, wherein one or more of the following conditions are met:
(1) in step m-1), the removal is carried out under an acidic condition, wherein the acid preferably comprises a protic acid and an aprotic acid; or the removal is carried out under an alkaline condition, wherein the base preferably comprises an organic base and an inorganic base; or the removal is carried out under a condition of a metal reagent, wherein the metal reagent is preferably selected from palladiums or platinums, further preferably palladium on carbon, platinum on activated carbon, platinum dioxide, and palladium hydroxide; preferably, in step m-1), when the PG₂ is 9-fluorenylmethoxycarbonyl (Fmoc), the protecting group PG₂ is removed from the compound of formula IV-2 or the salt thereof under an alkaline condition; the base is preferably selected from piperidine, diethylamine, morpholine, and DBU, more preferably diethylamine; a molar ratio of the compound of formula IV-2 or the salt thereof to the selected base is preferably selected from 1:0.2-1:40, more preferably 1:1-1:10;
preferably, in step m-1), when the PG₂ is benzyloxycarbonyl (Cbz), the protecting group PG₂ is removed from the compound of formula IV-2 or the salt thereof in a metal reagent-hydrogen system; the metal reagent is palladiums or platinums, preferably palladium on carbon, platinum on activated carbon, platinum dioxide, and palladium hydroxide, for example, palladium on carbon or palladium hydroxide; a mass ratio of the compound of formula IV-2 to the metal reagent is preferably 1:0.01-1, preferably 1:0.03-0.5, for example, 1:0.3; the hydrogen used is preferably at 1 atm to 50 atm, for example, 1 atm to 4 atm;
(2) in step m-1), when the PG₂ is 9-fluorenylmethoxycarbonyl (Fmoc), a reaction solvent is selected from DMF, dichloromethane, tetrahydrofuran, and 1,4-dioxane, preferably DMF; preferably, a mass-to-volume ratio (g/mL) of the compound of formula IV-2 or the salt thereof to the selected reaction solvent is selected from 1:5-1:50, preferably 1:6-1:20;
(3) in step m-1), when the PG₂ is 9-fluorenylmethoxycarbonyl (Fmoc), a reaction temperature is selected from 0 °C to 50 °C, preferably 15 °C to 30 °C;
(4) in step m-1), when the PG₂ is benzyloxycarbonyl (Cbz), the reaction temperature is 0 °C to 100 °C, preferably 20 °C to 70 °C;
(5) in step m-1), when the PG₂ is benzyloxycarbonyl (Cbz), the reaction solvent is selected from any one of alcohols, ethers, esters, amides, or water or a mixture thereof at any ratio, preferably methanol, ethanol, tetrahydrofuran, ethyl acetate, DMF, DMAc, NMP, or water, for example, methanol or tetrahydrofuran; the mass-to-volume ratio (g/mL) of the compound of formula IV-2 or the salt thereof to the selected reaction solvent is preferably selected from 1:3-1:50, for example, 1:10-1:30;
(6) in step m-2), the compound of formula X-3-1 or the salt thereof and the compound of formula X-1 or the salt thereof are subjected to a condensation reaction to obtain the compound of formula X-3 or the salt thereof;
(7) in step m-2), when E is hydroxy, the reaction is carried out under the action of a condensing agent and under an alkaline or neutral condition;
the condensing agent is preferably DMTMM, HATU, HBTU, COMU, or N-ethynyl-N-methylmethanesulfonamide, more preferably DMTMM or HBTU;
the base is selected from organic or inorganic bases, preferably triethylamine, N,Ndiisopropylethylamine, and N-methylmorpholine, more preferably N,Ndiisopropylethylamine;
a molar ratio of the compound of formula X-1 or the salt thereof to the condensing agent is preferably selected from 1:1-1:5, more preferably 1:1-1:1.5;
(8) in step m-2), when E is hydroxy, a molar ratio of the compound of formula X-3-1 or the salt thereof to the compound of formula X-1 or the salt thereof is selected from 1:0.9-1:1.2;
(9) in step m-2), when E is hydroxy, a reaction solvent is selected from one or any combination of DMF, acetonitrile, tetrahydrofuran, methanol, dichloromethane, and water, preferably DMF;
(10) in step m-2), when E is hydroxy, a reaction temperature is selected from -20 °C to 100 °C; for example - 20 °C to 30 °C; preferably 0 °C to 30 °C;
(11) in step m-2), when E is selected from halogen (e.g., chlorine, fluorine, or bromine), the compound of formula X-3-1 or the salt thereof is reacted with the compound of formula X-1 or the salt thereof under an alkaline condition;
the base is preferably selected from triethylamine, *N,N*-diisopropylethylamine, DBU, and *N-*methylmorpholine, more preferably *N,N*-diisopropylethylamine;
a molar ratio of the compound of formula X-1 or the salt thereof to the base of the reaction is preferably selected from 1:0.1-1:5; for example 1:1-1:5; more preferably 1:0.2-1:1;
(12) in step m-2), when E is halogen (e.g., chlorine, fluorine, or bromine), a molar ratio of the compound of formula X-1 or the salt thereof to the compound of formula X-3-1 or the salt thereof is selected from 1:0.8-1:2.0, preferably 1:0.9-1:1.2;
(13) in step m-2), when E is halogen (e.g., chlorine, fluorine, or bromine), the reaction solvent is selected from one or any combination of DMF, tetrahydrofuran, dichloromethane, and acetonitrile, preferably acetonitrile; a mass-to-volume ratio (g/mL) of the compound of formula X-1 or the salt thereof to the selected reaction solvent is preferably selected from 1:2-1:50, more preferably 1:3-1:10;
(14) in step m-2), when E is halogen (e.g., chlorine, fluorine, or bromine), the reaction temperature is selected from -20 °C to 100 °C, preferably 0 °C to 30 °C.

7. The preparation method for a compound of formula II or a salt thereof according to claim 5, wherein the preparation method for the compound of formula X-3 or the salt thereof further comprises a preparation method for the compound of formula IV-2 or the salt thereof as follows:
when R₈ is not H, the method comprises the following steps:
k-1) removing a protecting group PG₁ on an amino group from a compound of formula IV-1 or a salt thereof to obtain a compound of formula IV-2-1 or a salt thereof; and 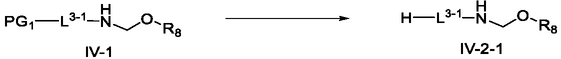
k-2) subjecting the compound of formula IV-2-1 or the salt thereof and a compound of formula IV-2-2 or a salt thereof to a condensation reaction to obtain the compound of formula IV-2 or the salt thereof; 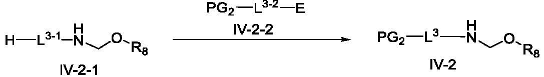
wherein PG₂-L³ is PG₂-L³⁻²-L³⁻¹;
wherein,
L³⁻¹ is selected from an amino acid residue, and the amino acid residue is selected from 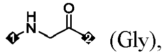 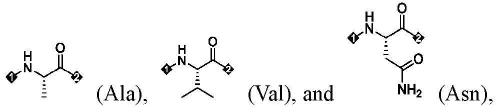 wherein position 1 is attached to L³⁻², and position 2 is attached to NH; preferably, L³⁻¹ is selected from an amino acid residue, and the amino acid residue is selected 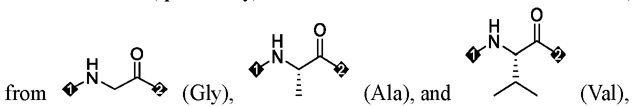 wherein position 1 is attached to L³⁻², and position 2 is attached to NH; more preferably, L³⁻¹ is Gly;
the PG₁ is selected from an amino protecting group; the PG₁ is preferably selected from benzyloxycarbonyl (Cbz), *tert*-butoxycarbonyl (Boc), 9-fluorenylmethoxycarbonyl (Fmoc), allyloxycarbonyl (Alloc), trimethylsilylethoxycarbonyl (Teoc), methoxycarbonyl, or ethoxycarbonyl; preferably, the PG₁ is selected from benzyloxycarbonyl (Cbz) and 9-fluorenylmethoxycarbonyl (Fmoc); more preferably, the PG₁ is 9-fluorenylmethoxycarbonyl (Fmoc); for example, the PG₁ is benzyloxycarbonyl (Cbz);
L³⁻² is selected from Val, Val-AA¹, Ala-AA¹, Gly-AA¹, Ala-Ala, AA¹-Val, Gly-Gly-Val, and Gly-Gly-Phe; preferably, L³⁻² is selected from Val-AA¹, Ala-Ala, and Gly-Gly-Phe; for example, L³⁻² is Val-AA¹;
specifically, L³⁻² is selected from 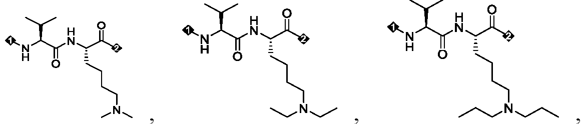 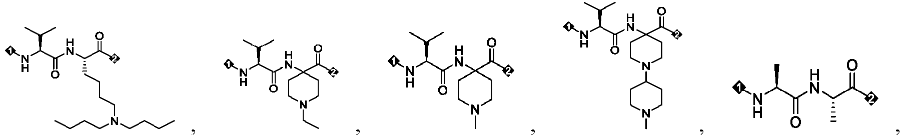 and 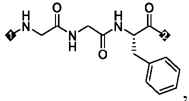 wherein position 1 is attached to L², and position 2 is attached to L³⁻¹; more preferably,
L³⁻² is 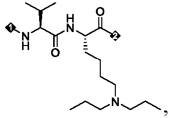 wherein position 1 is attached to L², and position 2 is attached to L³⁻¹;
L³, R₈, and E are as defined in any one of claims 1 to 3;
the PG₂ is as defined in any one of claim 5.

8. The preparation method for a compound of formula II or a salt thereof according to claim 7, wherein one or more of the following conditions are met:
(1) in step k-1), the removal is carried out under an acidic condition, wherein the acid comprises a protic acid and an aprotic acid; or the removal is carried out under an alkaline condition, wherein the base comprises an organic base and an inorganic base; or the removal is carried out under a condition of a metal reagent, wherein the metal reagent is preferably selected from palladiums or platinums, further preferably palladium on carbon, platinum on activated carbon, platinum dioxide, and palladium hydroxide;
preferably, in step k-1), when the PG₁ is 9-fluorenylmethoxycarbonyl (Fmoc), the protecting group PG₁ is removed from the compound of formula IV-1 or the salt thereof under an alkaline condition; the base is selected from organic or inorganic bases, preferably piperidine, diethylamine, morpholine, diisopropylamine, DBU, triethylamine, and *N,N*-diisopropylethylamine; more preferably, piperidine, diethylamine or morpholine, for example, diethylamine or DBU; a molar ratio of the compound of formula IV-1 or the salt thereof to the selected base is preferably selected from 1:0.2-1:40; for example 1:0.2-1:2; more preferably 1:1-1:10, for example, 1:0.4;
preferably, in step k-1), when the PG₁ is benzyloxycarbonyl (Cbz), the protecting group PG₁ is removed from the compound of formula IV-1 or the salt thereof in a metal reagent-hydrogen system; the metal reagent is selected from palladiums or platinums, preferably palladium on carbon, platinum on activated carbon, platinum dioxide, and palladium hydroxide, for example, palladium on carbon or palladium hydroxide; a mass ratio of the compound of formula IV-1 to the metal reagent is preferably 1:0.01-1, preferably 1:0.03-0.5, for example, 1:0.05 or 1:0.1; the hydrogen used is preferably at 1 atm to 50 atm;
(2) in step k-1), when the PG₁ is 9-fluorenylmethoxycarbonyl (Fmoc), a reaction solvent is selected from one or any combination of DMF, DMAc, NMP, dichloromethane, tetrahydrofuran, 1,4-dioxane, and acetonitrile, for example, DMF or tetrahydrofuran; a mass-to-volume ratio (g/mL) of the compound of formula IV-1 or the salt thereof to the selected reaction solvent is preferably selected from 1:5-1:50, more preferably 1:6-1:20;
(3) in step k-1), when the PG₁ is 9-fluorenylmethoxycarbonyl (Fmoc), a reaction temperature is selected from 0 °C to 50 °C, preferably 15 °C to 30 °C;
(4) in step k-1), when the PG₁ is benzyloxycarbonyl (Cbz), the reaction temperature is 0 °C to 100 °C, preferably 20 °C to 70 °C;
(5) in step k-1), when the PG₁ is benzyloxycarbonyl (Cbz), the reaction solvent is selected from any one of alcohols, ethers, esters, amides, or water or a mixture thereof at any ratio, preferably methanol, ethanol, tetrahydrofuran, ethyl acetate, DMF, DMAc, NMP, or water, for example, methanol or tetrahydrofuran; the mass-to-volume ratio (g/mL) of the compound of formula IV-1 or the salt thereof to the selected reaction solvent is preferably selected from 1:3-1:20, more preferably, 1:5-1:10;
(6) in step k-2), when E is hydroxy, the reaction is carried out under the action of a condensing agent and/or an anti-racemization reagent and under an alkaline or neutral condition, and preferably, when E is hydroxy, the reaction is carried out under the action of a condensing agent and under a neutral condition;
the condensing agent is preferably DMTMM, HATU, HBTU, EDCI, COMU, N-ethynyl-N-methylmethanesulfonamide, EEDQ, and T₃P, more preferably, DMTMM or HBTU;
the anti-racemization reagent is preferably selected from HOAt and HOBt;
the base is preferably selected from DBU, triethylamine, *N,N-diisopropylethylamine,* and *N-*methylmorpholine;
a molar ratio of the compound of formula IV-2-2 or the salt thereof to the condensing agent is preferably selected from 1:1-1:5, more preferably 1:1-1:1.5;
(7) in step k-2), when E is hydroxy, a molar ratio of the compound of formula IV-2-1 or the salt thereof to the compound of formula IV-2-2 or the salt thereof is selected from 1:0.8-1:3, preferably 1:0.8-1:1.2;
(8) in step k-2), when E is hydroxy, a reaction solvent is one or any combination of DMF, acetonitrile, tetrahydrofuran, methanol, dichloromethane, and water, preferably DMF or tetrahydrofuran; a mass-to-volume ratio (g/mL) of the compound of formula IV-2-1 or the salt thereof to the selected reaction solvent is preferably selected from 1:5-1:20;
(9) in step k-2), when E is hydroxy, a reaction temperature is selected from -20 °C to 100 °C; preferably -15 °C to 50 °C; most preferably below -15 °C.

9. The preparation method for a compound of formula II or a salt thereof according to claim 7, wherein the preparation method for the compound of formula IV-2 or the salt thereof further comprises a preparation method for the compound of formula IV-1 or the salt thereof as follows:
method I, comprising step j) reacting a compound of formula IV-1-1 or a salt thereof with a compound of formula R₈OH or a salt thereof under an acidic condition or an alkaline condition to obtain the compound of formula IV-1 or the salt thereof; 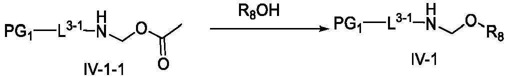
method II, comprising the following steps:
j-1) reacting a compound of formula IV-1-2 or a salt thereof with formaldehyde in water and under an alkaline condition to obtain a compound of formula IV-1-3 or a salt thereof; and 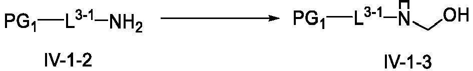
j-2) reacting the compound of formula IV-1-3 or the salt thereof with the compound of formula R₈OH or the salt thereof under an acidic condition to obtain the compound of formula IV-1 or the salt thereof; 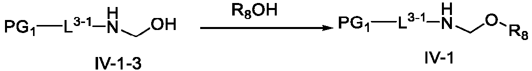
wherein L³⁻¹, PG₁, and R₈ are as defined in any scheme of claim 7.

10. The preparation method for a compound of formula II or a salt thereof according to claim 9, wherein one or more of the following conditions are met:
(1) in step j), the acid is a protic or aprotic acid, preferably, hydrogen chloride, hydrobromic acid, sulfuric acid, trifluoroacetic acid, *p*-toluenesulfonic acid, pyridinium *p*-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); more preferably, hydrogen chloride, sulfuric acid, or zinc acetate, for example, hydrogen chloride; in step j), a molar ratio of the compound of formula IV-1-1 or the salt thereof to the acid selected for the reaction is preferably selected from 1:0.01-1:0.2, for example, 1:0.05;
(2) in step j), the base is an organic or inorganic base, preferably sodium hydroxide, potassium *tert*-butoxide, potassium carbonate, triethylamine (Et₃N), N,N-diisopropylethylamine (DIPEA), and pyridine; more preferably, potassium tert-butoxide; a molar ratio of the compound of formula IV-1-1 or the salt thereof to the base selected for the reaction is preferably selected from 1:0.9-1:5;
(3) in step j), a molar ratio of the compound of formula IV-1-1 or the salt thereof to the compound of formula R₈OH or the salt thereof is selected from 1:1-1:20, preferably 1:2-1:15;
(4) in step j), a reaction solvent is selected from alcohols, ethers, haloalkanes, aromatic hydrocarbons, nitriles, amides, or sulfones; preferably, isopropanol, benzyl alcohol, *tert*-butanol, 2,2,2-trifluoroethanol, 2,2,3,3,3-pentafluoro-1-propanol, ethylene glycol diethyl ether, tetrahydrofuran, 1,4-dioxane, dichloromethane, 1,2-dichloroethane, toluene, acetonitrile, DMF, DMAc, NMP, or DMSO; further preferably, isopropanol, benzyl alcohol, *tert*-butanol, 2,2,2-trifluoroethanol, 2,2,3,3,3-pentafluoro-1-propanol, toluene, tetrahydrofuran, acetonitrile, DMF, DMAc, or NMP, for example, 2,2,2-trifluoroethanol or DMF; a mass-to-volume ratio (g/mL) of the compound of formula IV-1-1 or the salt thereof to the selected solvent is preferably selected from 1:2-1:10, more preferably 1:2.5-1:7;
(5) in step j), a reaction temperature is selected from 0 °C to 120 °C, for example, 10 °C to 50 °C;
(6) in step j-1), the base is selected from potassium carbonate, potassium bicarbonate, potassium phosphate, sodium carbonate, sodium bicarbonate, triethylamine, and diisopropylethylamine, preferably potassium carbonate;
(7) in step j-1), a molar ratio of the compound of formula IV-1-2 or the salt thereof to the base is selected from 1:0.05-1:1, preferably 1:0.1-1:0.5;
(8) in step j-1), a molar ratio of the compound of formula IV-1-2 or the salt thereof to formaldehyde is 1:0.5-1:2.5, preferably 1:1-1:2, for example, 1:1.8;
(9) in step j-1), a mass-to-volume ratio (g/mL) of the compound of formula IV-1-2 or the salt thereof to water is 1:3-1:50, preferably 1:5-1:20, for example, 1:15;
(10) in step j-1), a reaction temperature is 0 °C to 80 °C, preferably 10 °C to 50 °C;
(11) in step j-2), the acid is a protic or aprotic acid, preferably, hydrogen chloride, hydrobromic acid, sulfuric acid, trifluoroacetic acid, *p*-toluenesulfonic acid, pyridinium p-toluenesulfonate, zinc acetate, aluminum trichloride (AlCl₃), ferric trichloride (FeCl₃), boron trifluoride etherate (BF₃•Et₂O), and ytterbium(III) trifluoromethanesulfonate (Yb(OTf)₃); more preferably, hydrogen chloride, sulfuric acid, or zinc acetate, for example, hydrogen chloride; a molar ratio of the compound of formula IV-1-3 or the salt thereof to the acid selected for the reaction is preferably selected from 1:0.01-1:0.2, for example, 1:0.05;
(12) in step j-2), a molar ratio of the compound of formula IV-1-3 or the salt thereof to the compound of formula R₈OH or the salt thereof is selected from 1:1-1:50, preferably 1:2-1:30;
(13) in step j-2), a reaction solvent is selected from alcohols, ethers, haloalkanes, aromatic hydrocarbons, nitriles, amides, or sulfones; more preferably, isopropanol, benzyl alcohol, *tert*-butanol, 2,2,2-trifluoroethanol, 2,2,3,3,3-pentafluoro-1-propanol, ethylene glycol diethyl ether, tetrahydrofuran, 1,4-dioxane, dichloromethane, 1,2-dichloroethane, toluene, acetonitrile, DMF, DMAc, NMP, or DMSO; further preferably, isopropanol, benzyl alcohol, *tert*-butanol, 2,2,2-trifluoroethanol, 2,2,3,3,3-pentafluoro-1-propanol, toluene, tetrahydrofuran, acetonitrile, DMF, DMAc, or NMP, for example, 2,2,2-trifluoroethanol or DMF; a mass-to-volume ratio (g/mL) of the compound of formula IV-1-3 or the salt thereof to the selected solvent is preferably selected from 1:2-1:10, more preferably 1:2.5-1:7;
(14) in step j-2), a reaction temperature is selected from 0 °C to 120 °C, for example, 0 °C to 50 °C.

11. The preparation method for a compound of formula II or a salt thereof according to claim 1, wherein the preparation method for the compound of formula II or the salt thereof further comprises a preparation method for a compound of formula IIa or a salt thereof as follows: reacting the compound of formula III or the salt thereof with a compound of formula X-3a or a salt thereof after azeotropic dehydration to obtain the compound of formula IIa or the salt thereof, 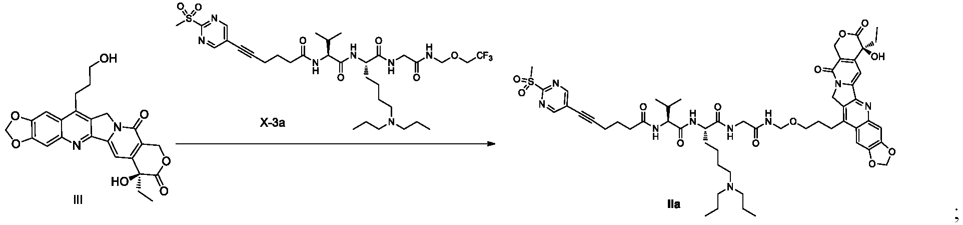 wherein
preferably, the preparation method for a compound of formula IIa or a salt thereof further comprises a preparation method of the compound of X-3a or the salt thereof as follows:
step m-1): removing a protecting group Cbz from a compound of formula IV-2-A or a salt thereof in a metal reagent-hydrogen system to obtain a compound of formula X-3-1-A or a salt thereof, 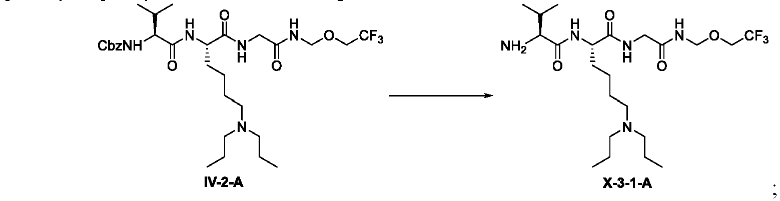 and
step m-2): reacting the compound of formula X-3-1-A or the salt thereof with a compound of formula X-1-A under an alkaline condition to obtain the compound of formula X-3a or the salt thereof, 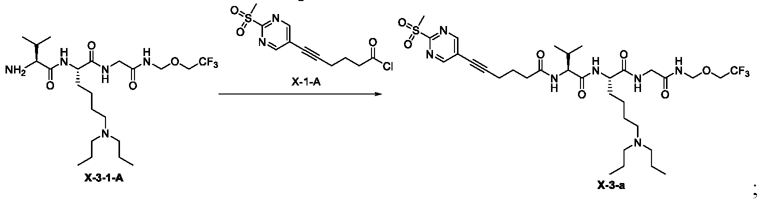
further preferably, the preparation method for a compound of formula IIa or a salt thereof further comprises a preparation method for a compound of formula IV-2a or a salt thereof as follows:
step k-1): removing a protecting group Cbz from a compound of formula IV-1-A or a salt thereof in a metal reagent-hydrogen system to obtain a compound of formula IV-2-1-A or a salt thereof, 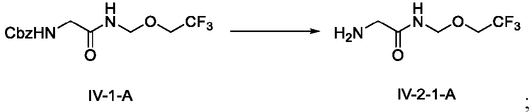 and
step k-2): reacting the compound of formula IV-2-1-A or the salt thereof with a compound of formula IV-2-2-B or a salt thereof under the action of a condensing agent and/or an anti-racemization reagent and under an alkaline or neutral condition to obtain the compound of formula IV-2-A or the salt thereof, 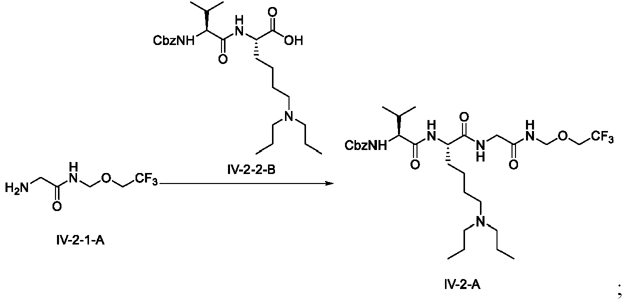
still further preferably, the preparation method for a compound of formula IIa or a salt thereof further comprises a preparation method for a compound of formula IV-1a or a salt thereof:
step j-1): reacting a compound of formula IV-1-A-2 or a salt thereof with formaldehyde in water and under an alkaline condition to obtain a compound of formula IV-1-A-3 or a salt thereof; and 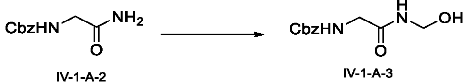
step j-2): reacting the compound of formula IV-1-A-3 or the salt thereof with CF₃CH₂OH under an acidic condition to obtain the compound of formula IV-1-A or the salt thereof;

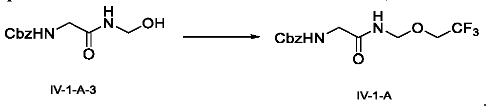

12. A preparation method for a compound of formula X-3 or a salt thereof, comprising the following steps:
m-1) removing a protecting group PG₂ on an amino group from a compound of formula IV-2 or a salt thereof to obtain a compound of formula X-3-1 or a salt thereof; and 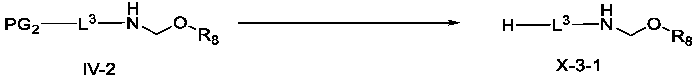
m-2) subjecting the compound of formula X-3-1 or the salt thereof and a compound of formula X-1 to a condensation reaction to obtain the compound of formula X-3 or the salt thereof; 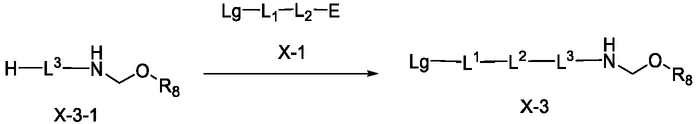
wherein,
E, PG₂, Lg, L¹, L², L³, and R₈ are as defined in any scheme of claim 5;
conditions and operations of the preparation method for a compound of formula X-3 or a salt thereof are as defined in any one of claims 5 to 10.

13. A preparation method for a compound of formula IV-2 or a salt thereof, wherein when R₈ is not H, the method comprises the following steps:
k-1) removing a protecting group PG₁ on an amino group from a compound of formula IV-1 or a salt thereof to obtain a compound of formula IV-2-1 or a salt thereof; and 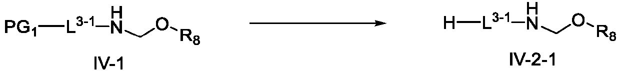
k-2) subjecting the compound of formula IV-2-1 or the salt thereof and a compound of formula IV-2-2 or a salt thereof to a condensation reaction to obtain the compound of formula IV-2 or the salt thereof; 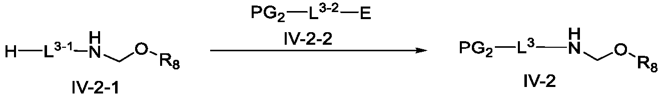
wherein PG₂-L³ is PG₂-L³⁻²-L³⁻¹;
wherein,
L³⁻¹, L³⁻², L³, PG₁, PG₂, R₈, and E are as defined in any scheme of claim 7;
conditions and operations of the preparation method for a compound of formula IV-2 or a salt thereof are as defined in any one of claims 7 to 10.

14. A preparation method for a compound of formula IV-1 or a salt thereof, comprising the following method I or method II:
method I, comprising step j) reacting a compound of formula IV-1-1 or a salt thereof with a compound of formula R₈OH or a salt thereof under an acidic condition or an alkaline condition to obtain the compound of formula IV-1 or the salt thereof; 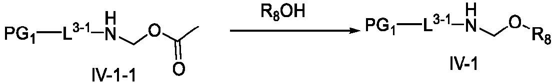
method II, comprising the following steps:
j-1) reacting a compound of formula IV-1-2 or a salt thereof with formaldehyde in water and under an alkaline condition to obtain a compound of formula IV-1-3 or a salt thereof; and 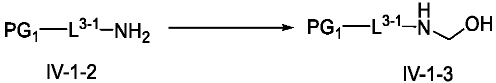
j-2) reacting the compound of formula IV-1-3 or the salt thereof with the compound of formula R₈OH or the salt thereof under an acidic condition to obtain the compound of formula IV-1 or the salt thereof; 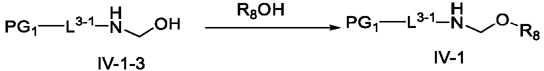
wherein L³⁻¹, PG₁, and R₈ are as defined in any scheme of claim 9;
conditions and operations of the preparation method for a compound of formula IV-1 or a salt thereof are as defined in any one of claims 9 to 10.

15. A compound of formula IV-1 or a salt thereof, a compound of formula IV-2-1 or a salt thereof, a compound of formula IV-2 or a salt thereof, a compound of formula X-3-1 or a salt thereof, and a compound of formula X-3 or a salt thereof, wherein 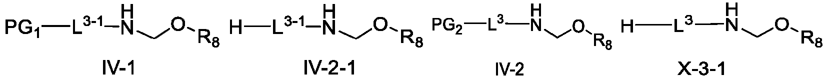 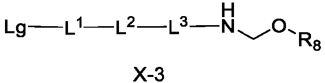
Lg, L¹, L², L³, and R₈ are as defined in any one of claims 1 to 3;
PG₂ is as defined in any scheme of claim 5;
L³⁻¹ and PG₁ are as defined in any scheme of claim 7;
preferably,
the compound of formula IV-1 or the salt thereof is selected from the following structures: 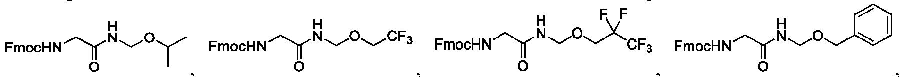 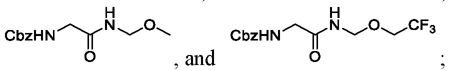
the compound of formula IV-2-1 or the salt thereof is selected from the following structures: 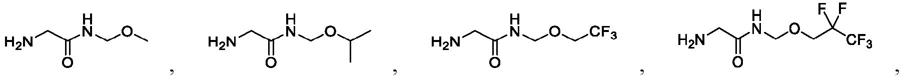 and 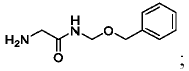
the compound of formula IV-2 or the salt thereof is selected from the following structures: 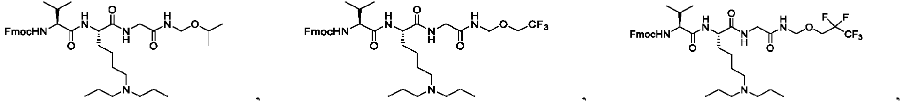 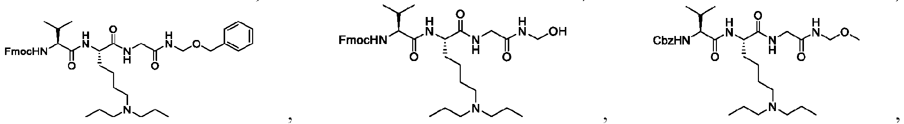 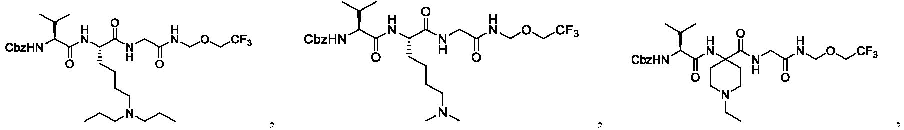 and 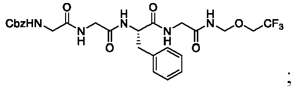
the compound of formula X-3-1 or the salt thereof is selected from the following structures: 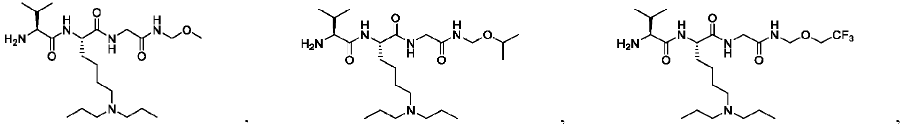 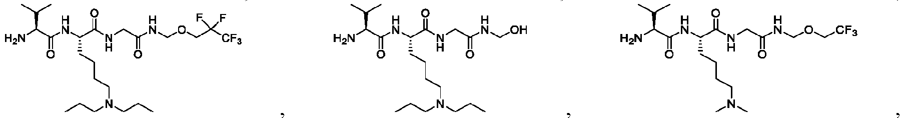 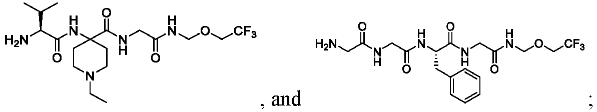
the compound of formula X-3 or the salt thereof is selected from the following structures: 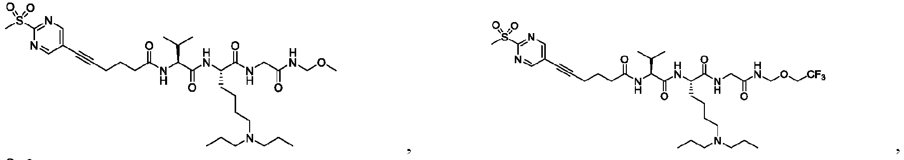 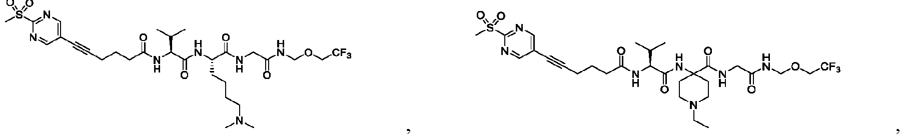 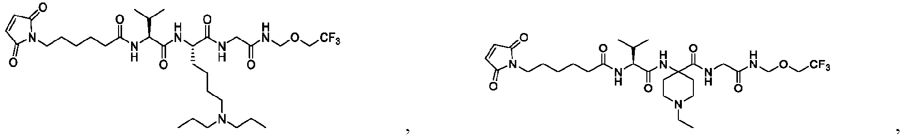 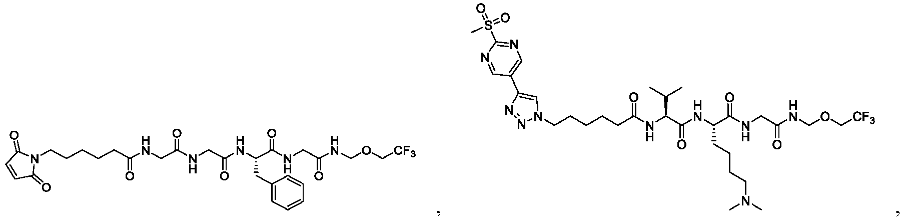 and
